# EUROPEAN PATENT APPLICATION

(11) **EP 4 397 764 A1**
(43) Date of publication of application: **10.07.2024**
(21) Application number: 22863450.7
(22) Date of filing: 30.08.2022
(51) Int. Cl.: C12N 15/62, C12N 7/01, C12N 5/10, C07K 16/10, A61K 39/42, A61P 31/18

(54) **GENE SEQUENCE CONSTRUCT FOR GENE THERAPY OF HUMAN IMMUNODEFICIENCY VIRUS INFECTION**

(30) Priority: 30.08.2021 WO PCT/CN2021/115422
(71) Applicant: KANGLIN BIOTECHNOLOGY (HANGZHOU) CO., LTD., Hangzhou, Zhejiang 310018 (CN)
(72) Inventor: WU, Haoquan, Hangzhou, Zhejiang 310018 (CN); SUN, Baozhen, Hangzhou, Zhejiang 310018 (CN); DANG, Ying, Hangzhou, Zhejiang 310018 (CN)
(74) Representative: K&L Gates LLP
(86) International application number: PCT/CN2022/115831
(87) International publication number: WO 2023/030315

(57) **Abstract**

A gene sequence construct for gene therapy of human immunodeficiency virus (HIV) infection. By sequentially linking, by means of a coding sequence of a linker polypeptide, gene coding sequences of respective single-chain variable fragment (scFv) regions of light chains and heavy chains of monoclonal antibodies having different binding site antigens involved in different steps of infecting human CD4+T cells by an HIV, gene coding sequences of respective scFv regions of light chains and heavy chains of monoclonal antibodies bound to a CD4 receptor site, and a gene coding sequence of a polypeptide for inhibiting the fusion of an HIV and a CD4+T cell membrane, a gene sequence construct is constructed in a promoter and downstream of a secretory signal peptide coding sequence to express a secretory antibody-like protein molecule coded by a single gene. The recombinant single-gene construct can be conveniently introduced into a target tissue cell by means of a viral vector, and a secretory expression antibody or antibody-like protein molecule has multi-antigen antigen tropism, and can effectively and widely block an infection process of an HIV on human CD4+T cells by binding to a plurality of binding sites involved in different steps of infecting human CD4+T cells by an HIV, and effectively avoid losing an HIV infection inhibition capability due to the escape mutation of an HIV, thereby achieving a long-term or even permanent treatment effect on HIV infection by single injection administration.

## Description

This application claims priority of PCT patent application No. PCT/CN2021/115422 filed on August 30, 2021, the entire content of which is incorporated here as a part of this application.

### Technical field

The invention belongs to the field of gene therapy/biomedicine technology, and specifically relates to a gene sequence construct for gene therapy of HIV infection. The gene sequence construct can be used for gene therapy against HIV infection. The gene sequence construct can be used to express polytropic neutralizing antibody-like proteins with broad-spectrum and efficient HIV-neutralizing activity both in vivo and in vitro, and can be used for clinical research on gene therapy drugs for HIV infection delivered by recombinant viruses or non-viral vectors, and for new drug research and development.

### Background of the Invention

AIDS (acquired immunodeficiency syndrome) caused by human immunodeficiency virus (HIV) infection is one of the most serious infectious diseases in the world today. Approximately 0.77 million of the 37.9 million HIV-infected people worldwide die of AIDS every year. With the development of anti-AIDS drugs, the expected survival period of actively treated HIV-infected patients has been greatly extended. However, the side effects and limitations of cocktail therapy, as well as the emerging drug-resistant strains of HIV, still causes long-term economic and social burdens, significant decline in the quality of life, and obvious suffering to the majority of AIDS patients.

Therefore, despite the progress that has been made in the anti-AIDS field, there is still a need to develop new drugs and methods for treating HIV infection.

### Summary of the invention

The present invention constructs a series of gene therapy constructs for resisting HIV infection based on recombinant viral vectors. For example, single-chain antibody variable region fragments (scFv) of a variety of broad-spectrum neutralizing antibodies against HIV and a neutralizing antibody against human CD4 receptor are combined with a HIV membrane fusion inhibitory polypeptide into a simple and efficient expression cassette.

Firstly, these gene sequence constructs for HIV infection gene therapy comprise one or more gene coding sequences of a single-chain antibody molecule (including a nanobody) without a constant region that has the ability to inhibit HIV infection and one or more gene coding sequences of a polypeptide (consisting of 2-50 amino acid residues) with the ability to inhibit HIV infection, to achieve the expression of a fusion protein molecule comprising the antibody molecule and the polypeptide against HIV infection encoded by a single gene, which has two or more than two target sites.

Specifically, the single-chain antibody molecule of the gene sequence construct described above includes a heavy chain variable region and/or a light chain variable region.

The light chain variable region comprises a light chain variable region of a kappa or lambda light chain.

The above-mentioned gene sequence construct comprises two or more than two gene coding sequences of single-chain antibody molecules without the constant region that have the ability to inhibit HIV infection.

Alternatively, the gene sequence construct comprises three or more than three gene coding sequences of single-chain antibody molecules without the constant region that have the ability to inhibit HIV infection.

Alternatively, the gene sequence construct comprises four or more than four gene coding sequences of single-chain antibody molecules without the constant region that have the ability to inhibit HIV infection.

In addition, the above-mentioned gene sequence construct may comprise two or more than two gene coding sequences of polypeptides with the ability to inhibit HIV infection. These polypeptides consist of 2-50 amino acid residues.

Alternatively, the gene sequence construct comprises three or more than three gene coding sequences of polypeptides with the ability to inhibit HIV infection.

Alternatively, the gene sequence construct comprises four or more than four gene coding sequences of polypeptides with the ability to inhibit HIV infection.

In the gene sequence construct described in any one of the above, the fusion protein molecule comprising the single-chain antibody molecule without the constant region and the polypeptide against HIV infection encoded by the single gene has three or more than three target sites.

Alternatively, the fusion protein molecule comprising the single-chain antibody molecule without the constant region and the polypeptide against HIV infection encoded by the single gene has four or more than four targets.

Alternatively, the fusion protein molecule comprising the single-chain antibody molecule without the constant region and the polypeptide against HIV infection encoded by the single gene has five or more than five targets.

Alternatively, the fusion protein molecule comprising the single-chain antibody molecule without the constant region and the polypeptide against HIV infection encoded by the single gene has six or more than six targets.

The gene sequence construct described in any one of the above comprises two or more than two gene coding sequences of single-chain antibody molecules without the constant region that have the ability to inhibit HIV infection and one or more gene coding sequences of the polypeptide with the ability to inhibit HIV infection.

In the gene sequence construct described above, the fusion protein molecule comprising the single-chain antibody molecule without the constant regions and the polypeptide against HIV infection encoded by the single gene has three or more than three target sites.

In the gene sequence construct described in any one of the above, the gene coding sequence of the single-chain antibody molecule without the constant regions that has the ability to inhibit HIV infection and the gene coding sequence of the polypeptide with the ability to inhibit HIV infection are directly or indirectly concatenated via a coding sequence of a linker polypeptide.

The gene sequence construct described in any one of the above comprises two or more than two gene coding sequences of single-chain antibody molecules without the constant region that have the ability to inhibit HIV infection, and the gene coding sequences of the antibody molecules are directly or indirectly concatenated via a coding sequence of a linker polypeptide.

The gene sequence construct described in any one of the above comprises two or more than two gene coding sequences of polypeptides with the ability to inhibit HIV infection, and the gene coding sequences of the polypeptides are directly or indirectly concatenated via a coding sequence of a linker polypeptide.

In the gene sequence construct of any one of the above, the one or more gene coding sequences of the single-chain antibody molecule without the constant region that has the ability to inhibit HIV infection comprises a gene coding sequence of an anti-HIV-1-gp160 (or its cleavage products gp120 and gp41) antibody molecule.

In the gene sequence construct of any one of the above, wherein the one or more gene coding sequences of the single-chain antibody molecule without the constant region that has the ability to inhibit HIV infection comprises a gene coding sequence of an antibody molecule that binds to human CD4 receptor site.

In the gene sequence construct of any one of the above, the one or more gene coding sequences of the polypeptide with the ability to inhibit HIV infection comprises a gene coding sequence of a polypeptide that inhibits the fusion of HIV and CD4+ T cell membranes.

The gene sequence construct described in any one of the above comprises two or more than two gene coding sequences of single-chain antibody molecules without the constant region that have the ability to inhibit HIV infection and one or more gene coding sequences of the polypeptide with the ability to inhibit HIV infection, and the two or more than two gene coding sequences of the single-chain antibody molecules without the constant region that have the ability to inhibit HIV infection comprise a gene coding sequence of an antibody molecule against HIV-1-gp160 (or its cleavage products gp120 and gp41) and a gene coding sequence of an antibody molecule that binds to human CD4 receptor site, and the one or more gene coding sequences of the polypeptide with the ability to inhibit HIV infection comprise a gene coding sequence of a polypeptide that inhibits the fusion of HIV and CD4+ T cell membranes.

The gene sequence construct described in any one of the above comprises (i) gene coding sequences of light chain complementarity determining regions (LCDR1, LCDR2 and LCDR3) and heavy chain complementarity determining regions (HCDR1, HCDR2 and HCDR3) of an anti-HIV-1-gp160 (or its cleavage products gp120 and gp41) monoclonal antibody , (ii) gene coding sequences of light chain complementarity determining regions (LCDR1, LCDR2 and LCDR3) and heavy chain complementarity determining regions (HCDR1, HCDR2 and HCDR3) of a monoclonal antibody that binds to human CD4 receptor site, (iii) a gene coding sequences of a polypeptide that inhibit the fusion of HIV and CD4+ T cell membranes, in which the coding sequences of the light chain and the heavy chain of the antibodies can be directly or indirectly concatenated via a coding sequence of a linker polypeptide in any order.

The gene sequence construct described in any one of the above comprises (i) gene coding sequences of light chain variable region (VL) and heavy chain variable region (VH) of an anti-HIV-1-gp160 (or its cleavage products gp120 and gp41) monoclonal antibody, (ii) gene coding sequences of light chain variable region (VL) and heavy chain variable region (VH) of a monoclonal antibody that binds to human CD4 receptor site, (iii) a gene coding sequence of a polypeptide that inhibits the fusion of HIV and CD4+ T cell membranes , in which the coding sequences of the light chain variable region (VL) and the heavy chain variable region (VH) of the antibodies can be directly or indirectly concatenated by a coding sequence of a linker polypeptide in any order.

The gene sequence construct described in any one of the above further comprises a promoter located upstream of the gene coding sequence of the single-chain antibody molecule without the constant region that has the ability to inhibit HIV infection and the gene coding sequence of the polypeptide with the ability to inhibit HIV infection.

The gene sequence construct described in any one of the above further comprises a secretion signal peptide coding sequence located upstream of the gene coding sequence of the single-chain antibody molecule without the constant region that has the ability to inhibit HIV infection and the gene coding sequence of the polypeptide with the ability to inhibit HIV infection.

The gene sequence construct described in any one of the above comprises a first gene coding sequence of the single-chain antibody molecule without the constant region that has the ability to inhibit HIV infection, a second gene coding sequence of the single-chain antibody molecule without the constant region that has the ability to inhibit HIV infection, and a gene coding sequence of the polypeptide with the ability to inhibit HIV infection, which is selected from:
VL2-linker-VH2-linker-VL 1 -linker-VH1 -linker; or
linker-VH2-linker-VL1-linker-VH1-linker-peptide inhibitor; or
other constructs that are constructed by arranging the combination of VL2 and VH2 or VL1 and VH1 in different orders in a construct.

VL2 and VH2 are the variable region fragments of light chain and heavy chain of the first antibody molecule respectively; VL1 and VH1 are the variable region fragments of light chain and heavy chain of the second antibody molecule respectively; the linker is a linker polypeptide; the peptide inhibitor is a polypeptide that inhibits HIV infection (for example, a polypeptide that inhibits the fusion of HIV and CD4+ T cell membranes).

The gene sequence construct described above is VL2-linker-VH2-linker-VL1-linker-VH1, or a construct in which the combinations of VL2 and VH2 or VL1 and VH1 are arranged in different orders.

The gene sequence construct described above is VL2-linker-VH2-linker-VL1-linker-VH1-linker-peptide inhibitor, or a construct in which the combinations of VL2 and VH2 or VL1 and VH1 are arranged in different orders.

In the gene sequence construct described above, the protein sequences of VL2 and VH2 include SEQ ID NO: 2, or a functional fragment thereof, or a homologous sequence that is at least 75%, 80%, 85%, 90%, 95%, 98%, or 99% identical thereto.

In the gene sequence construct described above, the protein sequences of VL1 and VH1 include SEQ ID NO: 3, or a functional fragment thereof, or a homologous sequence that is at least 75%, 80%, 85%, 90%, 95%, 98%, or 99% identical thereto.

In the gene sequence construct described above, the sequence of the linker polypeptide (linker) is selected from the following amino acid sequences: GGGGS, (GGGGS)₂, (GGGGS)₃, (GGGGS)₄, (GGGGS)₅, (GGGGS)₆ and (GGGGS)₇, or other optional linker polypeptide sequences.

In the gene sequence construct described above, the polypeptide that inhibits the fusion of HIV and CD4+ T cells membranes can be selected from the group consisting of membrane fusion inhibitory polypeptides P52, C34, T20, etc.

The sequence of the membrane fusion inhibitory polypeptide P52 includes SEQ ID NO: 5 or a homologous sequence that is at least 75%, 80%, 85%, 90%, 95%, 98%, or 99% identical thereto; the polypeptide sequence of C34 includes SEQ ID NO: 6 or a homologous sequence that is at least 75%, 80%, 85%, 90%, 95%, 98%, or 99% identical thereto; the polypeptide sequence of T20 includes SEQ ID NO: 7 or a homologous sequence that is at least 75%, 80%, 85%, 90%, 95%, 98%, or 99% identical thereto.

Furthermore, the present invention also provides a viral vector genome comprising any of the constructs described above and a corresponding viral vector system comprising the genome.

The viral vector system described above can be a lentiviral vector system or an adeno-associated virus vector system.

The lentiviral vector system can include the viral vector genome of any of the above constructs and other nucleotide sequences encoding and expressing packaging components required for the production of lentivirus, which are introduced into production cells to produce a lentiviral particle containing the genome of the construct described above.

The adeno-associated virus vector system can include the viral vector genome of any of the above constructs and other nucleotide sequences encoding and expressing packaging components required for the production of adeno-associated virus, which are introduced into production cells to produce an adeno-associated virus particle containing the genome of the construct described above.

Any virus particle produced above, which comprises the genome of any of the constructs described above, can express anti-HIV neutralizing antibody-like molecules after transducing cells, and can be used to administer to a patient to inhibit or prevent HIV infection.

The present invention also provides a pharmaceutical composition comprising the viral particle described above, and a pharmaceutically acceptable carrier or diluent, or cells transduced by the lentiviral particle described above in vitro, including but not limited to transduced muscle cells, liver cells, or CD4+ T cells.

The above-mentioned virus particle or the pharmaceutical composition containing the above virus particle can be used for injection into the body to express an antibody molecule protein with two or more than two target sites, which can efficiently and broadly block infection of HIV against human CD4+ T cells by binding to multiple binding sites involved in different steps of HIV infection against human CD4+ T cells, and are used for gene therapy of HIV infection, thereby achieving long-term treatment for a HIV-infected individual.

The present invention provides a method for inhibiting HIV infection, comprising administering the viral particle or the pharmaceutical composition described above to cells.

The cells include muscle cells, liver cells, or CD4+ T cells.

The above-mentioned cells can be transduced in vitro or in vivo using one of the above-mentioned viral particles or pharmaceutical compositions.

The present invention provides a method of treating HIV infection in a subject in need thereof, comprising administering to the subject a therapeutically effective dose of the viral particle or the pharmaceutical composition as described above.

The subjects described therein include an early-stage HIV infectors, a HIV-infected individual who is already received cocktail drug therapy, or a HIV-infected individual who is resistant to cocktail drug therapy.

The mode of administering drugs to the above-mentioned subject is intramuscular injection of one of the above-mentioned virus particles or pharmaceutical compositions.

Alternatively, one of the above-mentioned viral particles or pharmaceutical compositions or CD4+ T cells transduced thereby is injected intravenously.

The virus particle and pharmaceutical composition injected into the body through the above-mentioned methods can express anti-HIV protein molecules with multiple targets sites and secrete them into blood, which can act on multiple nodes of HIV infection, and can effectively block HIV infection path and effectively avoid the loss of the ability to inhibit HIV infection due to escape mutations of HIV, thereby achieving a long-term (for example, therapeutically effective lasts for one or several years) or even permanent therapeutic effect on HIV infection with a single injection.

Antibody-like molecules composed of multiple single-chain antibody variable region fragments (scFv) based on the above expression cassetteswere delivered to mice via lentivirus and adeno-associated virus vectors, and showed effective blood concentration, strong in vitro cytological HIV-1 virus neutralizing activity, and broad-spectrum neutralizing ability against both CXCR4 and CCR5 tropic viruses, suggesting a highly potential technical route for anti-HIV broad-spectrum neutralizing antibody gene therapy drugs.

The present invention can be used forvarious forms of anti-HIV gene therapy based on the genetic expression of broad-spectrum neutralizing antibodies.

### Description of the drawings

The drawings of the present invention are described in detail.
Figure 1. Schematic representation of the mature molecular structure of a tritropic anti-HIV neutralizing antibody expected to exist in monomeric form.
Figure 2. Schematic representation of the gene sequence constituent of a tritropic anti-HIV neutralizing antibodies.
Figure 3. Schematic representation of the mature molecular structure of an amphotropic anti-HIV neutralizing antibody expected to exist in monomeric form.
Figure 4. Schematic representation of the gene sequence constituent of an amphotropic anti-HIV neutralizing antibody.
Figure 5. Schematic representation of a gene construct for cloning the gene sequence of an amphotropic (KL-BsHIV01-02) or tritropic (KL-BsHIV01-003-02) anti-HIV neutralizing antibody into a lentiviral vector.
Figure 6. Profile of the latest generation lentiviral vector pKL-kan-lenti-CBH-WPRE used in the present invention.
Figure 7. Schematic representation of a gene construct for cloning the gene sequence of an amphotropic (KL-BsHIV01-02) or tritropic (KL-BsHIV01-003-02) anti-HIV neutralizing antibody into an adeno-associated virus vector.
Figure 8. Profile of the latest generation adeno-associated virus vector pAAV-MCS-CBH-WPRE used in the present invention.
Figure 9. Anti-HIV neutralizing antibodies-like detected by Western blot analysis. M, prestained protein size marker; 1 and 6, purified Flag-KL-BsHIV01-003-02; 2 and 7, purified KL-BsHIV01-003-02; 3-5 and 8-10, the products of purified Flag-KL-BsHIV01-003-02 digested with enterokinase under different conditions. 1-5, the primary antibody is rabbit Anti-KL-BsHIV01-003-02 polyclonal antibody; 6-10, the primary antibody is mouse Anti-DYKDDDDK (Flag-tag).
Figure 10. Neutralizing activity of tritropic KL-BsHIV01-003 (with Fc fragment) and KL-BsHIV01-003-02 (without Fc fragment) anti-HIV neutralizing antibodies-like.
Figure 11. Expression of different doses of anti-HIV neutralizing antibody adeno-associated virus gene therapy vector in BALB/c mice after intramuscular injection. The levels of anti-HIV neutralizing antibodies-like secreted into mouse serum were determined by ELISA.
Figure 12. Expression of different doses of anti-HIV neutralizing antibody adeno-associated virus gene therapy vector in BALB/c mice after intramuscular injection. Neutralizing activity of anti-HIV neutralizing antibodies-like secreted into mouse serum.

### Detailed description of the invention

Broadly neutralizing antibodies (bNAb) with HIV-1 neutralizing activity are produced in a small number of elite infected individuals over several years, which can efficiently and broadly bind to virus surface glycoproteins of HIV and neutralize the infection activity of HIV anainstCD4+ T cells, thereby representing a promising method for preventing or resisting HIV-1 infection. However, the specific mechanism for production of the bNAb is not clear. Most individuals infected with HIV-1 can only produce non-neutralizing antibodies. Currently, there is no research has successfully induced the production of broad-spectrum neutralizing antibodies in healthy subjects through standard immunization methods.

Without wishing to be bound by theory, it is believed that in some embodiments, recombinantly derived broad-spectrum neutralizing antibodies against HIV-1 virus can effectively reduce the viral load in a patient, and even if they cannot completely eliminate HIV from the body, they can help control the progression from HIV infection to AIDS. Compared with small molecular anti-HIV drugs, recombinantly derived neutralizing antibodies can also greatly reduce side effects and increase patient compliance. Clinically, recombinant broad-spectrum neutralizing antibodies can be used as vaccine replacement products for preventing HIV infection in specific situations, or as antiviral drugs at different disease stages.

However, like highly effective anti-HIV small molecular drugs, specific broad-spectrum neutralizing antibodies only target a single epitope of a single viral protein (HIV-gp160). The escape phenomenon caused by viral mutations is still difficult to avoid. Sincethe development, production, and use costs of broad-spectrum neutralizing antibodies are much higher than that of small-molecular anti-HIV drugs, combined use of drugs has no advantages. Therefore, a large number of anti-HIV broad-spectrum neutralizing antibody drugs under development are difficult to use clinically to date.

In response to the above problems, the research and development of broad-spectrum neutralizing antibody drugs against HIV mainly requires breakthroughs in the following two aspects:
1. Develop of amphotropic or polytropic neutralizing antibodies or antibody-like macromolecules to cover multiple targets to avoid escape caused by viral mutations.
2. Geneticization of broad-spectrum neutralizing antibodies or antibody-like macromolecules. Anti-HIV infection gene therapy drugs delivered by recombinant viruses or non-viral vectors stably express neutralizing antibodies or antibody-like macromolecules in the body in a genome-integrated or non-integrated manner for a long time. A single treatment is effective for a long time or even lifelong, greatly reducing the production and use costs of macromolecular antibody drugs.

The present invention adopts a polytropic antibody molecular structure, that is, the antigen-binding region consistsof two or more than two single-chain variable region fragments (scFv) of monoclonal broad-spectrum neutralizing antibodies concatenated by a linker polypeptide (linker).

Specific embodiments include constructing a series of antibody molecule gene constructs containing amphotropic/tritropic antibody single chain variable region fragments (scFv) and cloning them into recombinant adeno-associated virus and recombinant lentiviral vectors. Then the corresponding adeno-associated virus and lentivirus are packaged in 293T cells, and the 293T cells and differentiated and undifferentiated muscle cell lines are infected with a certain biological titer of the virus. The antibody molecules produced in the cell supernatant were tested quantitatively and qualitatively, and their neutralizing activity against the HIV-1 wild-type virus strain was tested in the infection activity analysis of the HIV-1 wild-type virus strain and the virus-sensitive reporter cell line TZM-bl.

The structure of the anti-HIV neutralizing antibody of the present invention, the constituentof the gene sequence construct, and the special terms involved will be further described in detail below in conjunction with the examples.

**Antibody**can be an immunoglobulin, an antigen-binding fragment, or a protein molecule derived therefrom that specifically recognizes and binds to an antigen (e.g., HIV-1 gp41 antigen). "Antibody" in the present invention is a broad definition covering various antibody structures, including but not limited to monoclonal antibodies, polyclonal antibodies, polytropic antibodies (e.g., amphotropic antibodies, tritropic antibodies), and antibody fragments, provided they possess specific antigen-binding activity. Examples of specific antibodies include intact immunoglobulins as well as antibody variants and fragments that retain binding affinity for the antigen. Examples of antibody fragments include, but are not limited to, variable region fragments (Fv), antigen-binding fragments (e.g., Fab, Fab', Fab'-SH, or F(ab')2 produced after proteolysis), single-chain antibodies molecules (eg, scFv), diabodies, nanobodies, and polytropic antibodies formed from combinations of antibody fragments. Antibody fragments include antigen-binding fragments produced by modification of intact antibodies or antigen-binding fragments synthesized de novo using recombinant DNA technology.

Single chain antibodies (scFv) are molecules obtained through genetic engineering and contain the light chain variable region (VL) and heavy chain variable region (VH) of one or more antibodies, each fragment is concatenated by a suitable linker polypeptide to form a fused single-chain molecule. The concatenation orders of VL and VH in a single-chain antibody molecule usually does not affect its antigen-binding function, thus both single-chain antibodies composed of two concatenation manners (VL-VH or VH-VL) will be used.

An antibody can have one or more antigen-binding sites. In the case of more than one antigen binding site, these binding sites may be the same or different. For example, a naturally occurring immunoglobulin has two identical antigen-binding sites, while a Fab fragment produced from an immunoglobulin by papain hydrolysis has only one antigen-binding site, and an amphotropic single-chain antibody (scFv) has two different antigen-binding sites.

Normally, a naturally occurring immunoglobulin consists of a light chain and a heavy chain linked by disulfide bonds. Immunoglobulin genes includeγ, α, δ, ε, µ, λ, andκ constant region genes as well as numerous immunoglobulin variable region genes. Light chains are of two types, λ and κ. There are five main types of heavy chains (γ, α, δ, ε, µ), which determine the functional classification of antibody molecules, namely IgG, IgA, IgD, IgE, and IgM.

Each heavy and light chain contains a constant region and a variable region. VH represents the variable region of the antibody heavy chain, including the heavy chain variable region of the antigen-binding fragment Fv, scFv, or Fab. VL represents the variable region of an antibody light chain, including the light chain variable region of an antigen-binding fragment Fv, scFv, or Fab. In the following examples, VH and VL work together to specifically recognize and bind antigens.

VH and VL contain three separated highly variable regions (also called complementarity determining regions (CDRs)) and a framework region. The sequences of the backbone regions of different light and heavy chains are relatively conserved within the same species. The backbone region of an antibody determines the position of the complementarity-determining regions in the three-dimensional structure. The complementarity determining region is mainly responsible for binding to the epitope of an antigen. The three complementarity determining regions on the light chain are labeled LCDR1, LCDR2, and LCDR3 from N-terminus to C-terminus, respectively. The three complementarity determining regions on the heavy chain are labeled HCDR1, HCDR2, and HCDR3 from N-terminus to C-terminus, respectively.

The protein sequences of VH and VL in the present invention include, in addition to the sequences disclosed in the following examples, any other sequences that carry functional fragments thereof, or a homologous sequence that is at least 75%, 80%, 85%, 90%, 95%, 98%, or 99% identical thereto.

**Constant region fragment of an antibody** is a region of immunoglobulin with a relatively stable amino acid sequence except for the variable region near the N-terminus where the amino acid sequence changes greatly, including the constant domain in the antigen-binding fragment (located at the N-terminus of the hinge region, including the light chain constant domain and the heavy chain constant domain) and the constant domain of the crystallizable fragment of the heavy chain (called the Fc fragment, located at the C-terminal of the hinge region). The Fc fragment usually refers to the last two constant region domains of immunoglobulins IgA, IgD, and IgG or the last three constant region domains of IgE and IgM. The Fc fragment may also include part or all of the hinge region sequence located at its N-terminus.

**Anti-HIV-1 neutralizing antibody or antigen-binding fragment** specifically binds to the HIV-1 envelope protein (e.g., binds to gp41), thereby inhibiting biological functions associated with the HIV-1 envelope (e.g., the ability to bind to target receptors). In the following examples, anti-HIV-1 neutralizing antibodies or antigen-binding fragments reduce the infection titers of HIV-1 strains of different tropisms on cells.

**Amphotropic or polytropic antibodies** is a recombinant molecule composed of two or more than two different antigen-binding domains, and therefore can bind two or more than two different antigenic determinants. Amphotropic or polytropic antibodies include molecules composed of two or more than two different antigen-binding domains linked through chemical synthesis or genetic engineering. The antigen-binding domains can be linked via a linker polypeptide. The antigen-binding domain can be a monoclonal antibody, an antigen-binding fragment (e.g., scFv or Fab), or a combination of antigen-binding domains from different sources.

**Linker polypeptide** is used to connect two protein molecules or fragments into a continuous single fusion molecule. For example, in the following examples, two or more antibody molecules or antigen-binding fragments (e.g., scFv) are connected to form a polytropic antibody molecule with two or more antigen-binding sites; or the light chain variable region (VL) and heavy chain variable region (VH) of an antibody are connected to form a single-chain antigen-binding sequence; or the antibody molecule or antigen-binding fragment is connected with other effector molecules, for example, the antigen-binding fragment scFv is connected to an HIV-1 membrane fusion inhibitory polypeptide to form a fusion protein. Linker polypeptides are typically rich in glycine (Gly or G) to increase linker flexibility, and rich in serine (Ser or S) or threonine (Thr or T) to increase solubility, for example, the (GGGGS)ₙ linkers with different lengths used in some of the following examples, n may be 1 or more than 1. However, the linker polypeptide sequence used in the examples is not limited to this, and also includes other optional linker polypeptide sequences.

**Antigenic determinant** is a specific chemical group or polypeptide sequence on a molecule that has antigenicity, that is, it can stimulate a specific immune response of the host. An antibody specifically binds to a specific epitope on a polypeptide, such as in the following examples, an antibody that specifically binds to an epitope on gp41.

**HIV-1 envelope protein** is first synthesized as a precursor protein with a size of 845-870 amino acid residues, called HIV gp160. gp160 forms a homotrimer in the host cell, which is glycosylated, cleaved to remove the signal peptide, and then cleaved by an intracellular protease at amino acid residues 511/512 to produce gp120 and gp41 polypeptide chains. gp120 and gp41 remain associated together in the homotrimer as the gp120/gp41 protomer. Mature gp120 consists of amino acid residues 31-511 of the HIV-1 envelope protein and is a highly N-glycosylated protein, constituting the majority of the domain of the HIV-1 envelope protein trimer exposed on the envelope surface. gp120 is responsible for binding to the human CD4 cell receptor as well as coreceptors (e.g., chemokine receptors CCR5 or CXCR4). gp41 consists of amino acid residues 512-860 of the HIV-1 envelope protein, including the intra-envelope domain, the transmembrane domain, and the extra-envelope domain. The extra-envelope domain of gp41 includes amino acid residues 512-644, which combines with gp120 to form a protomer, which together constitute the HIV-1 envelope protein homotrimer. The protruding extra-envelope domain of the HIV-1 envelope protein trimer undergoes several structural rearrangements, from a closed structure before fusion with the host cell membrane that can escape antibody recognition, to an intermediate structures that bind human CD4 cell receptors and coreceptors and a post-membrane fusion structures.

**HIV** membrane fusion inhibitory peptide: Membrane fusion of virus and host cells is a key step in HIV infection against cells. After binding of the glycoprotein gp120/gp41 homotrimer on the HIV envelope to the human CD4 cell receptor and co-receptor, the structure undergoes several changes. Finally, the gp41 trimer integrated on the HIV envelope is inserted into the host cell membrane, completing the fusion of the virus and host cell membranes, and resulting in entering of HIV genetic material into the cells. HIV membrane fusion inhibitory polypeptide binds to the envelope protein of the virus, thereby preventing it from undergoing the structural changes necessary for the fusion of the virus and the host CD4 cell membrane, and preventing HIV from infecting CD4 cells. HIV membrane fusion inhibitory polypeptides used in the present invention include the P52, C34, T20 sequences disclosed in the following examples or homologous sequences that are at least 75%, 80%, 85%, 90%, 95%, 98%, or 99% identical thereto.

**Gene sequence construct** is a vector composed of a recombinant polynucleotide sequence, which is composed of an expression control sequence connected to the nucleic acid sequence to be expressed. The expression vector contains sufficient cis-acting elements and other expression elements are provided by the host cell. Expression vectors include all vectors of the present invention, such as plasmids and viruses carrying integrated recombinant polynucleotide sequences (e.g., recombinant lentivirus and recombinant adeno-associated virus). The vector carries a nucleic acid sequence (DNA or RNA) that allows it to replicate in a host cell, such as a replication initiation site, one or more selectable marker genes, and other genetic structures disclosed in the present invention. Viral vectors are recombinant nucleic acid vectors that carry at least part of the nucleic acid sequence from one or more viruses. In certain examples below, a viral vector contains one or more nucleic acid sequences encoding a disclosed antibody or antigen-binding fragment that specifically binds to HIV-1 gpl60 and thereby neutralizes HIV-1. In some examples, the viral vector may be a recombinant adeno-associated virus (AAV) vector or a recombinant lentiviral vector. These viral vectors are replication-defective vectors and require other helper plasmids or vectors carrying gene functions or components necessary for viral replication to be amplified and packaged in cells to produce viral particles. The purified and prepared viral vectors or viral particles will not replicate and amplify in host cells when used to treat patients.

**Treatment of HIV infection:** HIV is a virus that attacks the body's immune system. It mainly targets the most important CD4 T lymphocytes in the human immune system, substantially destroys these cells, leading to the loss of immune function. Many patients with acute HIV infection experience flu-like symptoms 2 to 4 weeks after infection, which may last from days to weeks. Patients in the acute infection stage have a large amount of HIV in their blood and are highly infectious. Afterwards, the patient enters the asymptomatic chronic infection period, also known as the HIV latent period. However, the HIV in the patient's is still active, continues to proliferate, and can be spread. The average incubation period of HIV in the human is 8 to 9 years. During the incubation period of the HIV, HIV-infected people can live and work for many years without any symptoms. However, if people infected with HIV do not receive any anti-HIV infection treatment, they will develop into the most serious stage of HIV infection, that is, the AIDS stage after the incubation period. AIDS patients have a high viral load and can easily transmit HIV to others. Their immune systems are severely damaged and their bodies are susceptible to various diseases and malignant tumors, with a high mortality rate.

Currently, there is still a lack of effective drugs to cure HIV infection worldwide. The current treatment goals are: maximally and sustainably reducing viral load; rebuilding acquired immune function and maintaining immune function; improving quality of life; and reducing HIV-related morbidity and mortality. The currently commonly used anti-HIV infection method is a combined antiretroviral therapy (i.e., cocktail therapy), which greatly improves the efficacy of anti-HIV and significantly improves the quality of life and prognosis of patients. The earlier the cocktail therapy is started, the better the results. For example, in the acute phase of HIV infection, i.e., in the first few months after infection, treatment should be started as soon as HIV infection is diagnosed. However, cocktail therapy has side effects and limitations, such as the need of maintaining long-term compliance with continuous medication, as well as the emerging drug-resistant strains of HIV, which still causes long-term economic and social burdens, a significant decline in the quality of life, and obvious suffering to the majority of AIDS patients.

Broad-spectrum neutralizing antibodies with HIV-1 neutralizing activity identified and isolated from a small number of elite infected individuals can efficiently and broadly bind to virus surface glycoproteins of HIV and neutralize the infection activity of HIV against human CD4+ T cells, thereby representing a promising method for preventing or resisting HIV-1 infection. Compared with small molecular anti-HIV drugs, recombinantly derived neutralizing antibodies can also greatly reduce side effects and increase patient compliance. The only anti-HIV neutralizing antibody currently approved for clinical use is Ibalizumab, which targets the CD4 receptor on the surface of human T cells. It interferes with the binding of virus surface glycoproteins of HIV to CD4 receptors by binding to CD4 receptors, thereby neutralizing the infection activity of the virus against CD4+ T cells and showing excellent efficacy in patients with multi-drug resistance to HIV infection.

However, specific broad-spectrum neutralizing antibodies only target a single epitope of a single viral protein (HIV-gp160). The escape phenomenon caused by viral mutations is still difficult to avoid. Since the development, production, and use costs of broad-spectrum neutralizing antibodies are much higher than that of small-molecular anti-HIV drugs, combined use of drugs has no advantages. The present invention develops amphotropic and polytropic neutralizing antibodies or antibody-like macromoleculess, which cover multiple targets to avoid escape caused by viral mutations, and geneticizes the broad-spectrum neutralizing antibodies or antibody-like macromolecules, resulting in a breakthrough in the development of anti-HIV broad-spectrum neutralizing antibody drugs. Anti-HIV infection gene therapy drugs delivered by recombinant viruses or non-viral vectors stably express neutralizing antibodies or antibody-like macromolecules in the body in a genome-integrated or non-integrated manner for a long time. A single treatment is effective for a long time (for example, therapeutically effective lasts for one year or years) or even lifelong, greatly reducing the production and use costs of macromolecular antibody drugs.

The following examples explain the present invention and the present invention is not limited to the following examples.

### Examples

### I. Design of structure of HIV neutralizing antibodies

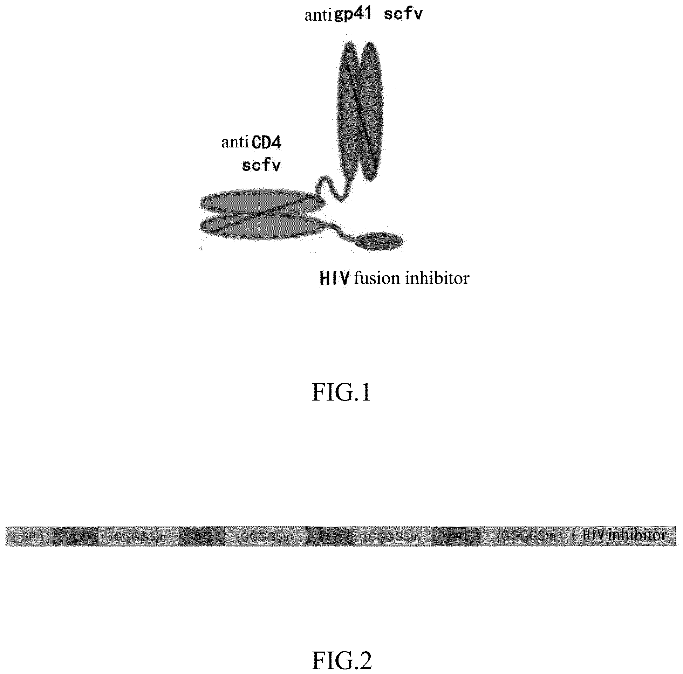

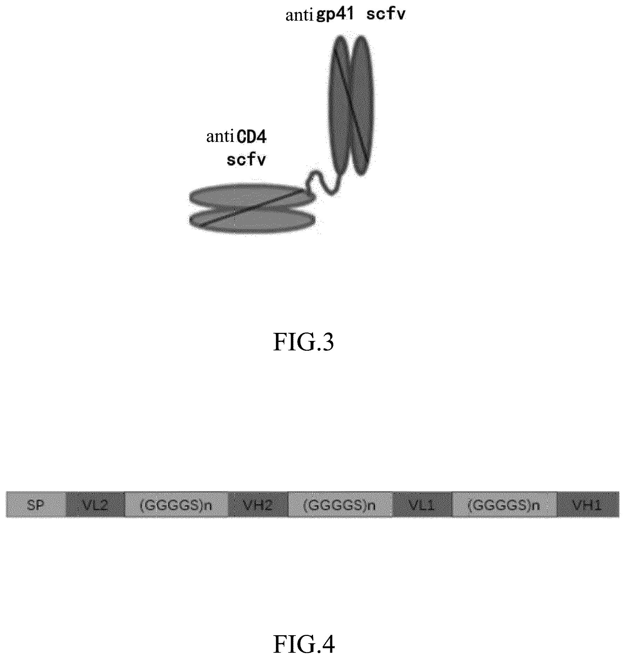

The structures of the anti-HIV neutralizing antibody and the antibody-like molecule used in the present invention are:
1. a tritropic anti-HIV neutralizing antibody-like scFv molecule, which: from the N-terminal to the C-terminal of the protein molecule consists of - signal peptide - scFv of anti-HIV-gp41 broad spectrum neutralizing antibody (VL-(ggggs)ₙ linker-VH)-(ggggs)ₙ linker- scFv of anti-human CD4 antibody (VL-(ggggs)ₙ linker-VH)-(ggggs)ₙ linker-HIV membrane fusion inhibitory short peptide. The coding sequence is expressed in cells, translated into protein, and then secreted out of the cells. The expected structure of the mature anti-HIV neutralizing antibody-like molecule is shown in Figure 1, and the gene structure is shown in Figure 2.
2. A amphotropic anti-HIV neutralizing antibody-like scFv molecule, which: from the N-terminal to the C-terminal of the protein molecule consists of - signal peptide - scFv of anti-HIV-gp41 broad spectrum neutralizing antibody (VL-(ggggs)ₙ linker-VH)-(ggggs)ₙ linker- scFv of the anti-human CD4 antibody (VL-(ggggs)ₙ linker-VH). The coding sequence is expressed in cells, translated into protein, and then secreted out of the cells. The expected structure of the mature anti-HIV neutralizing antibody-like molecule is shown in Figure 3, and the gene structure is shown in Figure 4.

### II. Gene sequences expressing HIV neutralizing antibodies

The sequence of anti-HIV-gp41 broad spectrum neutralizing antibody scFv are: signal peptide (the protein sequence is as set forth in SEQ ID NO: 1); anti-HIV-1-gp41-MPER antibody (10E8v4- 5R+100cF)-scFv (the protein sequence is as set forth in SEQ ID NO: 2); single-chain variable region fragment scFv of anti-human CD4 antibody (Ibalizumab) (the protein sequence is as set forth in SEQ ID NO: 3), HIV membrane fusion inhibitory short peptide P52 (the protein sequence is as set forth in SEQ ID NO: 4), HIV membrane fusion inhibitory short peptide C34 (the protein sequence is as set forth in SEQ ID NO: 5), HIV membrane fusion inhibitory short peptide T20 (the protein sequence is as set forth in SEQ ID NO: 6).

### III. A total of two gene expression cassettes for anti-HIV neutralizing antibody were constructed (as shown in Figure 2 and Figure 4), which are:

1. Anti-HIV amphotropic neutralizing antibody-like molecule KL-BsHIV01-02 containing anti-HIV neutralizing antibody 10E8v4-5R+100cF -scFv (the protein sequence is as set forth in SEQ ID NO: 7) (the DNA sequence is as set forth in SEQ ID NO: 8) (Figure 4).
2. Anti-HIV tritropic neutralizing antibody-like molecule KL-BsHIV01-003-02 containing anti-HIV neutralizing antibody 10E8v4-5R+100cF-scFv (the protein sequence is as set forth in SEQ ID NO: 9) (the DNA sequence is as set forth in SEQ ID NO: 10) (Figure 2).

### IV. Examples of gene therapy vector constructs for HIV neutralizing antibodies

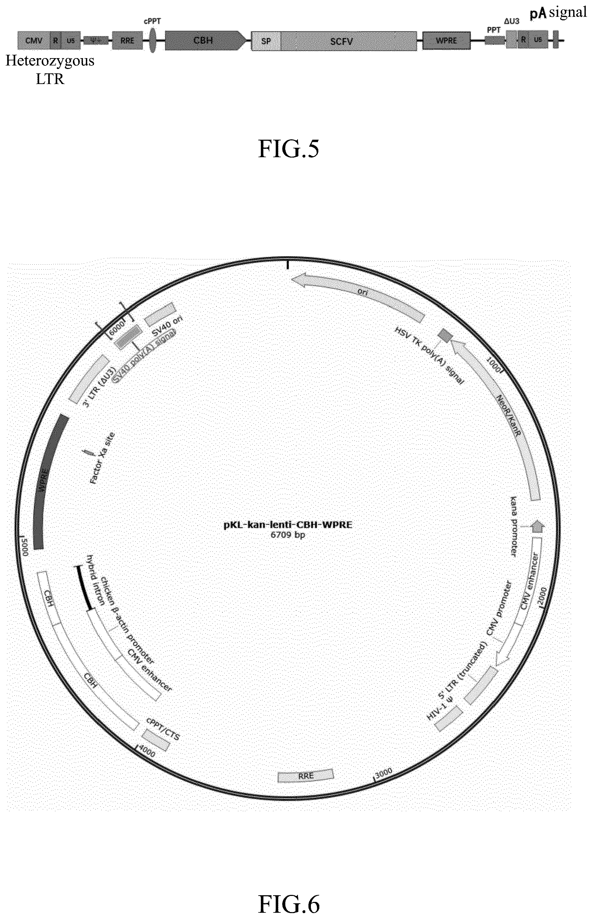

As shown in Figure 5, the above gene expression cassette for monoclonal antibody molecule was cloned into the latest generation lentiviral vector currently used, pKL-kan-lenti-CBH-WPRE (Figure 6) (the DNA sequence is as set forth in SEQ ID NO: 11) by multi-fragment recombinant ligation. The lentiviral vector includes: 5'LTR, in which the promoter region of the LTR is replaced with a CMV promoter; ψ packaging signal; retroviral export element RRE; cPPT; a promoter CBH; a polynucleotide encoding a polypeptide of an HIV neutralizing antibody fragment; the post-transcriptional regulatory element WPRE; PPT; ΔU3 3'LTR; and a poly(A) signal. The gene expression cassette for neutralizing antibody (Figure 2 and Figure 4) designed in this example was synthesized by Nanjing Genscript Biotechnology Co., Ltd., which together with the CBH promoter (the sequence is as set forth in SEQ ID NO: 22), were cloned between the multiple cloning sites AgeI/EcoRV on the lentiviral vector backbone pKL-kan-lenti-CBH-WPRE by homologous recombination methods well known in the art (Figure 6). After cloning was completed, the sequence information was confirmed by sequencing, and the plasmid was named as pKL-Kan-lenti-CBH-KL-BsHIV01-02 (the sequence is as set forth in SEQ ID NO: 12), pKL-Kan-lenti-CBH-KL-BsHIV01-003-02 (the sequence is as set forth in SEQ ID NO: 13).

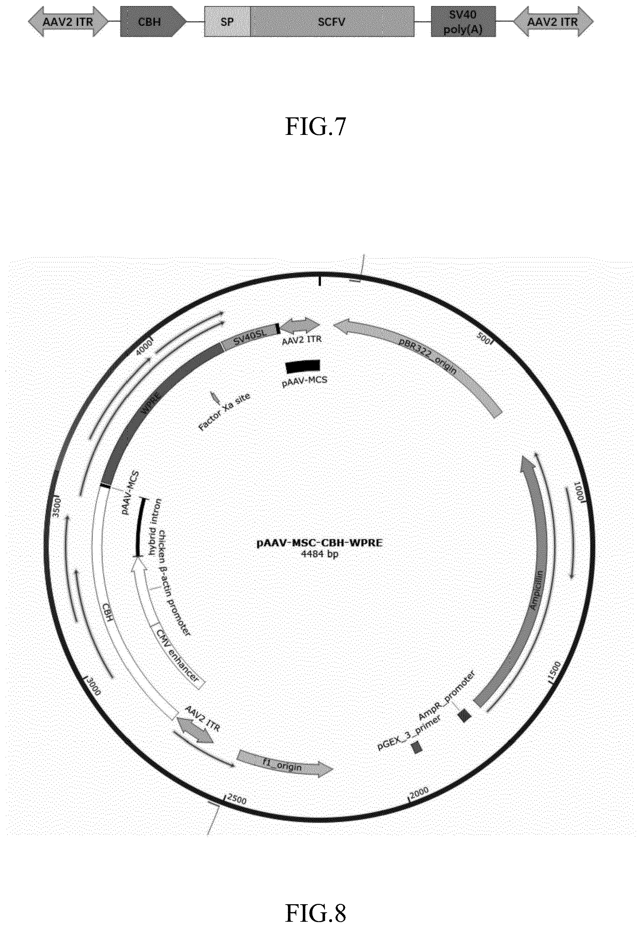

As shown in Figure 7, the HIV neutralizing antibody gene expression cassettes CBH-KL-BsHIV01-02 and CBH-KL-BsHIV01-003-02 present in pKL-Kan-lenti-CBH-KL-BsHI V01, were cloned betweenthe multi-cloning sites AgeI/SalI of on the currently used latest generation adeno-associated virus vector pAAV-MCS-CBH-WPRE (the sequence is as set forth in SEQ ID NO: 14) through multi-fragment recombination (see Figure 8). The adeno-associated virus vector includes: AAV2 ITR; a promoter CBH; a polynucleotide encoding HIV neutralizing antibody fragment; WPRE and SV40 poly(A) signal; AAV2 ITR. The plasmids were named as pAA V-CBH-KL-BsHIV01-02-WPRE (the sequence is as set forth in SEQ ID NO: 15) and pAAV-CBH-KL-BsHIV01-003-02-WPRE (the sequence is as set forth in SEQ ID NO: 16).

### V. Packaging and purification of viruses expressing HIV neutralizing antibodies

Lentiviral vectors (pKL-Kan-lenti-CBH-KL-BsHIV01-02and pKL-Kan-lenti-CBH-KL-BsHIV01-003-02) were used to package lentiviral vectors for HIV neutralizing antibody gene therapy in the 293T cell line. The HIV neutralizing antibody gene therapy lentiviral vectors constructed in the examples (pKL-Kan-lenti-CBH-KL-BsHIV01-02 and pKL-Kan-lenti-CBH-KL-BsHIV01-003-02), envelope plasmid (pKL-Kan-Vsvg, the nucleotide sequence is shown in SEQ ID NO: 17) and the packaging plasmid (pKL-Kan-Rev, the nucleotide sequence is shown in SEQ ID NO: 18; pKL-Kan-GagPol, the nucleotide sequence is shown in SEQ ID NO: 19) were mixed and co-transfected into 293T cells (purchased from the American Type Culture Collection Center (ATCC), ATCC deposit number: CRL-3216) at the same time, to perform the package of the HIV neutralizing antibody gene therapy lentivirus in the 293T cell line. The transfection method was transient transfection of eukaryotic cells mediated by PEI cationic polymer. The PEI cationic polymer is the PEI-Max transfection reagent purchased from Polysciences (Cat. No.: 24765-1), the transfection operation was carried out according to the standardized operation recommended by the manufacturer, and the transfection scale was 15cm cell culture dish. At 48 hours after the transfection was completed, the lentiviral vector was harvested (transfected cell culture supernatant). The supernatant was firstly centrifuged in a desktop bucket centrifuge at 4000 rpm at room temperature for 5 minutes to remove cell debris, and then centrifuged at 10000g at 4°C for 4 hours to obtain virus particle pellet. After removing the centrifugation supernatant, 1 mL DMEM complete medium was added to the virus particle pellet, the virus particles were resuspended with a microinjector, and the prepared virus resuspension was aliquoted and frozen at -80°C for later use.

AAV expression vectors (pAAV-CBH-KL-BsHIV01-02-WPRE and pAAV-CBH-KL-BsHIV01-003-02-WPRE) were used to package HIV neutralizing antibody gene therapy AAV vectors in the 293T cell line. The HIV neutralizing antibody gene therapy lentiviral vectors constructed in the examples, capsid plasmid (AAV2/8, the nucleotide sequence is shown in SEQ ID NO: 20) and packaging plasmid (pHelper, the nucleotide sequence is shown in SEQ ID NO: 21) were mixed and co-transfected into 293T cells at the same time, to perform the package of the HIV neutralizing antibody gene therapy vector AAV in the 293T cell line. The transfection method was transient transfection of eukaryotic cells mediated by PEI cationic polymer. The PEI cationic polymer is the PEI-Max transfection reagent purchased from Polysciences (, Cat. No.: 24765-1). The transfection operation was carried out according to the standardized operation recommended by the manufacturer, and the transfection scale was 15cm cell culture dish. The supernatant was discarded after 7 hours after transfection and replaced with 25 ml of toxin-producing medium. At 120 hours after the transfection is completed, the supernatant and cells were collected, centrifuged at 4200 rpm for 10 minutes, and then the supernatant and cells were separated. Lysis solution and ribozyme were added to the cells, lysed and digested for 1 hour, centrifuged at 10000g for 10 minutes and the lysate supernatant was obtained. The lysate supernatant and culture medium supernatant were purified by affinity chromatography and then aliquoted and frozen at -80°C for later use.

### VI. Functional verification of HIV neutralizing antibodies expressed in the supernatant of cells transduced with lentiviral gene therapy vectors

The packaged lentiviral vectors pKL-Kan-lenti-CBH-KL-BsHIV01-02 and pKL-Kan-lenti-CBH-BsHIV01-003-02 were used to infect 293T cells at different MOIs. After 48 hours, the supernatant and some cells were collected. The infection copy number of lentiviral vector was detected by probe method.
1. 293T cells infected with lentiviral vectors were collected, washed with PBS, centrifuged at 4200rpm for 5 minutes to collect the cells, resuspended in 20µl QE DNA Extraction Solution and subjected to the following program on a PCR machine to lyse the cells and extract the total DNA.

### Cell Lysis PCR Procedure:

| | Temperature | | Time |
|---|---|---|---|
| | 65°C | | 15 min |
| | 68°C | | 15 min |
| | 95°C | | 10 min |

Quantitative PCR was used to calculate the infected lentivirus copy number (VCN) of 293T cells using methods well known in the art.

2. PTZM-bl cells at 2E4 cells/well were plated. 50 µL of the antibody expression supernatant of different cells with a VCN of 1 were taken, diluted to different times, mixed with 50 µL of HIV pAD-8 and pNL4-3 respectively, incubated at 37°C for 30 minutes, and added to TZM-bl cells. The wells where only pAD-8 or pNL4-3 was added were negative controls (NC), and the well where HIV was not added was Blank. At 24 hours, the supernatant was diacarded, and 100 µL cell lysis solution was added. After 10 minutes, the lysed cells were collected, and centrifuged at 8000 rpm for 5 minutes. 100 µL of firefly luciferase detection reagent was added to 50 µL of lysed cells. RLU (relative light unit) was measured using a multifunctional microplate reader with chemiluminescence function. The results showed that in in vitro cell experiments, at the same infection copy number, 293T cell culture supernatant HIV transduced with the neutralizing antibody lentiviral gene therapy vectors contained antibodies that could neutralize HIV, and the neutralizing effect of the amphotropic neutralizing antibody KL-BsHIV01-02 was significantly better than that of KL-BsHIV01 (Table 1), while the neutralizing effect of the tritropic neutralizing antibody KL-BsHIV01-003-02 was significantly better than that of KL-BsHIV01- 003 (Table 2).

**Table 1. Neutralization percentage of HIV neutralizing antibody lentiviral gene therapy vector-transduced 293T cell culture supernatant against virus**

| **Cell culture supernatant dilution** | **pAD-8** | | **pNL4-3** | |
|---|---|---|---|---|
| | **KL-BsHIV01** | **KL-BsHIV01-02** | **KL-BsHIV01** | **KL-BsHIV01-02** |
| diluted 9 times | 98.00 | 99.98 | 99.99 | 99.99 |
| diluted 27 times | 80.23 | 99.61 | 98.49 | 99.35 |
| diluted 81 times | 42.56 | 99.64 | 90.40 | 99.67 |
| diluted 243 times | 3.39 | 84.56 | 69.03 | 99.41 |

**Table 2. Neutralization percentage of HIV neutralizing antibody lentiviral gene therapy vector-transduced 293T cell culture supernatant against virus**

| **HIV strains** | **Cell culture supernatant volume** | **KL-BsHIV01-003-02** | **KL-BsHIV01-003** |
|---|---|---|---|
| pAD-8 | 2 µL | 97.61 | 83.06 |
| | 5 µL | 99.80 | 96.32 |
| | 10 µL | 99.87 | 98.94 |
| pNL4-3 | 2 µL | 98.28 | 98.47 |
| | 5 µL | 98.69 | 98.77 |
| | 10 µL | 98.65 | 98.83 |

### VII. Functional verification of HIV neutralizing antibodies expressed in the supernatant of cells transduced with adeno-associated virus gene therapy vectors

C2C12 myoblasts were plated in 24-well cell culture plate at 1E5 cells per well, and differentiated into myotube cells using 2% horse serum medium to differentiate into myotube cells. The packaged adeno-associated viruses pAAV-CBH-KL-BsHIV01, pAAV-CBH-KL-BsHIV01-02, pAAV-CBH-KL-BsHIV01-003, and pAAV-CBH-KL-BsHIV01-003-02 were used to infect differentiated C2C12 cells at different MOIs. The supernatant was collected after 96 hours.

TZM-bl cells was plated at 2E4 cells/well. The cell expression supernatant of KL-BsHIV01, KL-BsHIV01-02, KL-BsHIV01-003, and KL-BsHIV01-003-02 with the same MOI of 8E4 vg were taken, the same volume of the cell expression supernatant of the amphotropic antibody KL-BsHIV01-02 and the tritropic antibody KL-BsHIV01-003-02 without the Fc segment as the amphotropic antibody KL-BsHIV01 and the tritropic antibody KL-BsHIV01-003 with the Fc segment were taken respectively, mixed with 50 µL of HIV pAD-8 or pNL4-3, incubated at 37°C for 30 minutes, and added to TZM-bl cells. The wells where only pAD-8 or pNL4-3 was added were negative controls (NC), and the well where HIV was not added was blank control. At 24 hours, the supernatant was discarded, and 100 µL cell lysis solution was added. After 10 minutes, the lysed cells was collected, and centrifuged at 8000 rpm for 5 minutes. 100 µL of firefly luciferase detection reagent was added to 50 µL of lysed cells. RLU (relative light unit) was measured using a multifunctional microplate reader with chemiluminescence function. The results showed (Table 3) that in *in vitro* cell experiments, under the same MOI, scFv monomers KL-BsHIV01-02 and KL-BsHIV01-003-02 without Fc segment had significantly better neutralizing effect on HIV than KL-BsHIV01 and KL-BsHIV01-003 with Fc segment.

**Table 3. Neutralization percentage of HIV neutralizing antibody adeno-associated virus gene therapy vector-transduced C2C12 cell culture supernatant against virus**

| **HIV** | **Antibody concentration** | **KL-BsHIV01** | **KL-BsHIV01-02** | **KL-BsHIV01-003** | **KL-BsHIV01-003-02** |
|---|---|---|---|---|---|
| pAD-8 | 2.5 ng/mL | 68.63 | 100.01 | 98.22 | 100.04 |
| | 5 ng/mL | 88.50 | 100.03 | 99.78 | 100.04 |
| | 10 ng/mL | 96.58 | 99.99 | 100.00 | 100.00 |
| pNL4-3 | 2.5 ng/mL | 95.92 | 100.33 | 100.46 | 100.53 |
| | 5 ng/mL | 100.06 | 100.57 | 100.61 | 100.55 |
| | 10 ng/mL | 100.67 | 100.38 | 100.13 | 100.19 |

### VIII. Expression of adeno-associated virus gene therapy vector in BALB/c mice after intramuscular injection

The HIV neutralizing antibody AAV gene therapy vector (pAAV-CBH-KL-BsHIV01-02-WPRE and pAAV-CBH-KL-BsHIV01-003-02-WPRE) was injected intramuscularly into the thigh muscles of the hind limbs of mice at a dose of 2E1 1 vg/mouse. Blood was taken every 1 week and serum was separated.

TZM-bl cells were plated at 2E4 cells/well. The same volume of serum dilutions were mixed with 50 µL of HIV pAD-8 or pNL4-3, incubated at 37°C for 30 minutes, and added to TZM-bl cells. The wells where only pAD-8 or pNL4-3 was added were negative controls (NC), and the well where HIV was not added was blank control. At 24 hours, the supernatant was discarded, and 100 µL cell lysis solution was added. After 10 minutes, the lysed cells were collected, and centrifuged at 8000 rpm for 5 minutes. 100 µL of firefly luciferase detection reagent was added to 50 µL of lysed cells. RLU (relative light unit) was measured using a multifunctional microplate reader with chemiluminescence function. The results showed (Table 4) that in *in vitro* cell experiments, after adding the same volume of HIV neutralizing antibodies, scFv monomers KL-BsHIV01-02 and KL-BsHIV01-003-02 without Fc segment of the IgG1 constant region had significantly better neutralizing effect on HIV than KL-BsHIV01 and KL-BsHIV01-003 with Fc segment (which are anti-HIV neutralizing antibody-like constructs in present inventor's another patent application).

**Table 4. Neutralization percentage of serum of mice injected intramuscularly with HIV neutralizing antibody adeno-associated virus gene therapy vector against virus**

| **HIV** | **Serum stock volume** | **KL-BsHIV01** | **KL-BsHIV01-02** | **KL-BsHIV01-003** | **KL-BsHIV01-003-02** |
|---|---|---|---|---|---|
| pAD-8 | 0.5 µL | 14.51 | 81.68 | 30.64 | 62.53 |
| | 0.05 µL | -12.10 | -4.43 | 5.64 | -6.85 |
| | 0.005 µL | -3.63 | -15.72 | 5.24 | -11.69 |
| pNL4-3 | 0.5 µL | 43.03 | 100.49 | 85.79 | 99.44 |
| | 0.05 µL | 16.57 | 78.98 | 34.21 | 56.66 |
| | 0.005 µL | 12.56 | 27.66 | 2.00 | 23.39 |

### IX. Identification of cells expressing HIV neutralizing antibodies

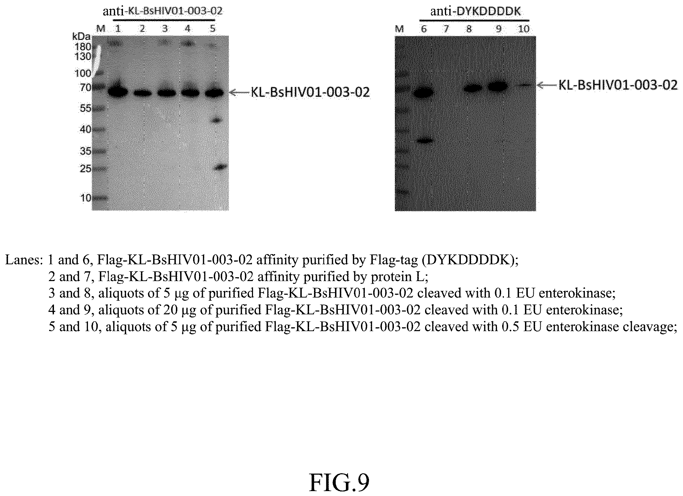

The lentiviral plasmid carrying Flag-KL-BsHIV01-003-02 was transiently transfected into 293T cells. After 48 hours, the supernatant was collected and Flag-KL-BsHIV01-003-02 was purified with anti-DYKDDDDK (i.e. Flag-tag) G1 Affinity Resin (GenScript, L00432-10). The obtained Flag-KL-BsHIV01-003-02 was digested with enterokinase (Nearshore, PE001-01A). Flag-KL-BsHIV01-003-02 before and after enzyme digestion was identified by Western blotting with rabbit Anti-KL-BsHIV01-003-02 or mouse Anti-DYKDDDDK (Genscript, A00187-100) (Figure 9). Western blotting results confirmed that the HIV neutralizing antibody KL-BsHIV01-003-02 with or without Flag-tag was expressed in cells and secreted into the supernatant, and had a molecular weight consistent with the prediction.

### X. Identification of activity of purified HIV neutralizing antibodies expressed in cells

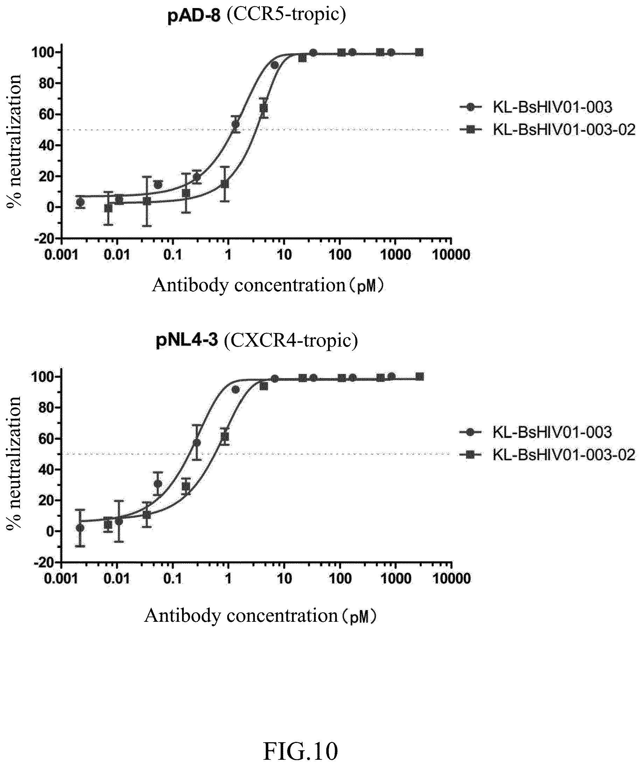

TZM-bl cells were plated at 2E4 cells/well. The dilutions of the purified antibodies were mixed with 50 µL of HIV pAD-8 or pNL4-3, incubated at 37°C for 30 minutes, and added to TZM-bl cells. The wells where only pAD-8 or pNL4-3 was added were negative controls, and the well where HIV was not added was blank control. After 24 hours of culture, the supernatant was discarded, and 100 µL cell lysis solution was added. After 10 minutes, the lysed cells were collected, and centrifuged at 8000 rpm for 5 minutes. 100 µL of firefly luciferase detection reagent was added to 50 µL of lysed cells. RLU (relative light unit) was measured using a multifunctional microplate reader with chemiluminescence function. The results showed that in cell experiments, the anti-HIV tritropic neutralizing antibody without Fc fragment (KL-BsHIV01-003-02), similar to the tritropic neutralizing antibody with Fc fragment (KL-BsHIV01-003), had strong HIV-neutralizing activity (Figure 10), with the IC50s for neutralizing HIV-1 strain pAD-8 (CCR5 tropism) of 3.1 pM and 1.2 pM respectively, and the IC50s for neutralizing HIV-1 strain pNL4-3 (CXCR4 tropism) of 0.2 pM and 0.6 pM respectively. The two HIV neutralizing antibodies of the present invention have stronger neutralizing activity against HIV than the reported HIV broad-spectrum neutralizing antibodies.

### XI. Expression of adeno-associated virus gene therapy vector in BALB/c mice after intramuscular injection

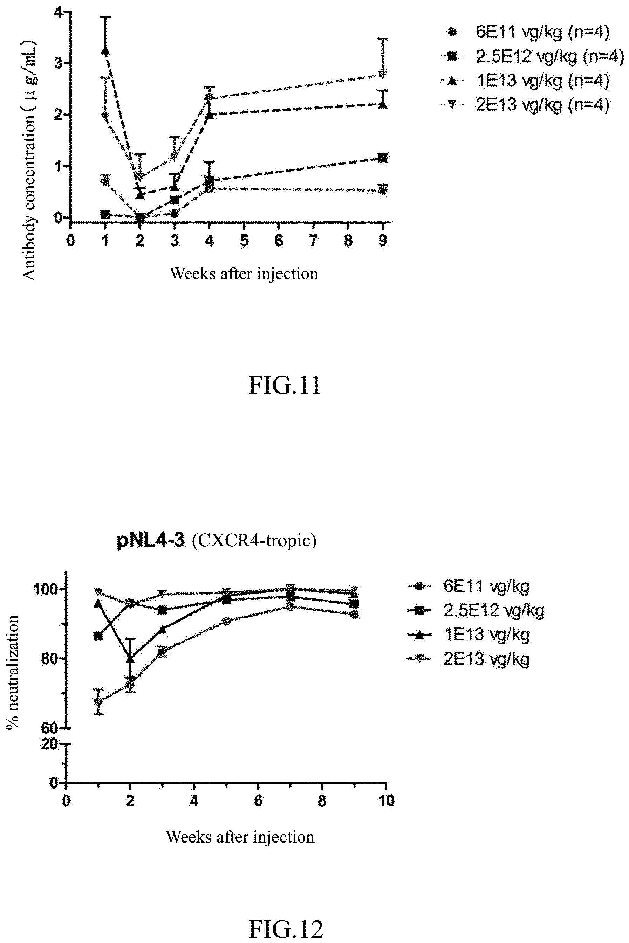

Different doses of AAV gene therapy vector (pAAV-CBH-KL-BsHIV01-003- -02WPRE) were injected intramuscularly into the thigh muscles of the hind limbs of mice. Blood was collected at regular intervals and the serum was separated.
1. The concentration of antibodies expressed in serum was detected by ELISA. The enzyme plate (Corning, Cat. No.: 42592) was coated with synthetic HIV MPER polypeptide. The expression supernatant and the purified and quantified KL-BsHIV01-003-02 standard were the primary antibody, rabbit anti-HIV membrane fusion-inhibiting short peptide was used as the secondary antibody, and HRP-labeled goat anti-rabbit IgG (KPL, 5220-0283) was used as the tertiary antibody. TMB was used for development, and the OD value at 450 nm was detected with a microplate reader. The ELISA results (Figure 11) showed that the anti-HIV neutralizing antibody adeno-associated virus vector could continuously and effectively express HIV neutralizing antibodies in mice.
2. TZM-bl cells were plated at 2E4 cells/well. A 200-fold serum dilution solution and 50 µL of HIV strain pNL4-3 were mixed, incubated at 37°C for 30 minutes, and added to TZM-bl cells. The wells where only pNL4-3 was added were negative controls, and the well where HIV was not added was blank control. After overnight culture, the supernatant was discarded, and 100 µL cell lysis solution was added. After 10 minutes, the lysed cells were collected and centrifuged at 8000 rpm for 5 minutes. 100 µL of firefly luciferase detection reagent was added to 50 µL of lysed cells. RLU (relative light unit) was measured using a multifunctional microplate reader with chemiluminescence function. The results showed that in cell experiments, HIV neutralizing antibodies expressed in mouse serum had excellent neutralizing activity against HIV strain pNL4-3 after 200-fold dilution, and the activity increased with increasing dose (Figure 12).

SEQ ID NO: 1
   MARPLCTLLLLMATLAGALA
SEQ ID NO: 2 
SELTQDPAVSVALKQTVTITCRGDSLRSHYASWYQKKPGQAPVLLFYGKNNR
PSGIPDRFSGSASGNRASLTITGAQAEDEADYYCSSRDKSGSRLSVFGGGTKL
TVLGGGGSGGGGSGGGGSEVRLRESGGGLVKPGGSLRLSCSASGFDFDNAW
MTWVRQPPGKGLEWVGRITGPGEGWSVDYAESVKGRFTISRDNTKNTLYLE
MNNVRTEDTGYYFCARTGKYYDFWFGYPPGEEYFQDWGQGTLVIVSS
SEQ ID NO: 3 
DIVMTQSPDSLAVSLGERVTMNCKSSQSLLYSTNQKNYLAWYQQKPGQSPK
LLIYWASTRESGVPDRFSGSGSGTDFTLTISSVQAEDVAVYYCQQYYSYRTFG
GGTKLEIKRTVAGGGGSGGGGSGGGGSQVQLQQSGPEVVKPGASVKMSCKA
SGYTFTSYVIHWVRQKPGQGLDWIGYINPYNDGTDYDEKFKGKATLTSDTST
STAYMELSSLRSEDTAVYYCAREKDNYATGAWFAYWGQGTLVTVSS
SEQ ID NO: 4
   WEQKIEELLKKAEEQQKKNEEELKKLEK
SEQ ID NO: 5
   YTSLIHSLIEESQNQQEKNEQELLELDKWASLWNWF
SEQ ID NO: 6
   LLEQENKEQQNQSEEILSHILSTYNNIERDWEMW
SEQ ID NO: 7 
MARPLCTLLLLMATLAGALASELTQDPAVSVALKQTVTITCRGDSLRSHYAS
WYQKKPGQAPVLLFYGKNNRPSGIPDRFSGSASGNRASLTITGAQAEDEADY
YCSSRDKSGSRLSVFGGGTKLTVLGGGGSGGGGSGGGGSEVRLRESGGGLVK
PGGSLRLSCSASGFDFDNAWMTWVRQPPGKGLEWVGRITGPGEGWSVDYAE
SVKGRFTISRDNTKNTLYLEMNNVRTEDTGYYFCARTGKYYDFWFGYPPGEE
YFQDWGQGTLVIVSSGGGGSGGGGSGGGGSGGGGSGGGGSDIVMTQSPDSL
AVSLGERVTMNCKSSQSLLYSTNQKNYLAWYQQKPGQSPKLLIYWASTRES
GVPDRFSGSGSGTDFTLTISSVQAEDVAVYYCQQYYSYRTFGGGTKLEIKRTV
AGGGGSGGGGSGGGGSQVQLQQSGPEVVKPGASVKMSCKASGYTFTSYVIH
WVRQKPGQGLDWIGYINPYNDGTDYDEKFKGKATLTSDTSTSTAYMELSSLR
SEDTAVYYCAREKDNYATGAWFAYWGQGTLVTVSS
SEQ ID NO: 8 
ATGGCGAGACCCCTGTGCACATTACTTCTGTTGATGGCTACCCTGGCAGGC
GCCCTCGCCAGCGAGCTGACACAGGACCCTGCCGTGTCCGTGGCCCTGAA
GCAGACCGTGACAATCACCTGCAGAGGCGATTCCCTGAGATCCCACTACG
CCTCCTGGTACCAGAAGAAGCCTGGCCAGGCCCCCGTGCTGCTGTTTTACG
GCAAGAATAACCGCCCCAGCGGCATCCCCGATAGATTTTCCGGCAGCGCC
TCCGGCAACAGAGCCAGCCTGACAATCACCGGCGCCCAGGCCGAGGACGA
GGCTGATTACTACTGCAGCTCCAGAGATAAGAGCGGCAGCAGACTGTCCG
TGTTTGGCGGCGGCACCAAGCTGACCGTGCTCGGAGGAGGAGGAAGCGGA
GGAGGAGGCTCAGGCGGCGGCGGCTCTGAGGTGAGGCTGAGAGAGTCCG
GCGGCGGCCTGGTGAAGCCCGGAGGATCTCTGAGGCTGTCCTGCTCCGCC
TCCGGCTTCGATTTTGACAATGCCTGGATGACCTGGGTGAGACAGCCCCCT
GGCAAGGGCCTGGAGTGGGTGGGAAGGATCACAGGCCCCGGCGAGGGCT
GGTCCGTGGATTACGCCGAGTCCGTGAAGGGCAGGTTCACAATCTCCAGG
 
GATAACACCAAGAACACCCTGTACCTGGAGATGAACAACGTGAGGACAGA
GGATACCGGCTACTACTTTTGCGCCAGAACAGGCAAGTACTACGACTTTTG
GTTCGGCTACCCCCCTGGCGAGGAGTACTTCCAGGATTGGGGCCAGGGCA
CCCTGGTCATTGTGTCCAGCGGCGGCGGCGGCAGTGGCGGCGGCGGAAGC
GGCGGCGGCGGCTCTGGCGGCGGCGGCAGCGGCGGCGGCGGCTCCGACAT
CGTGATGACCCAGTCTCCTGATAGCCTGGCCGTGAGCCTGGGCGAGAGAG
TGACAATGAACTGTAAGTCTAGCCAGAGCCTGCTGTACTCCACCAACCAG
AAGAATTACCTGGCCTGGTATCAGCAGAAGCCTGGCCAGTCCCCAAAGCT
GCTGATCTATTGGGCATCTACAAGGGAGAGCGGAGTGCCAGACAGATTCA
GCGGATCCGGATCTGGAACCGACTTCACCCTGACAATCTCCTCTGTGCAGG
CCGAGGACGTGGCCGTGTACTATTGCCAGCAGTACTATAGCTACAGGACA
TTCGGCGGCGGCACCAAGCTGGAGATCAAGCGCACCGTGGCCGGAGGAGG
AGGATCTGGCGGAGGAGGGTCCGGCGGCGGCGGCTCCCAGGTGCAGCTGC
AGCAGAGCGGACCAGAGGTGGTGAAGCCTGGAGCCTCCGTGAAGATGTCT
TGTAAGGCCAGCGGCTACACCTTCACATCCTATGTGATCCACTGGGTGAGG
CAGAAGCCAGGACAGGGACTGGACTGGATCGGCTACATCAACCCTTATAA
TGATGGCACCGACTACGATGAGAAGTTTAAGGGCAAGGCCACCCTGACAT
CCGATACCAGCACATCCACCGCCTATATGGAGCTGAGCTCCCTGCGGTCTG
AGGACACAGCCGTGTACTATTGCGCCAGAGAGAAGGATAACTACGCAACC
GGAGCATGGTTCGCATATTGGGGACAGGGTACCCTGGTCACCGTGTCTAG
CTAG
SEQ ID NO: 9 
MARPLCTLLLLMATLAGALASELTQDPAVSVALKQTVTITCRGDSLRSHYAS
WYQKKPGQAPVLLFYGKNNRPSGIPDRFSGSASGNRASLTITGAQAEDEADY
YCSSRDKSGSRLSVFGGGTKLTVLGGGGSGGGGSGGGGSEVRLRESGGGLVK
PGGSLRLSCSASGFDFDNAWMTWVRQPPGKGLEWVGRITGPGEGWSVDYAE
SVKGRFTISRDNTKNTLYLEMNNVRTEDTGYYFCARTGKYYDFWFGYPPGEE
YFQDWGQGTLVIVSSGGGGSGGGGSGGGGSGGGGSGGGGSDIVMTQSPDSL
AVSLGERVTMNCKSSQSLLYSTNQKNYLAWYQQKPGQSPKLLIYWASTRES
GVPDRFSGSGSGTDFTLTISSVQAEDVAVYYCQQYYSYRTFGGGTKLEIKRTV
AGGGGSGGGGSGGGGSQVQLQQSGPEVVKPGASVKMSCKASGYTFTSYVIH
WVRQKPGQGLDWIGYINPYNDGTDYDEKFKGKATLTSDTSTSTAYMELSSLR
SEDTAVYYCAREKDNYATGAWFAYWGQGTLVTVSSGGGGSGGGGSGGGGS
GGGGSGGGGSWEQKIEELLKKAEEQQKKNEEELKKLEK
SEQ ID NO: 10 
ATGGCGAGACCCCTGTGCACATTACTTCTGTTGATGGCTACCCTGGCAGGC
GCCCTCGCCAGCGAGCTGACACAGGACCCTGCCGTGTCCGTGGCCCTGAA
GCAGACCGTGACAATCACCTGCAGAGGCGATTCCCTGAGATCCCACTACG
CCTCCTGGTACCAGAAGAAGCCTGGCCAGGCCCCCGTGCTGCTGTTTTACG
GCAAGAATAACCGCCCCAGCGGCATCCCCGATAGATTTTCCGGCAGCGCC
TCCGGCAACAGAGCCAGCCTGACAATCACCGGCGCCCAGGCCGAGGACGA
GGCTGATTACTACTGCAGCTCCAGAGATAAGAGCGGCAGCAGACTGTCCG
TGTTTGGCGGCGGCACCAAGCTGACCGTGCTCGGAGGAGGAGGAAGCGGA
GGAGGAGGCTCAGGCGGCGGCGGCTCTGAGGTGAGGCTGAGAGAGTCCG
GCGGCGGCCTGGTGAAGCCCGGAGGATCTCTGAGGCTGTCCTGCTCCGCC
TCCGGCTTCGATTTTGACAATGCCTGGATGACCTGGGTGAGACAGCCCCCT
 
GGCAAGGGCCTGGAGTGGGTGGGAAGGATCACAGGCCCCGGCGAGGGCT
GGTCCGTGGATTACGCCGAGTCCGTGAAGGGCAGGTTCACAATCTCCAGG
GATAACACCAAGAACACCCTGTACCTGGAGATGAACAACGTGAGGACAGA
GGATACCGGCTACTACTTTTGCGCCAGAACAGGCAAGTACTACGACTTTTG
GTTCGGCTACCCCCCTGGCGAGGAGTACTTCCAGGATTGGGGCCAGGGCA
CCCTGGTCATTGTGTCCAGCGGCGGCGGCGGCAGTGGCGGCGGCGGAAGC
GGCGGCGGCGGCTCTGGCGGCGGCGGCAGCGGCGGCGGCGGCTCCGACAT
CGTGATGACCCAGTCTCCTGATAGCCTGGCCGTGAGCCTGGGCGAGAGAG
TGACAATGAACTGTAAGTCTAGCCAGAGCCTGCTGTACTCCACCAACCAG
AAGAATTACCTGGCCTGGTATCAGCAGAAGCCTGGCCAGTCCCCAAAGCT
GCTGATCTATTGGGCATCTACAAGGGAGAGCGGAGTGCCAGACAGATTCA
GCGGATCCGGATCTGGAACCGACTTCACCCTGACAATCTCCTCTGTGCAGG
CCGAGGACGTGGCCGTGTACTATTGCCAGCAGTACTATAGCTACAGGACA
TTCGGCGGCGGCACCAAGCTGGAGATCAAGCGCACCGTGGCCGGAGGAGG
AGGATCTGGCGGAGGAGGGTCCGGCGGCGGCGGCTCCCAGGTGCAGCTGC
AGCAGAGCGGACCAGAGGTGGTGAAGCCTGGAGCCTCCGTGAAGATGTCT
TGTAAGGCCAGCGGCTACACCTTCACATCCTATGTGATCCACTGGGTGAGG
CAGAAGCCAGGACAGGGACTGGACTGGATCGGCTACATCAACCCTTATAA
TGATGGCACCGACTACGATGAGAAGTTTAAGGGCAAGGCCACCCTGACAT
CCGATACCAGCACATCCACCGCCTATATGGAGCTGAGCTCCCTGCGGTCTG
AGGACACAGCCGTGTACTATTGCGCCAGAGAGAAGGATAACTACGCAACC
GGAGCATGGTTCGCATATTGGGGACAGGGTACCCTGGTCACCGTGTCTAG
CGGCGGAGGAGGAAGCGGAGGAGGCGGCTCTGGCGGAGGAGGTTCCGGA
GGAGGCGGAAGCGGCGGAGGAGGCTCTTGGGAGCAGAAGATCGAGGAGC
TGCTGAAGAAGGCCGAGGAGCAGCAGAAGAAGAATGAGGAGGAGCTGAA
GAAGCTGGAGAAGTAG
SEQ ID NO: 11 
GGCCGCGTTGCTGGCGTTTTTCCATAGGCTCCGCCCCCCTGACGAGCATCA
CAAAAATCGACGCTCAAGTCAGAGGTGGCGAAACCCGACAGGACTATAAA
GATACCAGGCGTTTCCCCCTGGAAGCTCCCTCGTGCGCTCTCCTGTTCCGA
CCCTGCCGCTTACCGGATACCTGTCCGCCTTTCTCCCTTCGGGAAGCGTGG
CGCTTTCTCATAGCTCACGCTGTAGGTATCTCAGTTCGGTGTAGGTCGTTC
GCTCCAAGCTGGGCTGTGTGCACGAACCCCCCGTTCAGCCCGACCGCTGC
GCCTTATCCGGTAACTATCGTCTTGAGTCCAACCCGGTAAGACACGACTTA
TCGCCACTGGCAGCAGCCACTGGTAACAGGATTAGCAGAGCGAGGTATGT
AGGCGGTGCTACAGAGTTCTTGAAGTGGTGGCCTAACTACGGCTACACTA
GAAGAACAGTATTTGGTATCTGCGCTCTGCTGAAGCCAGTTACCTTCGGAA
AAAGAGTTGGTAGCTCTTGATCCGGCAAACAAACCACCGCTGGTAGCGGT
GGTTTTTTTGTTTGCAAGCAGCAGATTACGCGCAGAAAAAAAGGATCTCA
AGAAGATCCTTTGATCTTTTCTACGGGGTCTGACGCTCAGTGGAACGAAAA
CTCACGTTAAGGGATTTTGGTCATGAAGCGCTTTTGAAGCTCGGATCCGAA
CAAACGACCCAACACCCGTGCGTTTTATTCTGTCTTTTTATTGCCGATCCC
CTCAGAAGAACTCGTCAAGAAGGCGATAGAAGGCGATGCGCTGCGAATCG
GGAGCGGCGATACCGTAAAGCACGAGGAAGCGGTCAGCCCATTCGCCGCC
AAGCTCTTCAGCAATATCACGGGTAGCCAACGCTATGTCCTGATAGCGGTC
CGCCACACCCAGCCGGCCACAGTCGATGAATCCAGAAAAGCGGCCATTTT
 
CCACCATGATATTCGGCAAGCAGGCATCGCCATGGGTCACGACGAGATCC
TCGCCGTCGGGCATGCTCGCCTTGAGCCTGGCGAACAGTTCGGCTGGCGC
GAGCCCCTGATGCTCTTCGTCCAGATCATCCTGATCGACAAGACCGGCTTC
CATCCGAGTACGTGCTCGCTCGATGCGATGTTTCGCTTGGTGGTCGAATGG
GCAGGTAGCCGGATCAAGCGTATGCAGCCGCCGCATTGCATCAGCCATGA
TGGATACTTTCTCGGCAGGAGCAAGGTGAGATGACAGGAGATCCTGCCCC
GGCACTTCGCCCAATAGCAGCCAGTCCCTTCCCGCTTCAGTGACAACGTCG
AGCACAGCTGCGCAAGGAACGCCCGTCGTGGCCAGCCACGATAGCCGCGC
TGCCTCGTCTTGCAGTTCATTCAGGGCACCGGACAGGTCGGTCTTGACAAA
AAGAACCGGGCGCCCCTGCGCTGACAGCCGGAACACGGCGGCATCAGAGC
AGCCGATTGTCTGTTGTGCCCAGTCATAGCCGAATAGCCTCTCCACCCAAG
CGGCCGGAGAACCTGCGTGCAATCCATCTTGTTCAATCATGCGAAACGAT
CCTCATCCTGTCTCTTGATCAGAGCTTGATCCCCTGCGCCATCAGATCCTT
GGCGGCAAGAAAGCCATCCAGTTTACTTTGCAGGGCTTCCCAACCTTACCA
GAGGCCTGCGCCGCGGCCAGCTGGCTAGCAATTCCCGGGTTAACTCTAGA
GACATTGATTATTGACTAGTTATTAATAGTAATCAATTACGGGGTCATTAG
TTCATAGCCCATATATGGAGTTCCGCGTTACATAACTTACGGTAAATGGCC
CGCCTGGCTGACCGCCCAACGACCCCCGCCCATTGACGTCAATAATGACG
TATGTTCCCATAGTAACGCCAATAGGGACTTTCCATTGACGTCAATGGGTG
GAGTATTTACGGTAAACTGCCCACTTGGCAGTACATCAAGTGTATCATATG
CCAAGTACGCCCCCTATTGACGTCAATGACGGTAAATGGCCCGCCTGGCA
TTATGCCCAGTACATGACCTTATGGGACTTTCCTACTTGGCAGTACATCTA
CGTATTAGTCATCGCTATTACCATGGTGATGCGGTTTTGGCAGTACATCAA
TGGGCGTGGATAGCGGTTTGACTCACGGGGATTTCCAAGTCTCCACCCCAT
TGACGTCAATGGGAGTTTGTTTTGGCACCAAAATCAACGGGACTTTCCAAA
ATGTCGTAACAACTCCGCCCCATTGACGCAAATGGGCGGTAGGCGTGTAC
GGTGGGAGGTCTATATAAGCAGAGCTCGTTTAGTGAACCGGGGTCTCTCT
GGTTAGACCAGATCTGAGCCTGGGAGCTCTCTGGCTAACTAGGGAACCCA
CTGCTTAAGCCTCAATAAAGCTTGCCTTGAGTGCTTCAAGTAGTGTGTGCC
CGTCTGTTGTGTGACTCTGGTAACTAGAGATCCCTCAGACCCTTTTAGTCA
GTGTGGAAAATCTCTAGCAGTGGCGCCCGAACAGGGACTTGAAAGCGAAA
GGGAAACCAGAGGAGCTCTCTCGACGCAGGACTCGGCTTGCTGAAGCGCG
CACGGCAAGAGGCGAGGGGCGGCGACTGGTGAGTACGCCAAAAATTTTGA
CTAGCGGAGGCTAGAAGGAGAGAGATGGGTGCGAGAGCGTCAGTATTAA
GCGGGGGAGAATTAGATCGCGATGGGAAAAAATTCGGTTAAGGCCAGGG
GGAAAGAAAAAATATAAATTAAAACATATAGTATGGGCAAGCAGGGAGC
TAGAACGATTCGCAGTTAATCCTGGCCTGTTAGAAACATCAGAAGGCTGT
AGACAAATACTGGGACAGCTACAACCATCCCTTCAGACAGGATCAGAAGA
ACTTAGATCATTATATAATACAGTAGCAACCCTCTATTGTGTGCATCAAAG
GATAGAGATAAAAGACACCAAGGAAGCTTTAGACAAGATAGAGGAAGAG
CAAAACAAAAGTAAGACCACCGCACAGCAAGCGGCCGCTGATCTTCAGAC
CTGGAGGAGGAGATATGAGGGACAATTGGAGAAGTGAATTATATAAATAT
AAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCC
TTGGGTTCTTGGGAGCAGCAGGAAGCACTATGGGCGCAGCGTCAATGACG
CTGACGGTACAGGCCAGACAATTATTGTCTGGTATAGTGCAGCAGCAGAA
 
CAATTTGCTGAGGGCTATTGAGGCGCAACAGCATCTGTTGCAACTCACAGT
CTGGGGCATCAAGCAGCTCCAGGCAAGAATCCTGGCTGTGGAAAGATACC
TAAAGGATCAACAGCTCCTGGGGATTTGGGGTTGCTCTGGAAAACTCATTT
GCACCACTGCTGTGCCTTGGAATGCTAGTTGGAGTAATAAATCTCTGGAAC
AGATTTGGAATCACACGACCTGGATGGAGTGGGACAGAGAAATTAACAAT
TACACAAGCTTAATACACTCCTTAATTGAAGAATCGCAAAACCAGCAAGA
AAAGAATGAACAAGAATTATTGGAATTAGATAAATGGGCAAGTTTGTGGA
ATTGGTTTAACATAACAAATTGGCTGTGGTATATAAAATTATTCATAATGA
TAGTAGGAGGCTTGGTAGGTTTAAGAATAGTTTTTGCTGTACTTTCTATAG
TGAATAGAGTTAGGCAGGGATATTCACCATTATCGTTTCAGACCCACCTCC
CAACCCCGAGGGGACCCGACAGGCCCGAAGGAATAGAAGAAGAAGGTGG
AGAGAGAGACAGAGACAGATCCATTCGATTAGTGAACGGATCTCGACGGT
ATCGGTTAACTTTTAAAAGAAAAGGGGGGATTGGGGGGTACAGTGCAGGG
GAAAGAATAGTAGACATAATAGCAACAGACATACAAACTAAAGAATTACA
AAAACAAATTACAAAAATTCAAAATTTTATCGATCACGAGACTAGCCTCG
AGGCATGCCTGCAGGAATTCCGTTACATAACTTACGGTAAATGGCCCGCCT
GGCTGACCGCCCAACGACCCCCGCCCATTGACGTCAATAGTAACGCCAAT
AGGGACTTTCCATTGACGTCAATGGGTGGAGTATTTACGGTAAACTGCCCA
CTTGGCAGTACATCAAGTGTATCATATGCCAAGTACGCCCCCTATTGACGT
CAATGACGGTAAATGGCCCGCCTGGCATTGTGCCCAGTACATGACCTTATG
GGACTTTCCTACTTGGCAGTACATCTACGTATTAGTCATCGCTATTACCAT
GGTCGAGGTGAGCCCCACGTTCTGCTTCACTCTCCCCATCTCCCCCCCCTC
CCCACCCCCAATTTTGTATTTATTTATTTTTTAATTATTTTGTGCAGCGATG
GGGGCGGGGGGGGGGGGGGCGCGCCAGGCGGGGCGGGGCGGGGCG
AGGGGCGGGGCGGGGCGAGGCGGAGAGGTGCGGCGGCAGCCAATCAGAG
CGGCGCGCTCCGAAAGTTTCCTTTTATGGCGAGGCGGCGGCGGCGGC
CCTATAAAAAGCGAAGCGCGCGGCGGGCGGGAGTCGCTGCGACGCTGCCT
TCGCCCCGTGCCCCGCTCCGCCGCCGCCTCGCGCCGCCCGCCCCGGCTCTG
ACTGACCGCGTTACTCCCACAGGTGAGCGGGCGGGACGGCCCTTCTCCTCC
GGGCTGTAATTAGCTGAGCAAGAGGTAAGGGTTTAAGGGATGGTTGGTTG
GTGGGGTATTAATGTTTAATTACCTGGAGCACCTGCCTGAAATCACTTTTT
TTCAGGTTGGACCGGTACGCGTGCCTCGGATCCTCCAGTGTGGTGTGCAGA
TATCCAGCACAGTCCCGGGCCGAGTCTAGACGTTTAAACCCGCTGATCAG
GTCGACAATCAACCTCTGGATTACAAAATTTGTGAAAGATTGACTGGTATT
CTTAACTATGTTGCTCCTTTTACGCTATGTGGATACGCTGCTTTAATGCCTT
TGTATCATGCTATTGCTTCCCGTATGGCTTTCATTTTCTCCTCCTTGTATAA
ATCCTGGTTGCTGTCTCTTTATGAGGAGTTGTGGCCCGTTGTCAGGCAACG
TGGCGTGGTGTGCACTGTGTTTGCTGACGCAACCCCCACTGGTTGGGGCAT
TGCCACCACCTGTCAGCTCCTTTCCGGGACTTTCGCTTTCCCCCTCCCTATT
GCCACGGCGGAACTCATCGCCGCCTGCCTTGCCCGCTGCTGGACAGGGGC
TCGGCTGTTGGGCACTGACAATTCCGTGGTGTTGTCGGGGAAGCTGACGTC
CTTTCCATGGCTGCTCGCCTGTGTTGCCACCTGGATTCTGCGCGGGACGTC
CTTCTGCTACGTCCCTTCGGCCCTCAATCCAGCGGACCTTCCTTCCCGCGG
CCTGCTGCCGGCTCTGCGGCCTCTTCCGCGTCTTCGCCTTCGCCCTCAGAC
GAGTCGGATCTCCCTTTGGGCCGCCTCCCCGCGGTACCTTTAAGACCAATG
ACTTACAAGGCAGCTGTAGATCTTAGCCACTTTTTAAAAGAAAAGGGGGG
 
ACTGGAAGGGCTAATTCACTCCCAACGAAGACAAGATCTGCTTTTTGCTTG
TACTGGGTCTCTCTGGTTAGACCAGATCTGAGCCTGGGAGCTCTCTGGCTA
ACTAGGGAACCCACTGCTTAAGCCTCAATAAAGCTTGCCTTGAGTGCTTCA
AGTAGTGTGTGCCCGTCTGTTGTGTGACTCTGGTAACTAGAGATCCCTCAG
ACCCTTTTAGTCAGTGTGGAAAATCTCTAGCAGTAGTAGTTCATGTCATCT
TATTATTCAGTATTTATAACTTGCAAAGAAATGAATATCAGAGAGTGAGA
GGAACTTGTTTATTGCAGCTTATAATGGTTACAAATAAAGCAATAGCATCA
CAAATTTCACAAATAAAGCATTTTTTTCACTGCATTCTAGTTGTGGTTTGTC
CAAACTCATCAATGTATCTTATCATGTCTGGCTCTAGCTATCCCGCCCCTA
ACTCCGCCCATCCCGCCCCTAACTCCGCCCAGTTCCGCCCATTCTCCGCCC
CATGGCTGACTAATTTTTTTTATTTATGCAGAGGCCGAGGCCGCCTCGGCC
TCTGAGCTATTCCAGAAGTAGTGAGGAGGCTTTTTTGGAGGCCGCTAGCGT
CGACCATTACTTATTGTTTTAGCTGTCCTCATGAATGTCTTTTCACTACCCA
TTTGCTTATCCTGCATCTCTCAGCCTTGACTCCACTCAGTTCTCTTGCTTAG
AGATACCACCTTTCCCCTGAAGTGTTCCTTCCATGTTTTACGGCGAGATGG
TTTCTCCTCGCCTGGCCACTCAGCCTTAGTTGTCTCTGTTGTCTTATAGAGG
TCTACTTGAAGAAGGAAAAACAGGGGGCATGGTTTGACTGTCCTGTGAGC
CCTTCTTCCCTGCCTCCCCCACTCACAGTGACCCGGAATCCCTCGACATGG
CAGTCTAGCACTAGTGCGGCCGCAGATCTGCTTCCTCGCTCACTGACTCGC
TGCGCTCGGTCGTTCGGCTGCGGCGAGCGGTATCAGCTCACTCAAAGGCG
GTAATACGGTTATCCACAGAATCAGGGGATAACGCAGGAAAGAACATGTG
AGCAAAAGGCCAGCAAAAGGCCAGGAACCGTAAAAA
SEQ ID NO: 12 
GGCCGCGTTGCTGGCGTTTTTCCATAGGCTCCGCCCCCCTGACGAGCATCA
CAAAAATCGACGCTCAAGTCAGAGGTGGCGAAACCCGACAGGACTATAAA
GATACCAGGCGTTTCCCCCTGGAAGCTCCCTCGTGCGCTCTCCTGTTCCGA
CCCTGCCGCTTACCGGATACCTGTCCGCCTTTCTCCCTTCGGGAAGCGTGG
CGCTTTCTCATAGCTCACGCTGTAGGTATCTCAGTTCGGTGTAGGTCGTTC
GCTCCAAGCTGGGCTGTGTGCACGAACCCCCCGTTCAGCCCGACCGCTGC
GCCTTATCCGGTAACTATCGTCTTGAGTCCAACCCGGTAAGACACGACTTA
TCGCCACTGGCAGCAGCCACTGGTAACAGGATTAGCAGAGCGAGGTATGT
AGGCGGTGCTACAGAGTTCTTGAAGTGGTGGCCTAACTACGGCTACACTA
GAAGAACAGTATTTGGTATCTGCGCTCTGCTGAAGCCAGTTACCTTCGGAA
AAAGAGTTGGTAGCTCTTGATCCGGCAAACAAACCACCGCTGGTAGCGGT
GGTTTTTTTGTTTGCAAGCAGCAGATTACGCGCAGAAAAAAAGGATCTCA
AGAAGATCCTTTGATCTTTTCTACGGGGTCTGACGCTCAGTGGAACGAAAA
CTCACGTTAAGGGATTTTGGTCATGAAGCGCTTTTGAAGCTCGGATCCGAA
CAAACGACCCAACACCCGTGCGTTTTATTCTGTCTTTTTATTGCCGATCCC
CTCAGAAGAACTCGTCAAGAAGGCGATAGAAGGCGATGCGCTGCGAATCG
GGAGCGGCGATACCGTAAAGCACGAGGAAGCGGTCAGCCCATTCGCCGCC
AAGCTCTTCAGCAATATCACGGGTAGCCAACGCTATGTCCTGATAGCGGTC
CGCCACACCCAGCCGGCCACAGTCGATGAATCCAGAAAAGCGGCCATTTT
CCACCATGATATTCGGCAAGCAGGCATCGCCATGGGTCACGACGAGATCC
TCGCCGTCGGGCATGCTCGCCTTGAGCCTGGCGAACAGTTCGGCTGGCGC
GAGCCCCTGATGCTCTTCGTCCAGATCATCCTGATCGACAAGACCGGCTTC
CATCCGAGTACGTGCTCGCTCGATGCGATGTTTCGCTTGGTGGTCGAATGG
 
GCAGGTAGCCGGATCAAGCGTATGCAGCCGCCGCATTGCATCAGCCATGA
TGGATACTTTCTCGGCAGGAGCAAGGTGAGATGACAGGAGATCCTGCCCC
GGCACTTCGCCCAATAGCAGCCAGTCCCTTCCCGCTTCAGTGACAACGTCG
AGCACAGCTGCGCAAGGAACGCCCGTCGTGGCCAGCCACGATAGCCGCGC
TGCCTCGTCTTGCAGTTCATTCAGGGCACCGGACAGGTCGGTCTTGACAAA
AAGAACCGGGCGCCCCTGCGCTGACAGCCGGAACACGGCGGCATCAGAGC
AGCCGATTGTCTGTTGTGCCCAGTCATAGCCGAATAGCCTCTCCACCCAAG
CGGCCGGAGAACCTGCGTGCAATCCATCTTGTTCAATCATGCGAAACGAT
CCTCATCCTGTCTCTTGATCAGAGCTTGATCCCCTGCGCCATCAGATCCTT
GGCGGCAAGAAAGCCATCCAGTTTACTTTGCAGGGCTTCCCAACCTTACCA
GAGGCCTGCGCCGCGGCCAGCTGGCTAGCAATTCCCGGGTTAACTCTAGA
GACATTGATTATTGACTAGTTATTAATAGTAATCAATTACGGGGTCATTAG
TTCATAGCCCATATATGGAGTTCCGCGTTACATAACTTACGGTAAATGGCC
CGCCTGGCTGACCGCCCAACGACCCCCGCCCATTGACGTCAATAATGACG
TATGTTCCCATAGTAACGCCAATAGGGACTTTCCATTGACGTCAATGGGTG
GAGTATTTACGGTAAACTGCCCACTTGGCAGTACATCAAGTGTATCATATG
CCAAGTACGCCCCCTATTGACGTCAATGACGGTAAATGGCCCGCCTGGCA
TTATGCCCAGTACATGACCTTATGGGACTTTCCTACTTGGCAGTACATCTA
CGTATTAGTCATCGCTATTACCATGGTGATGCGGTTTTGGCAGTACATCAA
TGGGCGTGGATAGCGGTTTGACTCACGGGGATTTCCAAGTCTCCACCCCAT
TGACGTCAATGGGAGTTTGTTTTGGCACCAAAATCAACGGGACTTTCCAAA
ATGTCGTAACAACTCCGCCCCATTGACGCAAATGGGCGGTAGGCGTGTAC
GGTGGGAGGTCTATATAAGCAGAGCTCGTTTAGTGAACCGGGGTCTCTCT
GGTTAGACCAGATCTGAGCCTGGGAGCTCTCTGGCTAACTAGGGAACCCA
CTGCTTAAGCCTCAATAAAGCTTGCCTTGAGTGCTTCAAGTAGTGTGTGCC
CGTCTGTTGTGTGACTCTGGTAACTAGAGATCCCTCAGACCCTTTTAGTCA
GTGTGGAAAATCTCTAGCAGTGGCGCCCGAACAGGGACTTGAAAGCGAAA
GGGAAACCAGAGGAGCTCTCTCGACGCAGGACTCGGCTTGCTGAAGCGCG
CACGGCAAGAGGCGAGGGGCGGCGACTGGTGAGTACGCCAAAAATTTTGA
CTAGCGGAGGCTAGAAGGAGAGAGATGGGTGCGAGAGCGTCAGTATTAA
GCGGGGGAGAATTAGATCGCGATGGGAAAAAATTCGGTTAAGGCCAGGG
GGAAAGAAAAAATATAAATTAAAACATATAGTATGGGCAAGCAGGGAGC
TAGAACGATTCGCAGTTAATCCTGGCCTGTTAGAAACATCAGAAGGCTGT
AGACAAATACTGGGACAGCTACAACCATCCCTTCAGACAGGATCAGAAGA
ACTTAGATCATTATATAATACAGTAGCAACCCTCTATTGTGTGCATCAAAG
GATAGAGATAAAAGACACCAAGGAAGCTTTAGACAAGATAGAGGAAGAG
CAAAACAAAAGTAAGACCACCGCACAGCAAGCGGCCGCTGATCTTCAGAC
CTGGAGGAGGAGATATGAGGGACAATTGGAGAAGTGAATTATATAAATAT
AAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCC
TTGGGTTCTTGGGAGCAGCAGGAAGCACTATGGGCGCAGCGTCAATGACG
CTGACGGTACAGGCCAGACAATTATTGTCTGGTATAGTGCAGCAGCAGAA
CAATTTGCTGAGGGCTATTGAGGCGCAACAGCATCTGTTGCAACTCACAGT
CTGGGGCATCAAGCAGCTCCAGGCAAGAATCCTGGCTGTGGAAAGATACC
TAAAGGATCAACAGCTCCTGGGGATTTGGGGTTGCTCTGGAAAACTCATTT
GCACCACTGCTGTGCCTTGGAATGCTAGTTGGAGTAATAAATCTCTGGAAC
 
AGATTTGGAATCACACGACCTGGATGGAGTGGGACAGAGAAATTAACAAT
TACACAAGCTTAATACACTCCTTAATTGAAGAATCGCAAAACCAGCAAGA
AAAGAATGAACAAGAATTATTGGAATTAGATAAATGGGCAAGTTTGTGGA
ATTGGTTTAACATAACAAATTGGCTGTGGTATATAAAATTATTCATAATGA
TAGTAGGAGGCTTGGTAGGTTTAAGAATAGTTTTTGCTGTACTTTCTATAG
TGAATAGAGTTAGGCAGGGATATTCACCATTATCGTTTCAGACCCACCTCC
CAACCCCGAGGGGACCCGACAGGCCCGAAGGAATAGAAGAAGAAGGTGG
AGAGAGAGACAGAGACAGATCCATTCGATTAGTGAACGGATCTCGACGGT
ATCGGTTAACTTTTAAAAGAAAAGGGGGGATTGGGGGGTACAGTGCAGGG
GAAAGAATAGTAGACATAATAGCAACAGACATACAAACTAAAGAATTACA
AAAACAAATTACAAAAATTCAAAATTTTATCGATCACGAGACTAGCCTCG
AGGCATGCCTGCAGGAATTCCGTTACATAACTTACGGTAAATGGCCCGCCT
GGCTGACCGCCCAACGACCCCCGCCCATTGACGTCAATAATGACGTATGTT
CCCATAGTAACGCCAATAGGGACTTTCCATTGACGTCAATGGGTGGAGTA
TTTACGGTAAACTGCCCACTTGGCAGTACATCAAGTGTATCATATGCCAAG
TACGCCCCCTATTGACGTCAATGACGGTAAATGGCCCGCCTGGCATTATGC
CCAGTACATGACCTTATGGGACTTTCCTACTTGGCAGTACATCTACGTATT
AGTCATCGCTATTACCATGGTCGAGGTGAGCCCCACGTTCTGCTTCACTCT
CCCCATCTCCCCCCCCTCCCCACCCCCAATTTTGTATTTATTTATTTTTTAA
TTATTTTGTGCAGCGATGGGGGCGGGGGGGGGGGGGGGGCGCGCGCCAGG
CGGGGCGGGGCGGGGCGAGGGGCGGGGCGGGGCGAGGCGGAGAGGTGCG
GCGGCAGCCAATCAGAGCGGCGCGCTCCGAAAGTTTCCTTTTATGGCGAG
GCGGCGGCGGCGGCGGCCCTATAAAAAGCGAAGCGCGCGGCGGGCGGGA
GTCGCTGCGACGCTGCCTTCGCCCCGTGCCCCGCTCCGCCGCCGCCTCGCG
CCGCCCGCCCCGGCTCTGACTGACCGCGTTACTCCCACAGGTGAGCGGGC
GGGACGGCCCTTCTCCTCCGGGCTGTAATTAGCTGAGCAAGAGGTAAGGG
TTTAAGGGATGGTTGGTTGGTGGGGTATTAATGTTTAATTACCTGGAGCAC
CTGCCTGAAATCACTTTTTTTCAGGTACCGGTGCCACCATGGCGAGACCCC
TGTGCACATTACTTCTGTTGATGGCTACCCTGGCAGGCGCCCTCGCCAGCG
AGCTGACACAGGACCCTGCCGTGTCCGTGGCCCTGAAGCAGACCGTGACA
ATCACCTGCAGAGGCGATTCCCTGAGATCCCACTACGCCTCCTGGTACCAG
AAGAAGCCTGGCCAGGCCCCCGTGCTGCTGTTTTACGGCAAGAATAACCG
CCCCAGCGGCATCCCCGATAGATTTTCCGGCAGCGCCTCCGGCAACAGAG
CCAGCCTGACAATCACCGGCGCCCAGGCCGAGGACGAGGCTGATTACTAC
TGCAGCTCCAGAGATAAGAGCGGCAGCAGACTGTCCGTGTTTGGCGGCGG
CACCAAGCTGACCGTGCTCGGAGGAGGAGGAAGCGGAGGAGGAGGCTCA
GGCGGCGGCGGCTCTGAGGTGAGGCTGAGAGAGTCCGGCGGCGGCCTGGT
GAAGCCCGGAGGATCTCTGAGGCTGTCCTGCTCCGCCTCCGGCTTCGATTT
TGACAATGCCTGGATGACCTGGGTGAGACAGCCCCCTGGCAAGGGCCTGG
AGTGGGTGGGAAGGATCACAGGCCCCGGCGAGGGCTGGTCCGTGGATTAC
GCCGAGTCCGTGAAGGGCAGGTTCACAATCTCCAGGGATAACACCAAGAA
CACCCTGTACCTGGAGATGAACAACGTGAGGACAGAGGATACCGGCTACT
ACTTTTGCGCCAGAACAGGCAAGTACTACGACTTTTGGTTCGGCTACCCCC
CTGGCGAGGAGTACTTCCAGGATTGGGGCCAGGGCACCCTGGTCATTGTG
TCCAGCGGCGGCGGCGGCAGTGGCGGCGGCGGAAGCGGCGGCGGCGGCT
CTGGCGGCGGCGGCAGCGGCGGCGGCGGCTCCGACATCGTGATGACCCAG
 
TCTCCTGATAGCCTGGCCGTGAGCCTGGGCGAGAGAGTGACAATGAACTG
TAAGTCTAGCCAGAGCCTGCTGTACTCCACCAACCAGAAGAATTACCTGG
CCTGGTATCAGCAGAAGCCTGGCCAGTCCCCAAAGCTGCTGATCTATTGG
GCATCTACAAGGGAGAGCGGAGTGCCAGACAGATTCAGCGGATCCGGATC
TGGAACCGACTTCACCCTGACAATCTCCTCTGTGCAGGCCGAGGACGTGG
CCGTGTACTATTGCCAGCAGTACTATAGCTACAGGACATTCGGCGGCGGC
ACCAAGCTGGAGATCAAGCGCACCGTGGCCGGAGGAGGAGGATCTGGCG
GAGGAGGGTCCGGCGGCGGCGGCTCCCAGGTGCAGCTGCAGCAGAGCGG
ACCAGAGGTGGTGAAGCCTGGAGCCTCCGTGAAGATGTCTTGTAAGGCCA
GCGGCTACACCTTCACATCCTATGTGATCCACTGGGTGAGGCAGAAGCCA
GGACAGGGACTGGACTGGATCGGCTACATCAACCCTTATAATGATGGCAC
CGACTACGATGAGAAGTTTAAGGGCAAGGCCACCCTGACATCCGATACCA
GCACATCCACCGCCTATATGGAGCTGAGCTCCCTGCGGTCTGAGGACACA
GCCGTGTACTATTGCGCCAGAGAGAAGGATAACTACGCAACCGGAGCATG
GTTCGCATATTGGGGACAGGGTACCCTGGTCACCGTGTCTAGCTAGGTCGA
CAATCAACCTCTGGATTACAAAATTTGTGAAAGATTGACTGGTATTCTTAA
CTATGTTGCTCCTTTTACGCTATGTGGATACGCTGCTTTAATGCCTTTGTAT
CATGCTATTGCTTCCCGTATGGCTTTCATTTTCTCCTCCTTGTATAAATCCT
GGTTGCTGTCTCTTTATGAGGAGTTGTGGCCCGTTGTCAGGCAACGTGGCG
TGGTGTGCACTGTGTTTGCTGACGCAACCCCCACTGGTTGGGGCATTGCCA
CCACCTGTCAGCTCCTTTCCGGGACTTTCGCTTTCCCCCTCCCTATTGCCAC
GGCGGAACTCATCGCCGCCTGCCTTGCCCGCTGCTGGACAGGGGCTCGGC
TGTTGGGCACTGACAATTCCGTGGTGTTGTCGGGGAAGCTGACGTCCTTTC
CATGGCTGCTCGCCTGTGTTGCCACCTGGATTCTGCGCGGGACGTCCTTCT
GCTACGTCCCTTCGGCCCTCAATCCAGCGGACCTTCCTTCCCGCGGCCTGC
TGCCGGCTCTGCGGCCTCTTCCGCGTCTTCGCCTTCGCCCTCAGACGAGTC
GGATCTCCCTTTGGGCCGCCTCCCCGCGGTACCTTTAAGACCAATGACTTA
CAAGGCAGCTGTAGATCTTAGCCACTTTTTAAAAGAAAAGGGGGGACTGG
AAGGGCTAATTCACTCCCAACGAAGACAAGATCTGCTTTTTGCTTGTACTG
GGTCTCTCTGGTTAGACCAGATCTGAGCCTGGGAGCTCTCTGGCTAACTAG
GGAACCCACTGCTTAAGCCTCAATAAAGCTTGCCTTGAGTGCTTCAAGTAG
TGTGTGCCCGTCTGTTGTGTGACTCTGGTAACTAGAGATCCCTCAGACCCT
TTTAGTCAGTGTGGAAAATCTCTAGCAGTAGTAGTTCATGTCATCTTATTA
TTCAGTATTTATAACTTGCAAAGAAATGAATATCAGAGAGTGAGAGGAAC
TTGTTTATTGCAGCTTATAATGGTTACAAATAAAGCAATAGCATCACAAAT
TTCACAAATAAAGCATTTTTTTCACTGCATTCTAGTTGTGGTTTGTCCAAAC
TCATCAATGTATCTTATCATGTCTGGCTCTAGCTATCCCGCCCCTAACTCC
GCCCATCCCGCCCCTAACTCCGCCCAGTTCCGCCCATTCTCCGCCCCATGG
CTGACTAATTTTTTTTATTTATGCAGAGGCCGAGGCCGCCTCGGCCTCTGA
GCTATTCCAGAAGTAGTGAGGAGGCTTTTTTGGAGGCCGCTAGCGTCGAC
CATTACTTATTGTTTTAGCTGTCCTCATGAATGTCTTTTCACTACCCATTTG
CTTATCCTGCATCTCTCAGCCTTGACTCCACTCAGTTCTCTTGCTTAGAGAT
ACCACCTTTCCCCTGAAGTGTTCCTTCCATGTTTTACGGCGAGATGGTTTCT
CCTCGCCTGGCCACTCAGCCTTAGTTGTCTCTGTTGTCTTATAGAGGTCTAC
TTGAAGAAGGAAAAACAGGGGGCATGGTTTGACTGTCCTGTGAGCCCTTC
TTCCCTGCCTCCCCCACTCACAGTGACCCGGAATCCCTCGACATGGCAGTC
 
TAGCACTAGTGCGGCCGCAGATCTGCTTCCTCGCTCACTGACTCGCTGCGC
TCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAA
AAGGCCAGCAAAAGGCCAGGAACCGTAAAAA
SEQ ID NO: 13 
GGCCGCGTTGCTGGCGTTTTTCCATAGGCTCCGCCCCCCTGACGAGCATCA
CAAAAATCGACGCTCAAGTCAGAGGTGGCGAAACCCGACAGGACTATAAA
GATACCAGGCGTTTCCCCCTGGAAGCTCCCTCGTGCGCTCTCCTGTTCCGA
CCCTGCCGCTTACCGGATACCTGTCCGCCTTTCTCCCTTCGGGAAGCGTGG
CGCTTTCTCATAGCTCACGCTGTAGGTATCTCAGTTCGGTGTAGGTCGTTC
GCTCCAAGCTGGGCTGTGTGCACGAACCCCCCGTTCAGCCCGACCGCTGC
GCCTTATCCGGTAACTATCGTCTTGAGTCCAACCCGGTAAGACACGACTTA
TCGCCACTGGCAGCAGCCACTGGTAACAGGATTAGCAGAGCGAGGTATGT
AGGCGGTGCTACAGAGTTCTTGAAGTGGTGGCCTAACTACGGCTACACTA
GAAGAACAGTATTTGGTATCTGCGCTCTGCTGAAGCCAGTTACCTTCGGAA
AAAGAGTTGGTAGCTCTTGATCCGGCAAACAAACCACCGCTGGTAGCGGT
GGTTTTTTTGTTTGCAAGCAGCAGATTACGCGCAGAAAAAAAGGATCTCA
AGAAGATCCTTTGATCTTTTCTACGGGGTCTGACGCTCAGTGGAACGAAAA
CTCACGTTAAGGGATTTTGGTCATGAAGCGCTTTTGAAGCTCGGATCCGAA
CAAACGACCCAACACCCGTGCGTTTTATTCTGTCTTTTTATTGCCGATCCC
CTCAGAAGAACTCGTCAAGAAGGCGATAGAAGGCGATGCGCTGCGAATCG
GGAGCGGCGATACCGTAAAGCACGAGGAAGCGGTCAGCCCATTCGCCGCC
AAGCTCTTCAGCAATATCACGGGTAGCCAACGCTATGTCCTGATAGCGGTC
CGCCACACCCAGCCGGCCACAGTCGATGAATCCAGAAAAGCGGCCATTTT
CCACCATGATATTCGGCAAGCAGGCATCGCCATGGGTCACGACGAGATCC
TCGCCGTCGGGCATGCTCGCCTTGAGCCTGGCGAACAGTTCGGCTGGCGC
GAGCCCCTGATGCTCTTCGTCCAGATCATCCTGATCGACAAGACCGGCTTC
CATCCGAGTACGTGCTCGCTCGATGCGATGTTTCGCTTGGTGGTCGAATGG
GCAGGTAGCCGGATCAAGCGTATGCAGCCGCCGCATTGCATCAGCCATGA
TGGATACTTTCTCGGCAGGAGCAAGGTGAGATGACAGGAGATCCTGCCCC
GGCACTTCGCCCAATAGCAGCCAGTCCCTTCCCGCTTCAGTGACAACGTCG
AGCACAGCTGCGCAAGGAACGCCCGTCGTGGCCAGCCACGATAGCCGCGC
TGCCTCGTCTTGCAGTTCATTCAGGGCACCGGACAGGTCGGTCTTGACAAA
AAGAACCGGGCGCCCCTGCGCTGACAGCCGGAACACGGCGGCATCAGAGC
AGCCGATTGTCTGTTGTGCCCAGTCATAGCCGAATAGCCTCTCCACCCAAG
CGGCCGGAGAACCTGCGTGCAATCCATCTTGTTCAATCATGCGAAACGAT
CCTCATCCTGTCTCTTGATCAGAGCTTGATCCCCTGCGCCATCAGATCCTT
GGCGGCAAGAAAGCCATCCAGTTTACTTTGCAGGGCTTCCCAACCTTACCA
GAGGCCTGCGCCGCGGCCAGCTGGCTAGCAATTCCCGGGTTAACTCTAGA
GACATTGATTATTGACTAGTTATTAATAGTAATCAATTACGGGGTCATTAG
TTCATAGCCCATATATGGAGTTCCGCGTTACATAACTTACGGTAAATGGCC
CGCCTGGCTGACCGCCCAACGACCCCCGCCCATTGACGTCAATAATGACG
TATGTTCCCATAGTAACGCCAATAGGGACTTTCCATTGACGTCAATGGGTG
GAGTATTTACGGTAAACTGCCCACTTGGCAGTACATCAAGTGTATCATATG
CCAAGTACGCCCCCTATTGACGTCAATGACGGTAAATGGCCCGCCTGGCA
TTATGCCCAGTACATGACCTTATGGGACTTTCCTACTTGGCAGTACATCTA
 
CGTATTAGTCATCGCTATTACCATGGTGATGCGGTTTTGGCAGTACATCAA
TGGGCGTGGATAGCGGTTTGACTCACGGGGATTTCCAAGTCTCCACCCCAT
TGACGTCAATGGGAGTTTGTTTTGGCACCAAAATCAACGGGACTTTCCAAA
ATGTCGTAACAACTCCGCCCCATTGACGCAAATGGGCGGTAGGCGTGTAC
GGTGGGAGGTCTATATAAGCAGAGCTCGTTTAGTGAACCGGGGTCTCTCT
GGTTAGACCAGATCTGAGCCTGGGAGCTCTCTGGCTAACTAGGGAACCCA
CTGCTTAAGCCTCAATAAAGCTTGCCTTGAGTGCTTCAAGTAGTGTGTGCC
CGTCTGTTGTGTGACTCTGGTAACTAGAGATCCCTCAGACCCTTTTAGTCA
GTGTGGAAAATCTCTAGCAGTGGCGCCCGAACAGGGACTTGAAAGCGAAA
GGGAAACCAGAGGAGCTCTCTCGACGCAGGACTCGGCTTGCTGAAGCGCG
CACGGCAAGAGGCGAGGGGCGGCGACTGGTGAGTACGCCAAAAATTTTGA
CTAGCGGAGGCTAGAAGGAGAGAGATGGGTGCGAGAGCGTCAGTATTAA
GCGGGGGAGAATTAGATCGCGATGGGAAAAAATTCGGTTAAGGCCAGGG
GGAAAGAAAAAATATAAATTAAAACATATAGTATGGGCAAGCAGGGAGC
TAGAACGATTCGCAGTTAATCCTGGCCTGTTAGAAACATCAGAAGGCTGT
AGACAAATACTGGGACAGCTACAACCATCCCTTCAGACAGGATCAGAAGA
ACTTAGATCATTATATAATACAGTAGCAACCCTCTATTGTGTGCATCAAAG
GATAGAGATAAAAGACACCAAGGAAGCTTTAGACAAGATAGAGGAAGAG
CAAAACAAAAGTAAGACCACCGCACAGCAAGCGGCCGCTGATCTTCAGAC
CTGGAGGAGGAGATATGAGGGACAATTGGAGAAGTGAATTATATAAATAT
AAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCC
TTGGGTTCTTGGGAGCAGCAGGAAGCACTATGGGCGCAGCGTCAATGACG
CTGACGGTACAGGCCAGACAATTATTGTCTGGTATAGTGCAGCAGCAGAA
CAATTTGCTGAGGGCTATTGAGGCGCAACAGCATCTGTTGCAACTCACAGT
CTGGGGCATCAAGCAGCTCCAGGCAAGAATCCTGGCTGTGGAAAGATACC
TAAAGGATCAACAGCTCCTGGGGATTTGGGGTTGCTCTGGAAAACTCATTT
GCACCACTGCTGTGCCTTGGAATGCTAGTTGGAGTAATAAATCTCTGGAAC
AGATTTGGAATCACACGACCTGGATGGAGTGGGACAGAGAAATTAACAAT
TACACAAGCTTAATACACTCCTTAATTGAAGAATCGCAAAACCAGCAAGA
AAAGAATGAACAAGAATTATTGGAATTAGATAAATGGGCAAGTTTGTGGA
ATTGGTTTAACATAACAAATTGGCTGTGGTATATAAAATTATTCATAATGA
TAGTAGGAGGCTTGGTAGGTTTAAGAATAGTTTTTGCTGTACTTTCTATAG
TGAATAGAGTTAGGCAGGGATATTCACCATTATCGTTTCAGACCCACCTCC
CAACCCCGAGGGGACCCGACAGGCCCGAAGGAATAGAAGAAGAAGGTGG
AGAGAGAGACAGAGACAGATCCATTCGATTAGTGAACGGATCTCGACGGT
ATCGGTTAACTTTTAAAAGAAAAGGGGGGATTGGGGGGTACAGTGCAGGG
GAAAGAATAGTAGACATAATAGCAACAGACATACAAACTAAAGAATTACA
AAAACAAATTACAAAAATTCAAAATTTTATCGATCACGAGACTAGCCTCG
AGGCATGCCTGCAGGAATTCCGTTACATAACTTACGGTAAATGGCCCGCCT
GGCTGACCGCCCAACGACCCCCGCCCATTGACGTCAATAATGACGTATGTT
CCCATAGTAACGCCAATAGGGACTTTCCATTGACGTCAATGGGTGGAGTA
TTTACGGTAAACTGCCCACTTGGCAGTACATCAAGTGTATCATATGCCAAG
TACGCCCCCTATTGACGTCAATGACGGTAAATGGCCCGCCTGGCATTATGC
CCAGTACATGACCTTATGGGACTTTCCTACTTGGCAGTACATCTACGTATT
AGTCATCGCTATTACCATGGTCGAGGTGAGCCCCACGTTCTGCTTCACTCT
 
CCCCATCTCCCCCCCCTCCCCACCCCCAATTTTGTATTTATTTATTTTTTAA
TTATTTTGTGCAGCGATGGGGGCGGGGGGGGGGGGGGGGCGCGCGCCAGG
CGGGGCGGGGCGGGGCGAGGGGCGGGGCGGGGCGAGGCGGAGAGGTGCG
GCGGCAGCCAATCAGAGCGGCGCGCTCCGAAAGTTTCCTTTTATGGCGAG
GCGGCGGCGGCGGCGGCCCTATAAAAAGCGAAGCGCGCGGCGGGCGGGA
GTCGCTGCGACGCTGCCTTCGCCCCGTGCCCCGCTCCGCCGCCGCCTCGCG
CCGCCCGCCCCGGCTCTGACTGACCGCGTTACTCCCACAGGTGAGCGGGC
GGGACGGCCCTTCTCCTCCGGGCTGTAATTAGCTGAGCAAGAGGTAAGGG
TTTAAGGGATGGTTGGTTGGTGGGGTATTAATGTTTAATTACCTGGAGCAC
CTGCCTGAAATCACTTTTTTTCAGGTACCGGTCGCCACCATGGCGAGACCC
CTGTGCACATTACTTCTGTTGATGGCTACCCTGGCAGGCGCCCTCGCCAGC
GAGCTGACACAGGACCCTGCCGTGTCCGTGGCCCTGAAGCAGACCGTGAC
AATCACCTGCAGAGGCGATTCCCTGAGATCCCACTACGCCTCCTGGTACCA
GAAGAAGCCTGGCCAGGCCCCCGTGCTGCTGTTTTACGGCAAGAATAACC
GCCCCAGCGGCATCCCCGATAGATTTTCCGGCAGCGCCTCCGGCAACAGA
GCCAGCCTGACAATCACCGGCGCCCAGGCCGAGGACGAGGCTGATTACTA
CTGCAGCTCCAGAGATAAGAGCGGCAGCAGACTGTCCGTGTTTGGCGGCG
GCACCAAGCTGACCGTGCTCGGAGGAGGAGGAAGCGGAGGAGGAGGCTC
AGGCGGCGGCGGCTCTGAGGTGAGGCTGAGAGAGTCCGGCGGCGGCCTGG
TGAAGCCCGGAGGATCTCTGAGGCTGTCCTGCTCCGCCTCCGGCTTCGATT
TTGACAATGCCTGGATGACCTGGGTGAGACAGCCCCCTGGCAAGGGCCTG
GAGTGGGTGGGAAGGATCACAGGCCCCGGCGAGGGCTGGTCCGTGGATTA
CGCCGAGTCCGTGAAGGGCAGGTTCACAATCTCCAGGGATAACACCAAGA
ACACCCTGTACCTGGAGATGAACAACGTGAGGACAGAGGATACCGGCTAC
TACTTTTGCGCCAGAACAGGCAAGTACTACGACTTTTGGTTCGGCTACCCC
CCTGGCGAGGAGTACTTCCAGGATTGGGGCCAGGGCACCCTGGTCATTGT
GTCCAGCGGCGGCGGCGGCAGTGGCGGCGGCGGAAGCGGCGGCGGCGGC
TCTGGCGGCGGCGGCAGCGGCGGCGGCGGCTCCGACATCGTGATGACCCA
GTCTCCTGATAGCCTGGCCGTGAGCCTGGGCGAGAGAGTGACAATGAACT
GTAAGTCTAGCCAGAGCCTGCTGTACTCCACCAACCAGAAGAATTACCTG
GCCTGGTATCAGCAGAAGCCTGGCCAGTCCCCAAAGCTGCTGATCTATTG
GGCATCTACAAGGGAGAGCGGAGTGCCAGACAGATTCAGCGGATCCGGAT
CTGGAACCGACTTCACCCTGACAATCTCCTCTGTGCAGGCCGAGGACGTG
GCCGTGTACTATTGCCAGCAGTACTATAGCTACAGGACATTCGGCGGCGG
CACCAAGCTGGAGATCAAGCGCACCGTGGCCGGAGGAGGAGGATCTGGC
GGAGGAGGGTCCGGCGGCGGCGGCTCCCAGGTGCAGCTGCAGCAGAGCG
GACCAGAGGTGGTGAAGCCTGGAGCCTCCGTGAAGATGTCTTGTAAGGCC
AGCGGCTACACCTTCACATCCTATGTGATCCACTGGGTGAGGCAGAAGCC
AGGACAGGGACTGGACTGGATCGGCTACATCAACCCTTATAATGATGGCA
CCGACTACGATGAGAAGTTTAAGGGCAAGGCCACCCTGACATCCGATACC
AGCACATCCACCGCCTATATGGAGCTGAGCTCCCTGCGGTCTGAGGACAC
AGCCGTGTACTATTGCGCCAGAGAGAAGGATAACTACGCAACCGGAGCAT
GGTTCGCATATTGGGGACAGGGTACCCTGGTCACCGTGTCTAGCGGCGGA
GGAGGAAGCGGAGGAGGCGGCTCTGGCGGAGGAGGTTCCGGAGGAGGCG
GAAGCGGCGGAGGAGGCTCTTGGGAGCAGAAGATCGAGGAGCTGCTGAA
GAAGGCCGAGGAGCAGCAGAAGAAGAATGAGGAGGAGCTGAAGAAGCTG
 
GAGAAGTAGGATATCCAGCACAGTCCCGGGCCGAGTCTAGACGTTTAAAC
CCGCTGATCAGGTCGACAATCAACCTCTGGATTACAAAATTTGTGAAAGA
TTGACTGGTATTCTTAACTATGTTGCTCCTTTTACGCTATGTGGATACGCTG
CTTTAATGCCTTTGTATCATGCTATTGCTTCCCGTATGGCTTTCATTTTCTC
CTCCTTGTATAAATCCTGGTTGCTGTCTCTTTATGAGGAGTTGTGGCCCGTT
GTCAGGCAACGTGGCGTGGTGTGCACTGTGTTTGCTGACGCAACCCCCACT
GGTTGGGGCATTGCCACCACCTGTCAGCTCCTTTCCGGGACTTTCGCTTTC
CCCCTCCCTATTGCCACGGCGGAACTCATCGCCGCCTGCCTTGCCCGCTGC
TGGACAGGGGCTCGGCTGTTGGGCACTGACAATTCCGTGGTGTTGTCGGG
GAAGCTGACGTCCTTTCCATGGCTGCTCGCCTGTGTTGCCACCTGGATTCT
GCGCGGGACGTCCTTCTGCTACGTCCCTTCGGCCCTCAATCCAGCGGACCT
TCCTTCCCGCGGCCTGCTGCCGGCTCTGCGGCCTCTTCCGCGTCTTCGCCTT
CGCCCTCAGACGAGTCGGATCTCCCTTTGGGCCGCCTCCCCGCGGTACCTT
TAAGACCAATGACTTACAAGGCAGCTGTAGATCTTAGCCACTTTTTAAAAG
AAAAGGGGGGACTGGAAGGGCTAATTCACTCCCAACGAAGACAAGATCTG
CTTTTTGCTTGTACTGGGTCTCTCTGGTTAGACCAGATCTGAGCCTGGGAG
CTCTCTGGCTAACTAGGGAACCCACTGCTTAAGCCTCAATAAAGCTTGCCT
TGAGTGCTTCAAGTAGTGTGTGCCCGTCTGTTGTGTGACTCTGGTAACTAG
AGATCCCTCAGACCCTTTTAGTCAGTGTGGAAAATCTCTAGCAGTAGTAGT
TCATGTCATCTTATTATTCAGTATTTATAACTTGCAAAGAAATGAATATCA
GAGAGTGAGAGGAACTTGTTTATTGCAGCTTATAATGGTTACAAATAAAG
CAATAGCATCACAAATTTCACAAATAAAGCATTTTTTTCACTGCATTCTAG
TTGTGGTTTGTCCAAACTCATCAATGTATCTTATCATGTCTGGCTCTAGCTA
TCCCGCCCCTAACTCCGCCCATCCCGCCCCTAACTCCGCCCAGTTCCGCCC
ATTCTCCGCCCCATGGCTGACTAATTTTTTTTATTTATGCAGAGGCCGAGG
CCGCCTCGGCCTCTGAGCTATTCCAGAAGTAGTGAGGAGGCTTTTTTGGAG
GCCGCTAGCGTCGACCATTACTTATTGTTTTAGCTGTCCTCATGAATGTCTT
TTCACTACCCATTTGCTTATCCTGCATCTCTCAGCCTTGACTCCACTCAGTT
CTCTTGCTTAGAGATACCACCTTTCCCCTGAAGTGTTCCTTCCATGTTTTAC
GGCGAGATGGTTTCTCCTCGCCTGGCCACTCAGCCTTAGTTGTCTCTGTTG
TCTTATAGAGGTCTACTTGAAGAAGGAAAAACAGGGGGCATGGTTTGACT
GTCCTGTGAGCCCTTCTTCCCTGCCTCCCCCACTCACAGTGACCCGGAATC
CCTCGACATGGCAGTCTAGCACTAGTGCGGCCGCAGATCTGCTTCCTCGCT
CACTGACTCGCTGCGCTCGGTCGTTCGGCTGCGGCGAGCGGTATCAGCTCA
CTCAAAGGCGGTAATACGGTTATCCACAGAATCAGGGGATAACGCAGGAA
AGAACATGTGAGCAAAAGGCCAGCAAAAGGCCAGGAACCGTAAAAA
SEQ ID NO: 14 
ACATGTGAGCAAAAGGCCAGCAAAAGGCCAGGAACCGTAAAAAGGCCGC
GTTGCTGGCGTTTTTCCATAGGCTCCGCCCCCCTGACGAGCATCACAAAAA
TCGACGCTCAAGTCAGAGGTGGCGAAACCCGACAGGACTATAAAGATACC
AGGCGTTTCCCCCTGGAAGCTCCCTCGTGCGCTCTCCTGTTCCGACCCTGC
CGCTTACCGGATACCTGTCCGCCTTTCTCCCTTCGGGAAGCGTGGCGCTTT
CTCATAGCTCACGCTGTAGGTATCTCAGTTCGGTGTAGGTCGTTCGCTCCA
AGCTGGGCTGTGTGCACGAACCCCCCGTTCAGCCCGACCGCTGCGCCTTAT
CCGGTAACTATCGTCTTGAGTCCAACCCGGTAAGACACGACTTATCGCCAC
TGGCAGCAGCCACTGGTAACAGGATTAGCAGAGCGAGGTATGTAGGCGGT
 
GCTACAGAGTTCTTGAAGTGGTGGCCTAACTACGGCTACACTAGAAGGAC
AGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTT
TGTTTGCAAGCAGCAGATTACGCGCAGAAAAAAAGGATCTCAAGAAGATC
CTTTGATCTTTTCTACGGGGTCTGACGCTCAGTGGAACGAAAACTCACGTT
AAGGGATTTTGGTCATGAGATTATCAAAAAGGATCTTCACCTAGATCCTTT
TAAATTAAAAATGAAGTTTTAAATCAATCTAAAGTATATATGAGTAAACTT
GGTCTGACAGTTACCAATGCTTAATCAGTGAGGCACCTATCTCAGCGATCT
GTCTATTTCGTTCATCCATAGTTGCCTGACTCCCCGTCGTGTAGATAACTA
CGATACGGGAGGGCTTACCATCTGGCCCCAGTGCTGCAATGATACCGCGA
GACCCACGCTCACCGGCTCCAGATTTATCAGCAATAAACCAGCCAGCCGG
AAGGGCCGAGCGCAGAAGTGGTCCTGCAACTTTATCCGCCTCCATCCAGT
CTATTAATTGTTGCCGGGAAGCTAGAGTAAGTAGTTCGCCAGTTAATAGTT
TGCGCAACGTTGTTGCCATTGCTACAGGCATCGTGGTGTCACGCTCGTCGT
TTGGTATGGCTTCATTCAGCTCCGGTTCCCAACGATCAAGGCGAGTTACAT
GATCCCCCATGTTGTGCAAAAAAGCGGTTAGCTCCTTCGGTCCTCCGATCG
TTGTCAGAAGTAAGTTGGCCGCAGTGTTATCACTCATGGTTATGGCAGCAC
TGCATAATTCTCTTACTGTCATGCCATCCGTAAGATGCTTTTCTGTGACTGG
TGAGTACTCAACCAAGTCATTCTGAGAATAGTGTATGCGGCGACCGAGTT
GCTCTTGCCCGGCGTCAATACGGGATAATACCGCGCCACATAGCAGAACT
TTAAAAGTGCTCATCATTGGAAAACGTTCTTCGGGGCGAAAACTCTCAAG
GATCTTACCGCTGTTGAGATCCAGTTCGATGTAACCCACTCGTGCACCCAA
CTGATCTTCAGCATCTTTTACTTTCACCAGCGTTTCTGGGTGAGCAAAAAC
AGGAAGGCAAAATGCCGCAAAAAAGGGAATAAGGGCGACACGGAAATGT
TGAATACTCATACTCTTCCTTTTTCAATATTATTGAAGCATTTATCAGGGTT
ATTGTCTCATGAGCGGATACATATTTGAATGTATTTAGAAAAATAAACAA
ATAGGGGTTCCGCGCACATTTCCCCGAAAAGTGCCACCTGACGTCTAAGA
AACCATTATTATCATGACATTAACCTATAAAAATAGGCGTATCACGAGGC
CCTTTCGTCTCGCGCGTTTCGGTGATGACGGTGAAAACCTCTGACACATGC
AGCTCCCGGAGACGGTCACAGCTTGTCTGTAAGCGGATGCCGGGAGCAGA
CAAGCCCGTCAGGGCGCGTCAGCGGGTGTTGGCGGGTGTCGGGGCTGGCT
TAACTATGCGGCATCAGAGCAGATTGTACTGAGAGTGCACCATAAAATTG
TAAACGTTAATATTTTGTTAAAATTCGCGTTAAATTTTTGTTAAATCAGCTC
ATTTTTTAACCAATAGGCCGAAATCGGCAAAATCCCTTATAAATCAAAAG
AATAGCCCGAGATAGGGTTGAGTGTTGTTCCAGTTTGGAACAAGAGTCCA
CTATTAAAGAACGTGGACTCCAACGTCAAAGGGCGAAAAACCGTCTATCA
GGGCGATGGCCCACTACGTGAACCATCACCCAAATCAAGTTTTTTGGGGTC
GAGGTGCCGTAAAGCACTAAATCGGAACCCTAAAGGGAGCCCCCGATTTA
GAGCTTGACGGGGAAAGCCGGCGAACGTGGCGAGAAAGGAAGGGAAGAA
AGCGAAAGGAGCGGGCGCTAGGGCGCTGGCAAGTGTAGCGGTCACGCTGC
GCGTAACCACCACACCCGCCGCGCTTAATGCGCCGCTACAGGGCGCGTAC
TATGGTTGCTTTGACGTATGCGGTGTGAAATACCGCACAGATGCGTAAGG
AGAAAATACCGCATCAGGCGCCCCTGCAGGCAGCTGCGCGCTCGCTCGCT
CACTGAGGCCGCCCGGGCAAAGCCCGGGCGTCGGGCGACCTTTGGTCGCC
CGGCCTCAGTGAGCGAGCGAGCGCGCAGAGAGGGAGTGGCCAACTCCATC
ACTAGGGGTTCCTACGCGTCGTTACATAACTTACGGTAAATGGCCCGCCTG
 
GCTGACCGCCCAACGACCCCCGCCCATTGACGTCAATAATGACGTATGTTC
CCATAGTAACGCCAATAGGGACTTTCCATTGACGTCAATGGGTGGAGTATT
TACGGTAAACTGCCCACTTGGCAGTACATCAAGTGTATCATATGCCAAGTA
CGCCCCCTATTGACGTCAATGACGGTAAATGGCCCGCCTGGCATTATGCCC
AGTACATGACCTTATGGGACTTTCCTACTTGGCAGTACATCTACGTATTAG
TCATCGCTATTACCATGGTCGAGGTGAGCCCCACGTTCTGCTTCACTCTCC
CCATCTCCCCCCCCTCCCCACCCCCAATTTTGTATTTATTTATTTTTTAATT
ATTTTGTGCAGCGATGGGGGCGGGGGGGGGGGGGGGGCGCGCGCCAGGC
GGGGCGGGGCGGGGCGAGGGGCGGGGCGGGGCGAGGCGGAGAGGTGCGG
CGGCAGCCAATCAGAGCGGCGCGCTCCGAAAGTTTCCTTTTATGGCGAGG
CGGCGGCGGCGGCGGCCCTATAAAAAGCGAAGCGCGCGGCGGGCGGGAG
TCGCTGCGACGCTGCCTTCGCCCCGTGCCCCGCTCCGCCGCCGCCTCGCGC
CGCCCGCCCCGGCTCTGACTGACCGCGTTACTCCCACAGGTGAGCGGGCG
GGACGGCCCTTCTCCTCCGGGCTGTAATTAGCTGAGCAAGAGGTAAGGGT
TTAAGGGATGGTTGGTTGGTGGGGTATTAATGTTTAATTACCTGGAGCACC
TGCCTGAAATCACTTTTTTTCAGGTACCGGTCGTCGACAATCAACCTCTGG
ATTACAAAATTTGTGAAAGATTGACTGGTATTCTTAACTATGTTGCTCCTT
TTACGCTATGTGGATACGCTGCTTTAATGCCTTTGTATCATGCTATTGCTTC
CCGTATGGCTTTCATTTTCTCCTCCTTGTATAAATCCTGGTTGCTGTCTCTT
TATGAGGAGTTGTGGCCCGTTGTCAGGCAACGTGGCGTGGTGTGCACTGT
GTTTGCTGACGCAACCCCCACTGGTTGGGGCATTGCCACCACCTGTCAGCT
CCTTTCCGGGACTTTCGCTTTCCCCCTCCCTATTGCCACGGCGGAACTCAT
CGCCGCCTGCCTTGCCCGCTGCTGGACAGGGGCTCGGCTGTTGGGCACTGA
CAATTCCGTGGTGTTGTCGGGGAAGCTGACGTCCTTTCCATGGCTGCTCGC
CTGTGTTGCCACCTGGATTCTGCGCGGGACGTCCTTCTGCTACGTCCCTTC
GGCCCTCAATCCAGCGGACCTTCCTTCCCGCGGCCTGCTGCCGGCTCTGCG
GCCTCTTCCGCGTCTTCGCCTTCGCCCTCAGACGAGTCGGATCTCCCTTTG
GGCCGCCTCCCCGCGAATTCGTTTATTTGTGAAATTTGTGATGCTATTGCTT
TATTTGTAACCATCTAGCTTTATTTGTGAAATTTGTGATGCTATTGCTTTAT
TTGTAACCATTATAAGCTGCAATAAACAAGTTAACAACAACAATTGCATTC
ATTTTATGTTTCAGGTTCAGGGGGAGATGTGGGAGGTTTTTTAAAGTTTAA
ACAGGAACCCCTAGTGATGGAGTTGGCCACTCCCTCTCTGCGCGCTCGCTC
GCTCACTGAGGCCGGGCGACCAAAGGTCGCCCGACGCCCGGGCGGCCTCA
GTGAGCGAGCGAGCGCGCAGCTGCCTGCAGG
SEQ ID NO: 15 
ACATGTGAGCAAAAGGCCAGCAAAAGGCCAGGAACCGTAAAAAGGCCGC
GTTGCTGGCGTTTTTCCATAGGCTCCGCCCCCCTGACGAGCATCACAAAAA
TCGACGCTCAAGTCAGAGGTGGCGAAACCCGACAGGACTATAAAGATACC
AGGCGTTTCCCCCTGGAAGCTCCCTCGTGCGCTCTCCTGTTCCGACCCTGC
CGCTTACCGGATACCTGTCCGCCTTTCTCCCTTCGGGAAGCGTGGCGCTTT
CTCATAGCTCACGCTGTAGGTATCTCAGTTCGGTGTAGGTCGTTCGCTCCA
AGCTGGGCTGTGTGCACGAACCCCCCGTTCAGCCCGACCGCTGCGCCTTAT
CCGGTAACTATCGTCTTGAGTCCAACCCGGTAAGACACGACTTATCGCCAC
TGGCAGCAGCCACTGGTAACAGGATTAGCAGAGCGAGGTATGTAGGCGGT
GCTACAGAGTTCTTGAAGTGGTGGCCTAACTACGGCTACACTAGAAGGAC
AGTATTTGGTATCTGCGCTCTGCTGAAGCCAGTTACCTTCGGAAAAAGAGT
 
TGGTAGCTCTTGATCCGGCAAACAAACCACCGCTGGTAGCGGTGGTTTTTT
TGTTTGCAAGCAGCAGATTACGCGCAGAAAAAAAGGATCTCAAGAAGATC
CTTTGATCTTTTCTACGGGGTCTGACGCTCAGTGGAACGAAAACTCACGTT
AAGGGATTTTGGTCATGAGATTATCAAAAAGGATCTTCACCTAGATCCTTT
TAAATTAAAAATGAAGTTTTAAATCAATCTAAAGTATATATGAGTAAACTT
GGTCTGACAGTTACCAATGCTTAATCAGTGAGGCACCTATCTCAGCGATCT
GTCTATTTCGTTCATCCATAGTTGCCTGACTCCCCGTCGTGTAGATAACTA
CGATACGGGAGGGCTTACCATCTGGCCCCAGTGCTGCAATGATACCGCGA
GACCCACGCTCACCGGCTCCAGATTTATCAGCAATAAACCAGCCAGCCGG
AAGGGCCGAGCGCAGAAGTGGTCCTGCAACTTTATCCGCCTCCATCCAGT
CTATTAATTGTTGCCGGGAAGCTAGAGTAAGTAGTTCGCCAGTTAATAGTT
TGCGCAACGTTGTTGCCATTGCTACAGGCATCGTGGTGTCACGCTCGTCGT
TTGGTATGGCTTCATTCAGCTCCGGTTCCCAACGATCAAGGCGAGTTACAT
GATCCCCCATGTTGTGCAAAAAAGCGGTTAGCTCCTTCGGTCCTCCGATCG
TTGTCAGAAGTAAGTTGGCCGCAGTGTTATCACTCATGGTTATGGCAGCAC
TGCATAATTCTCTTACTGTCATGCCATCCGTAAGATGCTTTTCTGTGACTGG
TGAGTACTCAACCAAGTCATTCTGAGAATAGTGTATGCGGCGACCGAGTT
GCTCTTGCCCGGCGTCAATACGGGATAATACCGCGCCACATAGCAGAACT
TTAAAAGTGCTCATCATTGGAAAACGTTCTTCGGGGCGAAAACTCTCAAG
GATCTTACCGCTGTTGAGATCCAGTTCGATGTAACCCACTCGTGCACCCAA
CTGATCTTCAGCATCTTTTACTTTCACCAGCGTTTCTGGGTGAGCAAAAAC
AGGAAGGCAAAATGCCGCAAAAAAGGGAATAAGGGCGACACGGAAATGT
TGAATACTCATACTCTTCCTTTTTCAATATTATTGAAGCATTTATCAGGGTT
ATTGTCTCATGAGCGGATACATATTTGAATGTATTTAGAAAAATAAACAA
ATAGGGGTTCCGCGCACATTTCCCCGAAAAGTGCCACCTGACGTCTAAGA
AACCATTATTATCATGACATTAACCTATAAAAATAGGCGTATCACGAGGC
CCTTTCGTCTCGCGCGTTTCGGTGATGACGGTGAAAACCTCTGACACATGC
AGCTCCCGGAGACGGTCACAGCTTGTCTGTAAGCGGATGCCGGGAGCAGA
CAAGCCCGTCAGGGCGCGTCAGCGGGTGTTGGCGGGTGTCGGGGCTGGCT
TAACTATGCGGCATCAGAGCAGATTGTACTGAGAGTGCACCATAAAATTG
TAAACGTTAATATTTTGTTAAAATTCGCGTTAAATTTTTGTTAAATCAGCTC
ATTTTTTAACCAATAGGCCGAAATCGGCAAAATCCCTTATAAATCAAAAG
AATAGCCCGAGATAGGGTTGAGTGTTGTTCCAGTTTGGAACAAGAGTCCA
CTATTAAAGAACGTGGACTCCAACGTCAAAGGGCGAAAAACCGTCTATCA
GGGCGATGGCCCACTACGTGAACCATCACCCAAATCAAGTTTTTTGGGGTC
GAGGTGCCGTAAAGCACTAAATCGGAACCCTAAAGGGAGCCCCCGATTTA
GAGCTTGACGGGGAAAGCCGGCGAACGTGGCGAGAAAGGAAGGGAAGAA
AGCGAAAGGAGCGGGCGCTAGGGCGCTGGCAAGTGTAGCGGTCACGCTGC
GCGTAACCACCACACCCGCCGCGCTTAATGCGCCGCTACAGGGCGCGTAC
TATGGTTGCTTTGACGTATGCGGTGTGAAATACCGCACAGATGCGTAAGG
AGAAAATACCGCATCAGGCGCCCCTGCAGGCAGCTGCGCGCTCGCTCGCT
CACTGAGGCCGCCCGGGCAAAGCCCGGGCGTCGGGCGACCTTTGGTCGCC
CGGCCTCAGTGAGCGAGCGAGCGCGCAGAGAGGGAGTGGCCAACTCCATC
ACTAGGGGTTCCTACGCGTCGTTACATAACTTACGGTAAATGGCCCGCCTG
GCTGACCGCCCAACGACCCCCGCCCATTGACGTCAATAATGACGTATGTTC
CCATAGTAACGCCAATAGGGACTTTCCATTGACGTCAATGGGTGGAGTATT
 
TACGGTAAACTGCCCACTTGGCAGTACATCAAGTGTATCATATGCCAAGTA
CGCCCCCTATTGACGTCAATGACGGTAAATGGCCCGCCTGGCATTATGCCC
AGTACATGACCTTATGGGACTTTCCTACTTGGCAGTACATCTACGTATTAG
TCATCGCTATTACCATGGTCGAGGTGAGCCCCACGTTCTGCTTCACTCTCC
CCATCTCCCCCCCCTCCCCACCCCCAATTTTGTATTTATTTATTTTTTAATT
ATTTTGTGCAGCGATGGGGGCGGGGGGGGGGGGGGGGCGCGCGCCAGGC
GGGGCGGGGCGGGGCGAGGGGCGGGGCGGGGCGAGGCGGAGAGGTGCGG
CGGCAGCCAATCAGAGCGGCGCGCTCCGAAAGTTTCCTTTTATGGCGAGG
CGGCGGCGGCGGCGGCCCTATAAAAAGCGAAGCGCGCGGCGGGCGGGAG
TCGCTGCGACGCTGCCTTCGCCCCGTGCCCCGCTCCGCCGCCGCCTCGCGC
CGCCCGCCCCGGCTCTGACTGACCGCGTTACTCCCACAGGTGAGCGGGCG
GGACGGCCCTTCTCCTCCGGGCTGTAATTAGCTGAGCAAGAGGTAAGGGT
TTAAGGGATGGTTGGTTGGTGGGGTATTAATGTTTAATTACCTGGAGCACC
TGCCTGAAATCACTTTTTTTCAGGTACCGGTCGCCACCATGGCGAGACCCC
TGTGCACATTACTTCTGTTGATGGCTACCCTGGCAGGCGCCCTCGCCAGCG
AGCTGACACAGGACCCTGCCGTGTCCGTGGCCCTGAAGCAGACCGTGACA
ATCACCTGCAGAGGCGATTCCCTGAGATCCCACTACGCCTCCTGGTACCAG
AAGAAGCCTGGCCAGGCCCCCGTGCTGCTGTTTTACGGCAAGAATAACCG
CCCCAGCGGCATCCCCGATAGATTTTCCGGCAGCGCCTCCGGCAACAGAG
CCAGCCTGACAATCACCGGCGCCCAGGCCGAGGACGAGGCTGATTACTAC
TGCAGCTCCAGAGATAAGAGCGGCAGCAGACTGTCCGTGTTTGGCGGCGG
CACCAAGCTGACCGTGCTCGGAGGAGGAGGAAGCGGAGGAGGAGGCTCA
GGCGGCGGCGGCTCTGAGGTGAGGCTGAGAGAGTCCGGCGGCGGCCTGGT
GAAGCCCGGAGGATCTCTGAGGCTGTCCTGCTCCGCCTCCGGCTTCGATTT
TGACAATGCCTGGATGACCTGGGTGAGACAGCCCCCTGGCAAGGGCCTGG
AGTGGGTGGGAAGGATCACAGGCCCCGGCGAGGGCTGGTCCGTGGATTAC
GCCGAGTCCGTGAAGGGCAGGTTCACAATCTCCAGGGATAACACCAAGAA
CACCCTGTACCTGGAGATGAACAACGTGAGGACAGAGGATACCGGCTACT
ACTTTTGCGCCAGAACAGGCAAGTACTACGACTTTTGGTTCGGCTACCCCC
CTGGCGAGGAGTACTTCCAGGATTGGGGCCAGGGCACCCTGGTCATTGTG
TCCAGCGGCGGCGGCGGCAGTGGCGGCGGCGGAAGCGGCGGCGGCGGCT
CTGGCGGCGGCGGCAGCGGCGGCGGCGGCTCCGACATCGTGATGACCCAG
TCTCCTGATAGCCTGGCCGTGAGCCTGGGCGAGAGAGTGACAATGAACTG
TAAGTCTAGCCAGAGCCTGCTGTACTCCACCAACCAGAAGAATTACCTGG
CCTGGTATCAGCAGAAGCCTGGCCAGTCCCCAAAGCTGCTGATCTATTGG
GCATCTACAAGGGAGAGCGGAGTGCCAGACAGATTCAGCGGATCCGGATC
TGGAACCGACTTCACCCTGACAATCTCCTCTGTGCAGGCCGAGGACGTGG
CCGTGTACTATTGCCAGCAGTACTATAGCTACAGGACATTCGGCGGCGGC
ACCAAGCTGGAGATCAAGCGCACCGTGGCCGGAGGAGGAGGATCTGGCG
GAGGAGGGTCCGGCGGCGGCGGCTCCCAGGTGCAGCTGCAGCAGAGCGG
ACCAGAGGTGGTGAAGCCTGGAGCCTCCGTGAAGATGTCTTGTAAGGCCA
GCGGCTACACCTTCACATCCTATGTGATCCACTGGGTGAGGCAGAAGCCA
GGACAGGGACTGGACTGGATCGGCTACATCAACCCTTATAATGATGGCAC
CGACTACGATGAGAAGTTTAAGGGCAAGGCCACCCTGACATCCGATACCA
GCACATCCACCGCCTATATGGAGCTGAGCTCCCTGCGGTCTGAGGACACA
GCCGTGTACTATTGCGCCAGAGAGAAGGATAACTACGCAACCGGAGCATG
 
GTTCGCATATTGGGGACAGGGTACCCTGGTCACCGTGTCTAGCTAGGTCGA
CAATCAACCTCTGGATTACAAAATTTGTGAAAGATTGACTGGTATTCTTAA
CTATGTTGCTCCTTTTACGCTATGTGGATACGCTGCTTTAATGCCTTTGTAT
CATGCTATTGCTTCCCGTATGGCTTTCATTTTCTCCTCCTTGTATAAATCCT
GGTTGCTGTCTCTTTATGAGGAGTTGTGGCCCGTTGTCAGGCAACGTGGCG
TGGTGTGCACTGTGTTTGCTGACGCAACCCCCACTGGTTGGGGCATTGCCA
CCACCTGTCAGCTCCTTTCCGGGACTTTCGCTTTCCCCCTCCCTATTGCCAC
GGCGGAACTCATCGCCGCCTGCCTTGCCCGCTGCTGGACAGGGGCTCGGC
TGTTGGGCACTGACAATTCCGTGGTGTTGTCGGGGAAGCTGACGTCCTTTC
CATGGCTGCTCGCCTGTGTTGCCACCTGGATTCTGCGCGGGACGTCCTTCT
GCTACGTCCCTTCGGCCCTCAATCCAGCGGACCTTCCTTCCCGCGGCCTGC
TGCCGGCTCTGCGGCCTCTTCCGCGTCTTCGCCTTCGCCCTCAGACGAGTC
GGATCTCCCTTTGGGCCGCCTCCCCGCGAATTCGTTTATTTGTGAAATTTGT
GATGCTATTGCTTTATTTGTAACCATCTAGCTTTATTTGTGAAATTTGTGAT
GCTATTGCTTTATTTGTAACCATTATAAGCTGCAATAAACAAGTTAACAAC
AACAATTGCATTCATTTTATGTTTCAGGTTCAGGGGGAGATGTGGGAGGTT
TTTTAAAGTTTAAACAGGAACCCCTAGTGATGGAGTTGGCCACTCCCTCTC
TGCGCGCTCGCTCGCTCACTGAGGCCGGGCGACCAAAGGTCGCCCGACGC
CCGGGCGGCCTCAGTGAGCGAGCGAGCGCGCAGCTGCCTGCAGG
SEQ ID NO: 16 
ACATGTGAGCAAAAGGCCAGCAAAAGGCCAGGAACCGTAAAAAGGCCGCGTTGCT
GGCGTTTTTCCATAGGCTCCGCCCCCCTGACGAGCATCACAAAAATCGACGCTCAAG
TCAGAGGTGGCGAAACCCGACAGGACTATAAAGATACCAGGCGTTTCCCCCTGGAA
GCTCCCTCGTGCGCTCTCCTGTTCCGACCCTGCCGCTTACCGGATACCTGTCCGCCTT
TCTCCCTTCGGGAAGCGTGGCGCTTTCTCATAGCTCACGCTGTAGGTATCTCAGTTCG
GTGTAGGTCGTTCGCTCCAAGCTGGGCTGTGTGCACGAACCCCCCGTTCAGCCCGAC
CGCTGCGCCTTATCCGGTAACTATCGTCTTGAGTCCAACCCGGTAAGACACGACTTA
TCGCCACTGGCAGCAGCCACTGGTAACAGGATTAGCAGAGCGAGGTATGTAGGCGG
TGCTACAGAGTTCTTGAAGTGGTGGCCTAACTACGGCTACACTAGAAGGACAGTATT
TGGTATCTGCGCTCTGCTGAAGCCAGTTACCTTCGGAAAAAGAGTTGGTAGCTCTTG
ATCCGGCAAACAAACCACCGCTGGTAGCGGTGGTTTTTTTGTTTGCAAGCAGCAGAT
TACGCGCAGAAAAAAAGGATCTCAAGAAGATCCTTTGATCTTTTCTACGGGGTCTGA
CGCTCAGTGGAACGAAAACTCACGTTAAGGGATTTTGGTCATGAGATTATCAAAAA
GGATCTTCACCTAGATCCTTTTAAATTAAAAATGAAGTTTTAAATCAATCTAAAGTA
TATATGAGTAAACTTGGTCTGACAGTTACCAATGCTTAATCAGTGAGGCACCTATCT
CAGCGATCTGTCTATTTCGTTCATCCATAGTTGCCTGACTCCCCGTCGTGTAGATAAC
TACGATACGGGAGGGCTTACCATCTGGCCCCAGTGCTGCAATGATACCGCGAGACC
CACGCTCACCGGCTCCAGATTTATCAGCAATAAACCAGCCAGCCGGAAGGGCCGAG
CGCAGAAGTGGTCCTGCAACTTTATCCGCCTCCATCCAGTCTATTAATTGTTGCCGG
GAAGCTAGAGTAAGTAGTTCGCCAGTTAATAGTTTGCGCAACGTTGTTGCCATTGCT
ACAGGCATCGTGGTGTCACGCTCGTCGTTTGGTATGGCTTCATTCAGCTCCGGTTCCC
AACGATCAAGGCGAGTTACATGATCCCCCATGTTGTGCAAAAAAGCGGTTAGCTCCT
TCGGTCCTCCGATCGTTGTCAGAAGTAAGTTGGCCGCAGTGTTATCACTCATGGTTA
TGGCAGCACTGCATAATTCTCTTACTGTCATGCCATCCGTAAGATGCTTTTCTGTGAC
TGGTGAGTACTCAACCAAGTCATTCTGAGAATAGTGTATGCGGCGACCGAGTTGCTC
TTGCCCGGCGTCAATACGGGATAATACCGCGCCACATAGCAGAACTTTAAAAGTGCT
 
CATCATTGGAAAACGTTCTTCGGGGCGAAAACTCTCAAGGATCTTACCGCTGTTGAG
ATCCAGTTCGATGTAACCCACTCGTGCACCCAACTGATCTTCAGCATCTTTTACTTTC
ACCAGCGTTTCTGGGTGAGCAAAAACAGGAAGGCAAAATGCCGCAAAAAAGGGAA
TAAGGGCGACACGGAAATGTTGAATACTCATACTCTTCCTTTTTCAATATTATTGAA
GCATTTATCAGGGTTATTGTCTCATGAGCGGATACATATTTGAATGTATTTAGAAAA
ATAAACAAATAGGGGTTCCGCGCACATTTCCCCGAAAAGTGCCACCTGACGTCTAA
GAAACCATTATTATCATGACATTAACCTATAAAAATAGGCGTATCACGAGGCCCTTT
CGTCTCGCGCGTTTCGGTGATGACGGTGAAAACCTCTGACACATGCAGCTCCCGGAG
ACGGTCACAGCTTGTCTGTAAGCGGATGCCGGGAGCAGACAAGCCCGTCAGGGCGC
GTCAGCGGGTGTTGGCGGGTGTCGGGGCTGGCTTAACTATGCGGCATCAGAGCAGA
TTGTACTGAGAGTGCACCATAAAATTGTAAACGTTAATATTTTGTTAAAATTCGCGT
TAAATTTTTGTTAAATCAGCTCATTTTTTAACCAATAGGCCGAAATCGGCAAAATCC
CTTATAAATCAAAAGAATAGCCCGAGATAGGGTTGAGTGTTGTTCCAGTTTGGAACA
AGAGTCCACTATTAAAGAACGTGGACTCCAACGTCAAAGGGCGAAAAACCGTCTAT
CAGGGCGATGGCCCACTACGTGAACCATCACCCAAATCAAGTTTTTTGGGGTCGAGG
TGCCGTAAAGCACTAAATCGGAACCCTAAAGGGAGCCCCCGATTTAGAGCTTGACG
GGGAAAGCCGGCGAACGTGGCGAGAAAGGAAGGGAAGAAAGCGAAAGGAGCGGG
CGCTAGGGCGCTGGCAAGTGTAGCGGTCACGCTGCGCGTAACCACCACACCCGCCG
CGCTTAATGCGCCGCTACAGGGCGCGTACTATGGTTGCTTTGACGTATGCGGTGTGA
AATACCGCACAGATGCGTAAGGAGAAAATACCGCATCAGGCGCCCCTGCAGGCAGC
TGCGCGCTCGCTCGCTCACTGAGGCCGCCCGGGCAAAGCCCGGGCGTCGGGCGACC
TTTGGTCGCCCGGCCTCAGTGAGCGAGCGAGCGCGCAGAGAGGGAGTGGCCAACTC
CATCACTAGGGGTTCCTACGCGTCGTTACATAACTTACGGTAAATGGCCCGCCTGGC
TGACCGCCCAACGACCCCCGCCCATTGACGTCAATAATGACGTATGTTCCCATAGTA
ACGCCAATAGGGACTTTCCATTGACGTCAATGGGTGGAGTATTTACGGTAAACTGCC
CACTTGGCAGTACATCAAGTGTATCATATGCCAAGTACGCCCCCTATTGACGTCAAT
GACGGTAAATGGCCCGCCTGGCATTATGCCCAGTACATGACCTTATGGGACTTTCCT
ACTTGGCAGTACATCTACGTATTAGTCATCGCTATTACCATGGTCGAGGTGAGCCCC
ACGTTCTGCTTCACTCTCCCCATCTCCCCCCCCTCCCCACCCCCAATTTTGTATTTATT
TATTTTTTAATTATTTTGTGCAGCGATGGGGGCGGGGGGGGGGGGGGGGCGCGCGC
CAGGCGGGGCGGGGCGGGGCGAGGGGCGGGGCGGGGCGAGGCGGAGAGGTGCGG
CGGCAGCCAATCAGAGCGGCGCGCTCCGAAAGTTTCCTTTTATGGCGAGGCGGCGG
CGGCGGCGGCCCTATAAAAAGCGAAGCGCGCGGCGGGCGGGAGTCGCTGCGACGCT
GCCTTCGCCCCGTGCCCCGCTCCGCCGCCGCCTCGCGCCGCCCGCCCCGGCTCTGAC
TGACCGCGTTACTCCCACAGGTGAGCGGGCGGGACGGCCCTTCTCCTCCGGGCTGTA
ATTAGCTGAGCAAGAGGTAAGGGTTTAAGGGATGGTTGGTTGGTGGGGTATTAATG
TTTAATTACCTGGAGCACCTGCCTGAAATCACTTTTTTTCAGGTACCGGTCGCCACCA
TGGCGAGACCCCTGTGCACATTACTTCTGTTGATGGCTACCCTGGCAGGCGCCCTCG
CCAGCGAGCTGACACAGGACCCTGCCGTGTCCGTGGCCCTGAAGCAGACCGTGACA
ATCACCTGCAGAGGCGATTCCCTGAGATCCCACTACGCCTCCTGGTACCAGAAGAA
GCCTGGCCAGGCCCCCGTGCTGCTGTTTTACGGCAAGAATAACCGCCCCAGCGGCAT
CCCCGATAGATTTTCCGGCAGCGCCTCCGGCAACAGAGCCAGCCTGACAATCACCG
GCGCCCAGGCCGAGGACGAGGCTGATTACTACTGCAGCTCCAGAGATAAGAGCGGC
AGCAGACTGTCCGTGTTTGGCGGCGGCACCAAGCTGACCGTGCTCGGAGGAGGAGG
AAGCGGAGGAGGAGGCTCAGGCGGCGGCGGCTCTGAGGTGAGGCTGAGAGAGTCC
GGCGGCGGCCTGGTGAAGCCCGGAGGATCTCTGAGGCTGTCCTGCTCCGCCTCCGGC
 
TTCGATTTTGACAATGCCTGGATGACCTGGGTGAGACAGCCCCCTGGCAAGGGCCTG
GAGTGGGTGGGAAGGATCACAGGCCCCGGCGAGGGCTGGTCCGTGGATTACGCCGA
GTCCGTGAAGGGCAGGTTCACAATCTCCAGGGATAACACCAAGAACACCCTGTACC
TGGAGATGAACAACGTGAGGACAGAGGATACCGGCTACTACTTTTGCGCCAGAACA
GGCAAGTACTACGACTTTTGGTTCGGCTACCCCCCTGGCGAGGAGTACTTCCAGGAT
TGGGGCCAGGGCACCCTGGTCATTGTGTCCAGCGGCGGCGGCAGTGGCGGCGG
CGGAAGCGGCGGCGGCTCTGGCGGCGGCAGCGGCGGCGGCTCCGAC
ATCGTGATGACCCAGTCTCCTGATAGCCTGGCCGTGAGCCTGGGCGAGAGAGTGAC
AATGAACTGTAAGTCTAGCCAGAGCCTGCTGTACTCCACCAACCAGAAGAATTACCT
GGCCTGGTATCAGCAGAAGCCTGGCCAGTCCCCAAAGCTGCTGATCTATTGGGCATC
TACAAGGGAGAGCGGAGTGCCAGACAGATTCAGCGGATCCGGATCTGGAACCGACT
TCACCCTGACAATCTCCTCTGTGCAGGCCGAGGACGTGGCCGTGTACTATTGCCAGC
AGTACTATAGCTACAGGACATTCGGCGGCGGCACCAAGCTGGAGATCAAGCGCACC
GTGGCCGGAGGAGGAGGATCTGGCGGAGGAGGGTCCGGCGGCGGCGGCTCCCAGG
TGCAGCTGCAGCAGAGCGGACCAGAGGTGGTGAAGCCTGGAGCCTCCGTGAAGATG
TCTTGTAAGGCCAGCGGCTACACCTTCACATCCTATGTGATCCACTGGGTGAGGCAG
AAGCCAGGACAGGGACTGGACTGGATCGGCTACATCAACCCTTATAATGATGGCAC
CGACTACGATGAGAAGTTTAAGGGCAAGGCCACCCTGACATCCGATACCAGCACAT
CCACCGCCTATATGGAGCTGAGCTCCCTGCGGTCTGAGGACACAGCCGTGTACTATT
GCGCCAGAGAGAAGGATAACTACGCAACCGGAGCATGGTTCGCATATTGGGGACAG
GGTACCCTGGTCACCGTGTCTAGCGGCGGAGGAGGAAGCGGAGGAGGCGGCTCTGG
CGGAGGAGGTTCCGGAGGAGGCGGAAGCGGCGGAGGAGGCTCTTGGGAGCAGAAG
ATCGAGGAGCTGCTGAAGAAGGCCGAGGAGCAGCAGAAGAAGAATGAGGAGGAGC
TGAAGAAGCTGGAGAAGTAGGTCGACGACAATCAACCTCTGGATTACAAAATTTGT
GAAAGATTGACTGGTATTCTTAACTATGTTGCTCCTTTTACGCTATGTGGATACGCTG
CTTTAATGCCTTTGTATCATGCTATTGCTTCCCGTATGGCTTTCATTTTCTCCTCCTTG
TATAAATCCTGGTTGCTGTCTCTTTATGAGGAGTTGTGGCCCGTTGTCAGGCAACGT
GGCGTGGTGTGCACTGTGTTTGCTGACGCAACCCCCACTGGTTGGGGCATTGCCACC
ACCTGTCAGCTCCTTTCCGGGACTTTCGCTTTCCCCCTCCCTATTGCCACGGCGGAAC
TCATCGCCGCCTGCCTTGCCCGCTGCTGGACAGGGGCTCGGCTGTTGGGCACTGACA
ATTCCGTGGTGTTGTCGGGGAAGCTGACGTCCTTTCCATGGCTGCTCGCCTGTGTTGC
CACCTGGATTCTGCGCGGGACGTCCTTCTGCTACGTCCCTTCGGCCCTCAATCCAGC
GGACCTTCCTTCCCGCGGCCTGCTGCCGGCTCTGCGGCCTCTTCCGCGTCTTCGCCTT
CGCCCTCAGACGAGTCGGATCTCCCTTTGGGCCGCCTCCCCGCGAATTCGTTTATTTG
TGAAATTTGTGATGCTATTGCTTTATTTGTAACCATCTAGCTTTATTTGTGAAATTTG
TGATGCTATTGCTTTATTTGTAACCATTATAAGCTGCAATAAACAAGTTAACAACAA
CAATTGCATTCATTTTATGTTTCAGGTTCAGGGGGAGATGTGGGAGGTTTTTTAAAG
TTTAAACAGGAACCCCTAGTGATGGAGTTGGCCACTCCCTCTCTGCGCGCTCGCTCG
CTCACTGAGGCCGGGCGACCAAAGGTCGCCCGACGCCCGGGCGGCCTCAGTGAGCG
AGCGAGCGCGCAGCTGCCTGCAGG
SEQ ID NO: 17 
GGCCGCGTTGCTGGCGTTTTTCCATAGGCTCCGCCCCCCTGACGAGCATCACAAAAA
TCGACGCTCAAGTCAGAGGTGGCGAAACCCGACAGGACTATAAAGATACCAGGCGT
TTCCCCCTGGAAGCTCCCTCGTGCGCTCTCCTGTTCCGACCCTGCCGCTTACCGGATA
CCTGTCCGCCTTTCTCCCTTCGGGAAGCGTGGCGCTTTCTCATAGCTCACGCTGTAGG
TATCTCAGTTCGGTGTAGGTCGTTCGCTCCAAGCTGGGCTGTGTGCACGAACCCCCC
 
GTTCAGCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAGCGAG
GTATGTAGGCGGTGCTACAGAGTTCTTGAAGTGGTGGCCTAACTACGGCTACACTAG
AAGAACAGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTTTGTTTG
CAAGCAGCAGATTACGCGCAGAAAAAAAGGATCTCAAGAAGATCCTTTGATCTTTT
CTACGGGGTCTGACGCTCAGTGGAACGAAAACTCACGTTAAGGGATTTTGGTCATGA
AGCGCTTTTGAAGCTCGGATCCGAACAAACGACCCAACACCCGTGCGTTTTATTCTG
TCTTTTTATTGCCGATCCCCTCAGAAGAACTCGTCAAGAAGGCGATAGAAGGCGATG
CGCTGCGAATCGGGAGCGGCGATACCGTAAAGCACGAGGAAGCGGTCAGCCCATTC
GCCGCCAAGCTCTTCAGCAATATCACGGGTAGCCAACGCTATGTCCTGATAGCGGTC
CGCCACACCCAGCCGGCCACAGTCGATGAATCCAGAAAAGCGGCCATTTTCCACCA
TGATATTCGGCAAGCAGGCATCGCCATGGGTCACGACGAGATCCTCGCCGTCGGGC
ATGCTCGCCTTGAGCCTGGCGAACAGTTCGGCTGGCGCGAGCCCCTGATGCTCTTCG
TCCAGATCATCCTGATCGACAAGACCGGCTTCCATCCGAGTACGTGCTCGCTCGATG
CGATGTTTCGCTTGGTGGTCGAATGGGCAGGTAGCCGGATCAAGCGTATGCAGCCGC
CGCATTGCATCAGCCATGATGGATACTTTCTCGGCAGGAGCAAGGTGAGATGACAG
GAGATCCTGCCCCGGCACTTCGCCCAATAGCAGCCAGTCCCTTCCCGCTTCAGTGAC
AACGTCGAGCACAGCTGCGCAAGGAACGCCCGTCGTGGCCAGCCACGATAGCCGCG
CTGCCTCGTCTTGCAGTTCATTCAGGGCACCGGACAGGTCGGTCTTGACAAAAAGAA
CCGGGCGCCCCTGCGCTGACAGCCGGAACACGGCGGCATCAGAGCAGCCGATTGTC
TGTTGTGCCCAGTCATAGCCGAATAGCCTCTCCACCCAAGCGGCCGGAGAACCTGCG
TGCAATCCATCTTGTTCAATCATGCGAAACGATCCTCATCCTGTCTCTTGATCAGAGC
TTGATCCCCTGCGCCATCAGATCCTTGGCGGCAAGAAAGCCATCCAGTTTACTTTGC
AGGGCTTCCCAACCTTACCAGAGGCCTGCGCCGCGGCCAGCTGGCTAGCAATTCCCG
GGTTAACTCTAGAGACATTGATTATTGACTAGTTATTAATAGTAATCAATTACGGGG
TCATTAGTTCATAGCCCATATATGGAGTTCCGCGTTACATAACTTACGGTAAATGGC
CCGCCTGGCTGACCGCCCAACGACCCCCGCCCATTGACGTCAATAATGACGTATGTT
CCCATAGTAACGCCAATAGGGACTTTCCATTGACGTCAATGGGTGGAGTATTTACGG
TAAACTGCCCACTTGGCAGTACATCAAGTGTATCATATGCCAAGTACGCCCCCTATT
GACGTCAATGACGGTAAATGGCCCGCCTGGCATTATGCCCAGTACATGACCTTATGG
GACTTTCCTACTTGGCAGTACATCTACGTATTAGTCATCGCTATTACCATGGTGATGC
GGTTTTGGCAGTACATCAATGGGCGTGGATAGCGGTTTGACTCACGGGGATTTCCAA
GTCTCCACCCCATTGACGTCAATGGGAGTTTGTTTTGGCACCAAAATCAACGGGACT
TTCCAAAATGTCGTAACAACTCCGCCCCATTGACGCAAATGGGCGGTAGGCGTGTAC
GGTGGGAGGTCTATATAAGCAGAGCTCGTTTAGTGAACCGTCAGATCGCCTGGAGA
CGCCATCCACGCTGTTTTGACCTCCATAGAAGACACCGGGACCGATCCAGCCTCCCC
TCGAAGCTTACATGTGGTACCGAGCTCGGATCCTGAGAACTTCAGGGTGAGTCTATG
GGACCCTTGATGTTTTCTTTCCCCTTCTTTTCTATGGTTAAGTTCATGTCATAGGAAG
GGGAGAAGTAACAGGGTACACATATTGACCAAATCAGGGTAATTTTGCATTTGTAAT
TTTAAAAAATGCTTTCTTCTTTTAATATACTTTTTTGTTTATCTTATTTCTAATACTTTC
CCTAATCTCTTTCTTTCAGGGCAATAATGATACAATGTATCATGCCTCTTTGCACCAT
TCTAAAGAATAACAGTGATAATTTCTGGGTTAAGGCAATAGCAATATTTCTGCATAT
AAATATTTCTGCATATAAATTGTAACTGATGTAAGAGGTTTCATATTGCTAATAGCA
GCTACAATCCAGCTACCATTCTGCTTTTATTTTATGGTTGGGATAAGGCTGGATTATT
CTGAGTCCAAGCTAGGCCCTTTTGCTAATCATGTTCATACCTCTTATCTTCCTCCCAC
 
AGCTCCTGGGCAACGTGCTGGTCTGTGTGCTGGCCCATCACTTTGGCAAAGCACGTG
AGATCTGAATTCTGACACTATGAAGTGCCTTTTGTACTTAGCCTTTTTATTCATTGGG
GTGAATTGCAAGTTCACCATAGTTTTTCCACACAACCAAAAAGGAAACTGGAAAAA
TGTTCCTTCTAATTACCATTATTGCCCGTCAAGCTCAGATTTAAATTGGCATAATGAC
TTAATAGGCACAGCCTTACAAGTCAAAATGCCCAAGAGTCACAAGGCTATTCAAGC
AGACGGTTGGATGTGTCATGCTTCCAAATGGGTCACTACTTGTGATTTCCGCTGGTA
TGGACCGAAGTATATAACACATTCCATCCGATCCTTCACTCCATCTGTAGAACAATG
CAAGGAAAGCATTGAACAAACGAAACAAGGAACTTGGCTGAATCCAGGCTTCCCTC
CTCAAAGTTGTGGATATGCAACTGTGACGGATGCCGAAGCAGTGATTGTCCAGGTG
ACTCCTCACCATGTGCTGGTTGATGAATACACAGGAGAATGGGTTGATTCACAGTTC
ATCAACGGAAAATGCAGCAATTACATATGCCCCACTGTCCATAACTCTACAACCTGG
CATTCTGACTATAAGGTCAAAGGGCTATGTGATTCTAACCTCATTTCCATGGACATC
ACCTTCTTCTCAGAGGACGGAGAGCTATCATCCCTGGGAAAGGAGGGCACAGGGTT
CAGAAGTAACTACTTTGCTTATGAAACTGGAGGCAAGGCCTGCAAAATGCAATACT
GCAAGCATTGGGGAGTCAGACTCCCATCAGGTGTCTGGTTCGAGATGGCTGATAAG
GATCTCTTTGCTGCAGCCAGATTCCCTGAATGCCCAGAAGGGTCAAGTATCTCTGCT
CCATCTCAGACCTCAGTGGATGTAAGTCTAATTCAGGACGTTGAGAGGATCTTGGAT
TATTCCCTCTGCCAAGAAACCTGGAGCAAAATCAGAGCGGGTCTTCCAATCTCTCCA
GTGGATCTCAGCTATCTTGCTCCTAAAAACCCAGGAACCGGTCCTGCTTTCACCATA
ATCAATGGTACCCTAAAATACTTTGAGACCAGATACATCAGAGTCGATATTGCTGCT
CCAATCCTCTCAAGAATGGTCGGAATGATCAGTGGAACTACCACAGAAAGGGAACT
GTGGGATGACTGGGCACCATATGAAGACGTGGAAATTGGACCCAATGGAGTTCTGA
GGACCAGTTCAGGATATAAGTTTCCTTTATACATGATTGGACATGGTATGTTGGACT
CCGATCTTCATCTTAGCTCAAAGGCTCAGGTGTTCGAACATCCTCACATTCAAGACG
CTGCTTCGCAACTTCCTGATGATGAGAGTTTATTTTTTGGTGATACTGGGCTATCCAA
AAATCCAATCGAGCTTGTAGAAGGTTGGTTCAGTAGTTGGAAAAGCTCTATTGCCTC
TTTTTTCTTTATCATAGGGTTAATCATTGGACTATTCTTGGTTCTCCGAGTTGGTATCC
ATCTTTGCATTAAATTAAAGCACACCAAGAAAAGACAGATTTATACAGACATAGAG
ATGAACCGACTTGGAAAGTAACTCAAATCCTGCACAACAGATTCTTCATGTTTGGAC
CAAATCAACTTGTGATACCATGCTCAAAGAGGCCTCAATTATATTTGAGTTTTTAATT
TTTATGAAAAAAAAAAAAAAAAACGGAATTCACCCCACCAGTGCAGGCTGCCTATC
AGAAAGTGGTGGCTGGTGTGGCTAATGCCCTGGCCCACAAGTATCACTAAGCTCGCT
TTCTTGCTGTCCAATTTCTATTAAAGGTTCCTTTGTTCCCTAAGTCCAACTACTAAAC
TGGGGGATATTATGAAGGGCCTTGAGCATCTGGATTCTGCCTAATAAAAAACATTTA
TTTTCATTGCAATGATGTATTTAAATTATTTCTGAATATTTTACTAAAAAGGGAATGT
GGGAGGTCAGTGCATTTAAAACATAAAGAAATGAAGAGCTAGTTCAAACCTTGGGA
AAATACACTATATCTTAAACTCCATGAAAGAAGGTGAGGCTGCAAACAGCTAATGC
ACATTGGCAACAGCCCCTGATGCCTATGCCTTATTCATCCCTCAGAAAAGGATTCAA
GTAGAGGCTTGATTTGGAGGTTAAAGTTTTGCTATGCTGTATTTTAGTCGACCATTAC
TTATTGTTTTAGCTGTCCTCATGAATGTCTTTTCACTACCCATTTGCTTATCCTGCATC
TCTCAGCCTTGACTCCACTCAGTTCTCTTGCTTAGAGATACCACCTTTCCCCTGAAGT
GTTCCTTCCATGTTTTACGGCGAGATGGTTTCTCCTCGCCTGGCCACTCAGCCTTAGT
TGTCTCTGTTGTCTTATAGAGGTCTACTTGAAGAAGGAAAAACAGGGGGCATGGTTT
GACTGTCCTGTGAGCCCTTCTTCCCTGCCTCCCCCACTCACAGTGACCCGGAATCCCT
CGACATGGCAGTCTAGCACTAGTGCGGCCGCAGATCTGCTTCCTCGCTCACTGACTC
GCTGCGCTCGGTCGTTCGGCTGCGGCGAGCGGTATCAGCTCACTCAAAGGCGGTAAT
 
ACGGTTATCCACAGAATCAGGGGATAACGCAGGAAAGAACATGTGAGCAAAAGGC
CAGCAAAAGGCCAGGAACCGTAAAAA
SEQ ID NO: 18 
GGCCGCGTTGCTGGCGTTTTTCCATAGGCTCCGCCCCCCTGACGAGCATCACAAAAA
TCGACGCTCAAGTCAGAGGTGGCGAAACCCGACAGGACTATAAAGATACCAGGCGT
TTCCCCCTGGAAGCTCCCTCGTGCGCTCTCCTGTTCCGACCCTGCCGCTTACCGGATA
CCTGTCCGCCTTTCTCCCTTCGGGAAGCGTGGCGCTTTCTCATAGCTCACGCTGTAGG
TATCTCAGTTCGGTGTAGGTCGTTCGCTCCAAGCTGGGCTGTGTGCACGAACCCCCC
GTTCAGCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAGCGAG
GTATGTAGGCGGTGCTACAGAGTTCTTGAAGTGGTGGCCTAACTACGGCTACACTAG
AAGAACAGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTTTGTTTG
CAAGCAGCAGATTACGCGCAGAAAAAAAGGATCTCAAGAAGATCCTTTGATCTTTT
CTACGGGGTCTGACGCTCAGTGGAACGAAAACTCACGTTAAGGGATTTTGGTCATGA
AGCGCTTTTGAAGCTCGGATCCGAACAAACGACCCAACACCCGTGCGTTTTATTCTG
TCTTTTTATTGCCGATCCCCTCAGAAGAACTCGTCAAGAAGGCGATAGAAGGCGATG
CGCTGCGAATCGGGAGCGGCGATACCGTAAAGCACGAGGAAGCGGTCAGCCCATTC
GCCGCCAAGCTCTTCAGCAATATCACGGGTAGCCAACGCTATGTCCTGATAGCGGTC
CGCCACACCCAGCCGGCCACAGTCGATGAATCCAGAAAAGCGGCCATTTTCCACCA
TGATATTCGGCAAGCAGGCATCGCCATGGGTCACGACGAGATCCTCGCCGTCGGGC
ATGCTCGCCTTGAGCCTGGCGAACAGTTCGGCTGGCGCGAGCCCCTGATGCTCTTCG
TCCAGATCATCCTGATCGACAAGACCGGCTTCCATCCGAGTACGTGCTCGCTCGATG
CGATGTTTCGCTTGGTGGTCGAATGGGCAGGTAGCCGGATCAAGCGTATGCAGCCGC
CGCATTGCATCAGCCATGATGGATACTTTCTCGGCAGGAGCAAGGTGAGATGACAG
GAGATCCTGCCCCGGCACTTCGCCCAATAGCAGCCAGTCCCTTCCCGCTTCAGTGAC
AACGTCGAGCACAGCTGCGCAAGGAACGCCCGTCGTGGCCAGCCACGATAGCCGCG
CTGCCTCGTCTTGCAGTTCATTCAGGGCACCGGACAGGTCGGTCTTGACAAAAAGAA
CCGGGCGCCCCTGCGCTGACAGCCGGAACACGGCGGCATCAGAGCAGCCGATTGTC
TGTTGTGCCCAGTCATAGCCGAATAGCCTCTCCACCCAAGCGGCCGGAGAACCTGCG
TGCAATCCATCTTGTTCAATCATGCGAAACGATCCTCATCCTGTCTCTTGATCAGAGC
TTGATCCCCTGCGCCATCAGATCCTTGGCGGCAAGAAAGCCATCCAGTTTACTTTGC
AGGGCTTCCCAACCTTACCAGAGGCCTGCGCCGCGGCCAGCTGGCTAGCAATTCCCG
GGTTAACTCTAGAGAATGTAGTCTTATGCAATACTCTTGTAGTCTTGCAACATGGTA
ACGATGAGTTAGCAACATGCCTTACAAGGAGAGAAAAAGCACCGTGCATGCCGATT
GGTGGAAGTAAGGTGGTACGATCGTGCCTTATTAGGAAGGCAACAGACGGGTCTGA
CATGGATTGGACGAACCACTGAATTCCGCATTGCAGAGATATTGTATTTAAGTGCCT
AGCTCGATACAATAAACGCCATTTGACCATTCACCACATTGGTGTGCACCTCCAAGC
TCGAGCTCGTTTAGTGAACCGTCAGATCGCCTGGAGACGCCATCCACGCTGTTTTGA
CCTCCATAGAAGACACCGGGACCGATCCAGCCTCCCCTCGAAGCTAGTCGATTAGG
CATCTCCTATGGCAGGAAGAAGCGGAGACAGCGACGAAGACCTCCTCAAGGCAGTC
AGACTCATCAAGTTTCTCTATCAAAGCAACCCACCTCCCAATCCCGAGGGGACCCGA
CAGGCCCGAAGGAATAGAAGAAGAAGGTGGAGAGAGAGACAGAGACAGATCCATT
CGATTAGTGAACGGATCCTTAGCACTTATCTGGGACGATCTGCGGAGCCTGTGCCTC
TTCAGCTACCACCGCTTGAGAGACTTACTCTTGATTGTAACGAGGATTGTGGAACTT
CTGGGACGCAGGGGGTGGGAAGCCCTCAAATATTGGTGGAATCTCCTACAATATTG
 
GAGTCAGGAGCTAAAGAATAGTGCTGTTAGCTTGCTCAATGCCACAGCTATAGCAGT
AGCTGAGGGGACAGATAGGGTTATAGAAGTAGTACAAGAAGCTTGGCACTGGCCGT
CGTTTTACAACGTCGTGATCTGAGCCTGGGAGATCTCTGGCTAACTAGGGAACCCAC
TGCTTAAGCCTCAATAAAGCTTGCCTTGAGTGCTTCAAGTAGTGTGTGCCCGTCTGTT
GTGTGACTCTGGTAACTAGAGATCAGGAAAACCCTGGCGTTACCCAACTTAATCGCC
TTGCAGCACATCCCCCTTTCGCCAGCTGGCGTAATAGCGAAGAGGCCCGCACCGATC
GCCCTTCCCAACAGTTGCGCAGCCTGAATGGCGAATGGCGCCTGATGCGGTATTTTC
TCCTTACGCATCTGTGCGGTATTTCACACCGCATACGTCAAAGCAACCATAGTGTCG
ACCATTACTTATTGTTTTAGCTGTCCTCATGAATGTCTTTTCACTACCCATTTGCTTAT
CCTGCATCTCTCAGCCTTGACTCCACTCAGTTCTCTTGCTTAGAGATACCACCTTTCC
CCTGAAGTGTTCCTTCCATGTTTTACGGCGAGATGGTTTCTCCTCGCCTGGCCACTCA
GCCTTAGTTGTCTCTGTTGTCTTATAGAGGTCTACTTGAAGAAGGAAAAACAGGGGG
CATGGTTTGACTGTCCTGTGAGCCCTTCTTCCCTGCCTCCCCCACTCACAGTGACCCG
GAATCCCTCGACATGGCAGTCTAGCACTAGTGCGGCCGCAGATCTGCTTCCTCGCTC
ACTGACTCGCTGCGCTCGGTCGTTCGGCTGCGGCGAGCGGTATCAGCTCACTCAAAG
GCGGTAATACGGTTATCCACAGAATCAGGGGATAACGCAGGAAAGAACATGTGAGC
AAAAGGCCAGCAAAAGGCCAGGAACCGTAAAAA
SEQ ID NO: 19 
GGCCGCGTTGCTGGCGTTTTTCCATAGGCTCCGCCCCCCTGACGAGCATCACAAAAA
TCGACGCTCAAGTCAGAGGTGGCGAAACCCGACAGGACTATAAAGATACCAGGCGT
TTCCCCCTGGAAGCTCCCTCGTGCGCTCTCCTGTTCCGACCCTGCCGCTTACCGGATA
CCTGTCCGCCTTTCTCCCTTCGGGAAGCGTGGCGCTTTCTCATAGCTCACGCTGTAGG
TATCTCAGTTCGGTGTAGGTCGTTCGCTCCAAGCTGGGCTGTGTGCACGAACCCCCC
GTTCAGCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAGCGAG
GTATGTAGGCGGTGCTACAGAGTTCTTGAAGTGGTGGCCTAACTACGGCTACACTAG
AAGAACAGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTTTGTTTG
CAAGCAGCAGATTACGCGCAGAAAAAAAGGATCTCAAGAAGATCCTTTGATCTTTT
CTACGGGGTCTGACGCTCAGTGGAACGAAAACTCACGTTAAGGGATTTTGGTCATGA
AGCGCTTTTGAAGCTCGGATCCGAACAAACGACCCAACACCCGTGCGTTTTATTCTG
TCTTTTTATTGCCGATCCCCTCAGAAGAACTCGTCAAGAAGGCGATAGAAGGCGATG
CGCTGCGAATCGGGAGCGGCGATACCGTAAAGCACGAGGAAGCGGTCAGCCCATTC
GCCGCCAAGCTCTTCAGCAATATCACGGGTAGCCAACGCTATGTCCTGATAGCGGTC
CGCCACACCCAGCCGGCCACAGTCGATGAATCCAGAAAAGCGGCCATTTTCCACCA
TGATATTCGGCAAGCAGGCATCGCCATGGGTCACGACGAGATCCTCGCCGTCGGGC
ATGCTCGCCTTGAGCCTGGCGAACAGTTCGGCTGGCGCGAGCCCCTGATGCTCTTCG
TCCAGATCATCCTGATCGACAAGACCGGCTTCCATCCGAGTACGTGCTCGCTCGATG
CGATGTTTCGCTTGGTGGTCGAATGGGCAGGTAGCCGGATCAAGCGTATGCAGCCGC
CGCATTGCATCAGCCATGATGGATACTTTCTCGGCAGGAGCAAGGTGAGATGACAG
GAGATCCTGCCCCGGCACTTCGCCCAATAGCAGCCAGTCCCTTCCCGCTTCAGTGAC
AACGTCGAGCACAGCTGCGCAAGGAACGCCCGTCGTGGCCAGCCACGATAGCCGCG
CTGCCTCGTCTTGCAGTTCATTCAGGGCACCGGACAGGTCGGTCTTGACAAAAAGAA
CCGGGCGCCCCTGCGCTGACAGCCGGAACACGGCGGCATCAGAGCAGCCGATTGTC
TGTTGTGCCCAGTCATAGCCGAATAGCCTCTCCACCCAAGCGGCCGGAGAACCTGCG
TGCAATCCATCTTGTTCAATCATGCGAAACGATCCTCATCCTGTCTCTTGATCAGAGC
 
TTGATCCCCTGCGCCATCAGATCCTTGGCGGCAAGAAAGCCATCCAGTTTACTTTGC
AGGGCTTCCCAACCTTACCAGAGGCCTGCGCCGCGGCCAGCTGGCTAGCAATTCCCG
GGTTAACTCTAGAGACATTGATTATTGACTAGTTATTAATAGTAATCAATTACGGGG
TCATTAGTTCATAGCCCATATATGGAGTTCCGCGTTACATAACTTACGGTAAATGGC
CCGCCTGGCTGACCGCCCAACGACCCCCGCCCATTGACGTCAATAATGACGTATGTT
CCCATAGTAACGCCAATAGGGACTTTCCATTGACGTCAATGGGTGGAGTATTTACGG
TAAACTGCCCACTTGGCAGTACATCAAGTGTATCATATGCCAAGTACGCCCCCTATT
GACGTCAATGACGGTAAATGGCCCGCCTGGCATTATGCCCAGTACATGACCTTATGG
GACTTTCCTACTTGGCAGTACATCTACGTATTAGTCATCGCTATTACCATGGTGATGC
GGTTTTGGCAGTACATCAATGGGCGTGGATAGCGGTTTGACTCACGGGGATTTCCAA
GTCTCCACCCCATTGACGTCAATGGGAGTTTGTTTTGGCACCAAAATCAACGGGACT
TTCCAAAATGTCGTAACAACTCCGCCCCATTGACGCAAATGGGCGGTAGGCGTGTAC
GGTGGGAGGTCTATATAAGCAGAGCTCGTTTAGTGAACCGTCAGATCGCCTGGAGA
CGCCATCCACGCTGTTTTGACCTCCATAGAAGACACCGGGACCGATCCAGCCTCCCC
TCGAAGCTTACATGTGGTACCGAGCTCGGATCCTGAGAACTTCAGGGTGAGTCTATG
GGACCCTTGATGTTTTCTTTCCCCTTCTTTTCTATGGTTAAGTTCATGTCATAGGAAG
GGGAGAAGTAACAGGGTACACATATTGACCAAATCAGGGTAATTTTGCATTTGTAAT
TTTAAAAAATGCTTTCTTCTTTTAATATACTTTTTTGTTTATCTTATTTCTAATACTTTC
CCTAATCTCTTTCTTTCAGGGCAATAATGATACAATGTATCATGCCTCTTTGCACCAT
TCTAAAGAATAACAGTGATAATTTCTGGGTTAAGGCAATAGCAATATTTCTGCATAT
AAATATTTCTGCATATAAATTGTAACTGATGTAAGAGGTTTCATATTGCTAATAGCA
GCTACAATCCAGCTACCATTCTGCTTTTATTTTATGGTTGGGATAAGGCTGGATTATT
CTGAGTCCAAGCTAGGCCCTTTTGCTAATCATGTTCATACCTCTTATCTTCCTCCCAC
AGCTCCTGGGCAACGTGCTGGTCTGTGTGCTGGCCCATCACTTTGGCAAAGCACGTG
AGATCTGAATTCGAGATCTGCCGCCGCCATGGGTGCGAGAGCGTCAGTATTAAGCG
GGGGAGAATTAGATCGATGGGAAAAAATTCGGTTAAGGCCAGGGGGAAAGAAAAA
ATATAAATTAAAACATATAGTATGGGCAAGCAGGGAGCTAGAACGATTCGCAGTTA
ATCCTGGCCTGTTAGAAACATCAGAAGGCTGTAGACAAATACTGGGACAGCTACAA
CCATCCCTTCAGACAGGATCAGAAGAACTTAGATCATTATATAATACAGTAGCAACC
CTCTATTGTGTGCATCAAAGGATAGAGATAAAAGACACCAAGGAAGCTTTAGACAA
GATAGAGGAAGAGCAAAACAAAAGTAAGAAAAAAGCACAGCAAGCAGCAGCTGAC
ACAGGACACAGCAATCAGGTCAGCCAAAATTACCCTATAGTGCAGAACATCCAGGG
GCAAATGGTACATCAGGCCATATCACCTAGAACTTTAAATGCATGGGTAAAAGTAG
TAGAAGAGAAGGCTTTCAGCCCAGAAGTGATACCCATGTTTTCAGCATTATCAGAAG
GAGCCACCCCACAAGATTTAAACACCATGCTAAACACAGTGGGGGGACATCAAGCA
GCCATGCAAATGTTAAAAGAGACCATCAATGAGGAAGCTGCAGAATGGGATAGAGT
GCATCCAGTGCATGCAGGGCCTATTGCACCAGGCCAGATGAGAGAACCAAGGGGAT
CAGACATCGCTGGAACTACTAGTACCCTTCAGGAACAAATAGGATGGATGACACAT
AATCCACCTATCCCAGTAGGAGAAATCTATAAAAGATGGATAATCCTGGGATTAAA
TAAAATAGTAAGAATGTATAGCCCTACCAGCATTCTGGACATAAGACAAGGACCAA
AGGAACCCTTTAGAGACTATGTAGACCGATTCTATAAAACTCTAAGAGCCGAGCAA
GCTTCACAAGAGGTAAAAAATTGGATGACAGAAACCTTGTTGGTCCAAAATGCGAA
CCCAGATTGTAAGACTATTTTAAAAGCATTGGGACCAGGAGCGACACTAGAAGAAA
TGATGACAGCATGTCAGGGAGTGGGGGGACCCGGCCATAAAGCAAGAGTTTTGGCT
GAAGCAATGAGCCAAGTAACAAATCCAGCTACCATAATGATACAGAAAGGCAATTT
TAGGAACCAAAGAAAGACTGTTAAGTGTTTCAATTGTGGCAAAGAAGGGCACATAG
 
CCAAAAATTGCAGGGCCCCTAGGAAAAAGGGCTGTTGGAAATGTGGAAAGGAAGG
ACACCAAATGAAAGATTGTACTGAGAGACAGGCTAATTTTTTAGGGAAGATCTGGC
CTTCCCACAAGGGAAGGCCAGGGAATTTTCTTCAGAGCAGACCAGAGCCAACAGCC
CCACCAGAAGAGAGCTTCAGGTTTGGGGAAGAGACAACAACTCCCTCTCAGAAGCA
GGAGCCGATAGACAAGGAACTGTATCCTTTAGCTTCCCTCAGATCACTCTTTGGCAG
CGACCCCTCGTCACAATAAAGATAGGGGGGCAATTAAAGGAAGCTCTATTAGATAC
TGGTGCTGACGACACAGTATTAGAAGAAATGAATTTGCCAGGAAGATGGAAACCAA
AAATGATAGGGGGAATTGGAGGTTTTATCAAAGTAAGACAGTATGATCAGATACTC
ATAGAAATCTGCGGACATAAAGCTATAGGTACAGTATTAGTAGGACCTACACCTGTC
AACATAATTGGAAGAAATCTGTTGACTCAGATTGGCTGCACTTTAAATTTTCCCATT
AGTCCTATTGAGACTGTACCAGTAAAATTAAAGCCAGGAATGGATGGCCCAAAAGT
TAAACAATGGCCATTGACAGAAGAAAAAATAAAAGCATTAGTAGAAATTTGTACAG
AAATGGAAAAGGAAGGAAAAATTTCAAAAATTGGGCCTGAAAATCCATACAATACT
CCAGTATTTGCCATAAAGAAAAAAGACAGTACTAAATGGAGAAAATTAGTAGATTT
CAGAGAACTTAATAAGAGAACTCAAGATTTCTGGGAAGTTCAATTAGGAATACCAC
ATCCTGCAGGGTTAAAACAGAAAAAATCAGTAACAGTACTGGATGTGGGCGATGCA
TATTTTTCAGTTCCCTTAGATAAAGACTTCAGGAAGTATACTGCATTTACCATACCTA
GTATAAACAATGAGACACCAGGGATTAGATATCAGTACAATGTGCTTCCACAGGGA
TGGAAAGGATCACCAGCAATATTCCAGTGTAGCATGACAAAAATCTTAGAGCCTTTT
AGAAAACAAAATCCAGACATAGTCATCTATCAATACATGGATGATTTGTATGTAGG
ATCTGACTTAGAAATAGGGCAGCATAGAACAAAAATAGAGGAACTGAGACAACATC
TGTTGAGGTGGGGATTTACCACACCAGACAAAAAACATCAGAAAGAACCTCCATTC
CTTTGGATGGGTTATGAACTCCATCCTGATAAATGGACAGTACAGCCTATAGTGCTG
CCAGAAAAGGACAGCTGGACTGTCAATGACATACAGAAATTAGTGGGAAAATTGAA
TTGGGCAAGTCAGATTTATGCAGGGATTAAAGTAAGGCAATTATGTAAACTTCTTAG
GGGAACCAAAGCACTAACAGAAGTAGTACCACTAACAGAAGAAGCAGAGCTAGAA
CTGGCAGAAAACAGGGAGATTCTAAAAGAACCGGTACATGGAGTGTATTATGACCC
ATCAAAAGACTTAATAGCAGAAATACAGAAGCAGGGGCAAGGCCAATGGACATATC
AAATTTATCAAGAGCCATTTAAAAATCTGAAAACAGGAAAGTATGCAAGAATGAAG
GGTGCCCACACTAATGATGTGAAACAATTAACAGAGGCAGTACAAAAAATAGCCAC
AGAAAGCATAGTAATATGGGGAAAGACTCCTAAATTTAAATTACCCATACAAAAGG
AAACATGGGAAGCATGGTGGACAGAGTATTGGCAAGCCACCTGGATTCCTGAGTGG
GAGTTTGTCAATACCCCTCCCTTAGTGAAGTTATGGTACCAGTTAGAGAAAGAACCC
ATAATAGGAGCAGAAACTTTCTATGTAGATGGGGCAGCCAATAGGGAAACTAAATT
AGGAAAAGCAGGATATGTAACTGACAGAGGAAGACAAAAAGTTGTCCCCCTAACGG
ACACAACAAATCAGAAGACTGAGTTACAAGCAATTCATCTAGCTTTGCAGGATTCG
GGATTAGAAGTAAACATAGTGACAGACTCACAATATGCATTGGGAATCATTCAAGC
ACAACCAGATAAGAGTGAATCAGAGTTAGTCAGTCAAATAATAGAGCAGTTAATAA
AAAAGGAAAAAGTCTACCTGGCATGGGTACCAGCACACAAAGGAATTGGAGGAAA
TGAACAAGTAGATAAATTGGTCAGTGCTGGAATCAGGAAAGTACTATTTTTAGATGG
AATAGATAAGGCCCAAGAAGAACATGAGAAATATCACAGTAATTGGAGAGCAATG
GCTAGTGATTTTAACCTACCACCTGTAGTAGCAAAAGAAATAGTAGCCAGCTGTGAT
AAATGTCAGCTAAAAGGGGAAGCCATGCATGGACAAGTAGACTGTAGCCCAGGAAT
ATGGCAGCTAGATTGTACACATTTAGAAGGAAAAGTTATCTTGGTAGCAGTTCATGT
AGCCAGTGGATATATAGAAGCAGAAGTAATTCCAGCAGAGACAGGGCAAGAAACA
GCATACTTCCTCTTAAAATTAGCAGGAAGATGGCCAGTAAAAACAGTACATACAGA
 
CAATGGCAGCAATTTCACCAGTACTACAGTTAAGGCCGCCTGTTGGTGGGCGGGGAT
CAAGCAGGAATTTGGCATTCCCTACAATCCGCAGTCACAAGGAGTAATAGAATCTAT
GAATAAAGAATTAAAGAAAATTATAGGACAGGTAAGAGATCAGGCTGAACATCTTA
AAACAGCAGTACAAATGGCAGTATTCATCCACAATTTTAAAAGAAAAGGGGGGATT
GGGGGGTACAGTGCAGGGGAAAGAATAGTAGACATAATAGCAACAGACATACAAA
CTAAAGAATTACAAAAACAAATTACAAAAATTCAAAATTTTCGGGTTTATTACAGGG
ACAGCAGAGATCCAGTTTGGAAAGGACCAGCAAAGCTCCTCTGGAAAGGTGAAGGG
GCAGTAGTAATACAAGATAATAGTGACATAAAAGTAGTGCCAAGAAGAAAAGCAA
AGATCATCAGGGATTATGGAAAACAGATGGCAGGTGATGATTGTGTGGCAAGTAGA
CAGGATGAGGATTAACACATGGAATTCCGGAGCGGCCGCAGGAGCTTTGTTCCTTG
GGTTCTTGGGAGCAGCAGGAAGCACTATGGGCGCAGCGTCAATGACGCTGACGGTA
CAGGCCAGACAATTATTGTCTGGTATAGTGCAGCAGCAGAACAATTTGCTGAGGGCT
ATTGAGGCGCAACAGCATCTGTTGCAACTCACAGTCTGGGGCATCAAGCAGCTCCA
GGCAAGAATCCTGGCTGTGGAAAGATACCTAAAGGATCAACAGCTCCTGGGGATTT
GGGGTTGCTCTGGAAAACTCATTTGCACCACTGCTGTGCCTTGGAATGCTAGTTGGA
GTAATAAATCTCTGGAACAGATTTGGAATCACACGACCTGGATGGAGTGGGACAGA
GAAATTAACAATTACACAAGCTTCCGCGGAATTCACCCCACCAGTGCAGGCTGCCTA
TCAGAAAGTGGTGGCTGGTGTGGCTAATGCCCTGGCCCACAAGTATCACTAAGCTCG
CTTTCTTGCTGTCCAATTTCTATTAAAGGTTCCTTTGTTCCCTAAGTCCAACTACTAA
ACTGGGGGATATTATGAAGGGCCTTGAGCATCTGGATTCTGCCTAATAAAAAACATT
TATTTTCATTGCAATGATGTATTTAAATTATTTCTGAATATTTTACTAAAAAGGGAAT
GTGGGAGGTCAGTGCATTTAAAACATAAAGAAATGAAGAGCTAGTTCAAACCTTGG
GAAAATACACTATATCTTAAACTCCATGAAAGAAGGTGAGGCTGCAAACAGCTAAT
GCACATTGGCAACAGCCCCTGATGCCTATGCCTTATTCATCCCTCAGAAAAGGATTC
AAGTAGAGGCTTGATTTGGAGGTTAAAGTTTTGCTATGCTGTATTTTACATTACTTAT
TGTTTTAGCTGTCCTCATGAATGTCTTTTCACTACCCATTTGCTTATCCTGCATCTCTC
AGCCTTGACTCCACTCAGTTCTCTTGCTTAGAGATACCACCTTTCCCCTGAAGTGTTC
CTTCCATGTTTTACGGCGAGATGGTTTCTCCTCGCCTGGCCACTCAGCCTTAGTTGTC
TCTGTTGTCTTATAGAGGTCTACTTGAAGAAGGAAAAACAGGGGGCATGGTTTGACT
GTCCTGTGAGCCCTTCTTCCCTGCCTCCCCCACTCACAGTGACCCGGAATCCCTCGAC
ATGGCAGTCTAGCACTAGTGCGGCCGCAGATCTGCTTCCTCGCTCACTGACTCGCTG
CGCTCGGTCGTTCGGCTGCGGCGAGCGGTATCAGCTCACTCAAAGGCGGTAATACG
GTTATCCACAGAATCAGGGGATAACGCAGGAAAGAACATGTGAGCAAAAGGCCAG
CAAAAGGCCAGGAACCGTAAAAAGTCGACCATTACTTATTGTTTTAGCTGTCCTCAT
GAATGTCTTTTCACTACCCATTTGCTTATCCTGCATCTCTCAGCCTTGACTCCACTCA
GTTCTCTTGCTTAGAGATACCACCTTTCCCCTGAAGTGTTCCTTCCATGTTTTACGGC
GAGATGGTTTCTCCTCGCCTGGCCACTCAGCCTTAGTTGTCTCTGTTGTCTTATAGAG
GTCTACTTGAAGAAGGAAAAACAGGGGGCATGGTTTGACTGTCCTGTGAGCCCTTCT
TCCCTGCCTCCCCCACTCACAGTGACCCGGAATCCCTCGACATGGCAGTCTAGCACT
AGTGCGGCCGCAGATCTGCTTCCTCGCTCACTGACTCGCTGCGCTCGGTCGTTCGGC
TGCGGCGAGCGGTATCAGCTCACTCAAAGGCGGTAATACGGTTATCCACAGAATCA
GGGGATAACGCAGGAAAGAACATGTGAGCAAAAGGCCAGCAAAAGGCCAGGAACC
GTAAAAA
SEQ ID NO: 20 
ATGCCGGGGTTTTACGAGATTGTGATTAAGGTCCCCAGCGACCTTGACGAGCATCTG
CCCGGCATTTCTGACAGCTTTGTGAACTGGGTGGCCGAGAAGGAATGGGAGTTGCC
 
GCCAGATTCTGACATGGATCTGAATCTGATTGAGCAGGCACCCCTGACCGTGGCCGA
GAAGCTGCAGCGCGACTTTCTGACGGAATGGCGCCGTGTGAGTAAGGCCCCGGAGG
CTCTTTTCTTTGTGCAATTTGAGAAGGGAGAGAGCTACTTCCACATGCACGTGCTCG
TGGAAACCACCGGGGTGAAATCCATGGTTTTGGGACGTTTCCTGAGTCAGATTCGCG
AAAAACTGATTCAGAGAATTTACCGCGGGATCGAGCCGACTTTGCCAAACTGGTTCG
CGGTCACAAAGACCAGAAATGGCGCCGGAGGCGGGAACAAGGTGGTGGATGAGTG
CTACATCCCCAATTACTTGCTCCCCAAAACCCAGCCTGAGCTCCAGTGGGCGTGGAC
TAATATGGAACAGTATTTAAGCGCCTGTTTGAATCTCACGGAGCGTAAACGGTTGGT
GGCGCAGCATCTGACGCACGTGTCGCAGACGCAGGAGCAGAACAAAGAGAATCAG
AATCCCAATTCTGATGCGCCGGTGATCAGATCAAAAACTTCAGCCAGGTACATGGA
GCTGGTCGGGTGGCTCGTGGACAAGGGGATTACCTCGGAGAAGCAGTGGATCCAGG
AGGACCAGGCCTCATACATCTCCTTCAATGCGGCCTCCAACTCGCGGTCCCAAATCA
AGGCTGCCTTGGACAATGCGGGAAAGATTATGAGCCTGACTAAAACCGCCCCCGAC
TACCTGGTGGGCCAGCAGCCCGTGGAGGACATTTCCAGCAATCGGATTTATAAAATT
TTGGAACTAAACGGGTACGATCCCCAATATGCGGCTTCCGTCTTTCTGGGATGGGCC
ACGAAAAAGTTCGGCAAGAGGAACACCATCTGGCTGTTTGGGCCTGCAACTACCGG
GAAGACCAACATCGCGGAGGCCATAGCCCACACTGTGCCCTTCTACGGGTGCGTAA
ACTGGACCAATGAGAACTTTCCCTTCAACGACTGTGTCGACAAGATGGTGATCTGGT
GGGAGGAGGGGAAGATGACCGCCAAGGTCGTGGAGTCGGCCAAAGCCATTCTCGGA
GGAAGCAAGGTGCGCGTGGACCAGAAATGCAAGTCCTCGGCCCAGATAGACCCGAC
TCCCGTGATCGTCACCTCCAACACCAACATGTGCGCCGTGATTGACGGGAACTCAAC
GACCTTCGAACACCAGCAGCCGTTGCAAGACCGGATGTTCAAATTTGAACTCACCCG
CCGTCTGGATCATGACTTTGGGAAGGTCACCAAGCAGGAAGTCAAAGACTTTTTCCG
GTGGGCAAAGGATCACGTGGTTGAGGTGGAGCATGAATTCTACGTCAAAAAGGGTG
GAGCCAAGAAAAGACCCGCCCCCAGTGACGCAGATATAAGTGAGCCCAAACGGGT
GCGCGAGTCAGTTGCGCAGCCATCGACGTCAGACGCGGAAGCTTCGATCAACTACG
CAGACAGGTACCAAAACAAATGTTCTCGTCACGTGGGCATGAATCTGATGCTGTTTC
CCTGCAGACAATGCGAGAGAATGAATCAGAATTCAAATATCTGCTTCACTCACGGA
CAGAAAGACTGTTTAGAGTGCTTTCCCGTGTCAGAATCTCAACCCGTTTCTGTCGTC
AAAAAGGCGTATCAGAAACTGTGCTACATTCATCATATCATGGGAAAGGTGCCAGA
CGCTTGCACTGCCTGCGATCTGGTCAATGTGGATTTGGATGACTGCATCTTTGAACA
ATAAATGATTTAAATCAGGTATGGCTGCCGATGGTTATCTTCCAGATTGGCTCGAGG
ACAACCTCTCTGAGGGCATTCGCGAGTGGTGGGCGCTGAAACCTGGAGCCCCGAAG
CCCAAAGCCAACCAGCAAAAGCAGGACGACGGCCGGGGTCTGGTGCTTCCTGGCTA
CAAGTACCTCGGACCCTTCAACGGACTCGACAAGGGGGAGCCCGTCAACGCGGCGG
ACGCAGCGGCCCTCGAGCACGACAAGGCCTACGACCAGCAGCTGCAGGCGGGTGAC
AATCCGTACCTGCGGTATAACCACGCCGACGCCGAGTTTCAGGAGCGTCTGCAAGA
AGATACGTCTTTTGGGGGCAACCTCGGGCGAGCAGTCTTCCAGGCCAAGAAGCGGG
TTCTCGAACCTCTCGGTCTGGTTGAGGAAGGCGCTAAGACGGCTCCTGGAAAGAAG
AGACCGGTAGAGCCATCACCCCAGCGTTCTCCAGACTCCTCTACGGGCATCGGCAA
GAAAGGCCAACAGCCCGCCAGAAAAAGACTCAATTTTGGTCAGACTGGCGACTCAG
AGTCAGTTCCAGACCCTCAACCTCTCGGAGAACCTCCAGCAGCGCCCTCTGGTGTGG
GACCTAATACAATGGCTGCAGGCGGTGGCGCACCAATGGCAGACAATAACGAAGGC
GCCGACGGAGTGGGTAGTTCCTCGGGAAATTGGCATTGCGATTCCACATGGCTGGGC
GACAGAGTCATCACCACCAGCACCCGAACCTGGGCCCTGCCCACCTACAACAACCA
CCTCTACAAGCAAATCTCCAACGGGACATCGGGAGGAGCCACCAACGACAACACCT
 
ACTTCGGCTACAGCACCCCCTGGGGGTATTTTGACTTTAACAGATTCCACTGCCACTT
TTCACCACGTGACTGGCAGCGACTCATCAACAACAACTGGGGATTCCGGCCCAAGA
GACTCAGCTTCAAGCTCTTCAACATCCAGGTCAAGGAGGTCACGCAGAATGAAGGC
ACCAAGACCATCGCCAATAACCTCACCAGCACCATCCAGGTGTTTACGGACTCGGA
GTACCAGCTGCCGTACGTTCTCGGCTCTGCCCACCAGGGCTGCCTGCCTCCGTTCCC
GGCGGACGTGTTCATGATTCCCCAGTACGGCTACCTAACACTCAACAACGGTAGTCA
GGCCGTGGGACGCTCCTCCTTCTACTGCCTGGAATACTTTCCTTCGCAGATGCTGAG
AACCGGCAACAACTTCCAGTTTACTTACACCTTCGAGGACGTGCCTTTCCACAGCAG
CTACGCCCACAGCCAGAGCTTGGACCGGCTGATGAATCCTCTGATTGACCAGTACCT
GTACTACTTGTCTCGGACTCAAACAACAGGAGGCACGGCAAATACGCAGACTCTGG
GCTTCAGCCAAGGTGGGCCTAATACAATGGCCAATCAGGCAAAGAACTGGCTGCCA
GGACCCTGTTACCGCCAACAACGCGTCTCAACGACAACCGGGCAAAACAACAATAG
CAACTTTGCCTGGACTGCTGGGACCAAATACCATCTGAATGGAAGAAATTCATTGGC
TAATCCTGGCATCGCTATGGCAACACACAAAGACGACGAGGAGCGTTTTTTTCCCAG
TAACGGGATCCTGATTTTTGGCAAACAAAATGCTGCCAGAGACAATGCGGATTACA
GCGATGTCATGCTCACCAGCGAGGAAGAAATCAAAACCACTAACCCTGTGGCTACA
GAGGAATACGGTATCGTGGCAGATAACTTGCAGCAGCAAAACACGGCTCCTCAAAT
TGGAACTGTCAACAGCCAGGGGGCCTTACCCGGTATGGTCTGGCAGAACCGGGACG
TGTACCTGCAGGGTCCCATCTGGGCCAAGATTCCTCACACGGACGGCAACTTCCACC
CGTCTCCGCTGATGGGCGGCTTTGGCCTGAAACATCCTCCGCCTCAGATCCTGATCA
AGAACACGCCTGTACCTGCGGATCCTCCGACCACCTTCAACCAGTCAAAGCTGAACT
CTTTCATCACGCAATACAGCACCGGACAGGTCAGCGTGGAAATTGAATGGGAGCTG
CAGAAGGAAAACAGCAAGCGCTGGAACCCCGAGATCCAGTACACCTCCAACTACTA
CAAATCTACAAGTGTGGACTTTGCTGTTAATACAGAAGGCGTGTACTCTGAACCCCG
CCCCATTGGCACCCGTTACCTCACCCGTAATCTGTAATTGCCTGTTAATCAATAAAC
CGGTTGATTCGTTTCAGTTGAACTTTGGTCTCTGCGAAGGGCGAATTCGTTTAAACCT
GCAGGACTAGAGGTCCTGTATTAGAGGTCACGTGAGTGTTTTGCGACATTTTGCGAC
ACCATGTGGTCACGCTGGGTATTTAAGCCCGAGTGAGCACGCAGGGTCTCCATTTTG
AAGCGGGAGGTTTGAACGCGCAGCCGCCAAGCCGAATTCTGCAGATATCCATCACA
CTGGCGGCCGCTCGACTAGAGCGGCCGCCACCGCGGTGGAGCTCCAGCTTTTGTTCC
CTTTAGTGAGGGTTAATTGCGCGCTTGGCGTAATCATGGTCATAGCTGTTTCCTGTGT
GAAATTGTTATCCGCTCACAATTCCACACAACATACGAGCCGGAAGCATAAAGTGT
AAAGCCTGGGGTGCCTAATGAGTGAGCTAACTCACATTAATTGCGTTGCGCTCACTG
CCCGCTTTCCAGTCGGGAAACCTGTCGTGCCAGCTGCATTAATGAATCGGCCAACGC
GCGGGGAGAGGCGGTTTGCGTATTGGGCGCTCTTCCGCTTCCTCGCTCACTGACTCG
CTGCGCTCGGTCGTTCGGCTGCGGCGAGCGGTATCAGCTCACTCAAAGGCGGTAATA
CGGTTATCCACAGAATCAGGGGATAACGCAGGAAAGAACATGTGAGCAAAAGGCC
AGCAAAAGGCCAGGAACCGTAAAAAGGCCGCGTTGCTGGCGTTTTTCCATAGGCTC
CGCCCCCCTGACGAGCATCACAAAAATCGACGCTCAAGTCAGAGGTGGCGAAACCC
GACAGGACTATAAAGATACCAGGCGTTTCCCCCTGGAAGCTCCCTCGTGCGCTCTCC
TGTTCCGACCCTGCCGCTTACCGGATACCTGTCCGCCTTTCTCCCTTCGGGAAGCGTG
GCGCTTTCTCATAGCTCACGCTGTAGGTATCTCAGTTCGGTGTAGGTCGTTCGCTCCA
AGCTGGGCTGTGTGCACGAACCCCCCGTTCAGCCCGACCGCTGCGCCTTATCCGGTA
ACTATCGTCTTGAGTCCAACCCGGTAAGACACGACTTATCGCCACTGGCAGCAGCCA
CTGGTAACAGGATTAGCAGAGCGAGGTATGTAGGCGGTGCTACAGAGTTCTTGAAG
TGGTGGCCTAACTACGGCTACACTAGAAGAACAGTATTTGGTATCTGCGCTCTGCTG
 
AAGCCAGTTACCTTCGGAAAAAGAGTTGGTAGCTCTTGATCCGGCAAACAAACCAC
CGCTGGTAGCGGTGGTTTTTTTGTTTGCAAGCAGCAGATTACGCGCAGAAAAAAAGG
ATCTCAAGAAGATCCTTTGATCTTTTCTACGGGGTCTGACGCTCAGTGGAACGAAAA
CTCACGTTAAGGGATTTTGGTCATGAGATTATCAAAAAGGATCTTCACCTAGATCCT
TTTAAATTAAAAATGAAGTTTTAAATCAATCTAAAGTATATATGAGTAAACTTGGTC
TGACAGTTACCAATGCTTAATCAGTGAGGCACCTATCTCAGCGATCTGTCTATTTCGT
TCATCCATAGTTGCCTGACTCCCCGTCGTGTAGATAACTACGATACGGGAGGGCTTA
CCATCTGGCCCCAGTGCTGCAATGATACCGCGAGACCCACGCTCACCGGCTCCAGAT
TTATCAGCAATAAACCAGCCAGCCGGAAGGGCCGAGCGCAGAAGTGGTCCTGCAAC
TTTATCCGCCTCCATCCAGTCTATTAATTGTTGCCGGGAAGCTAGAGTAAGTAGTTC
GCCAGTTAATAGTTTGCGCAACGTTGTTGCCATTGCTACAGGCATCGTGGTGTCACG
CTCGTCGTTTGGTATGGCTTCATTCAGCTCCGGTTCCCAACGATCAAGGCGAGTTAC
ATGATCCCCCATGTTGTGCAAAAAAGCGGTTAGCTCCTTCGGTCCTCCGATCGTTGT
CAGAAGTAAGTTGGCCGCAGTGTTATCACTCATGGTTATGGCAGCACTGCATAATTC
TCTTACTGTCATGCCATCCGTAAGATGCTTTTCTGTGACTGGTGAGTACTCAACCAAG
TCATTCTGAGAATAGTGTATGCGGCGACCGAGTTGCTCTTGCCCGGCGTCAATACGG
GATAATACCGCGCCACATAGCAGAACTTTAAAAGTGCTCATCATTGGAAAACGTTCT
TCGGGGCGAAAACTCTCAAGGATCTTACCGCTGTTGAGATCCAGTTCGATGTAACCC
ACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATG
TTGAATACTCATACTCTTCCTTTTTCAATATTATTGAAGCATTTATCAGGGTTATTGT
CTCATGAGCGGATACATATTTGAATGTATTTAGAAAAATAAACAAATAGGGGTTCCG
CGCACATTTCCCCGAAAAGTGCCACCTAAATTGTAAGCGTTAATATTTTGTTAAAAT
TCGCGTTAAATTTTTGTTAAATCAGCTCATTTTTTAACCAATAGGCCGAAATCGGCA
AAATCCCTTATAAATCAAAAGAATAGACCGAGATAGGGTTGAGTGTTGTTCCAGTTT
GGAACAAGAGTCCACTATTAAAGAACGTGGACTCCAACGTCAAAGGGCGAAAAACC
GTCTATCAGGGCGATGGCCCACTACGTGAACCATCACCCTAATCAAGTTTTTTGGGG
TCGAGGTGCCGTAAAGCACTAAATCGGAACCCTAAAGGGAGCCCCCGATTTAGAGC
TTGACGGGGAAAGCCGGCGAACGTGGCGAGAAAGGAAGGGAAGAAAGCGAAAGGA
GCGGGCGCTAGGGCGCTGGCAAGTGTAGCGGTCACGCTGCGCGTAACCACCACACC
CGCCGCGCTTAATGCGCCGCTACAGGGCGCGTCCCATTCGCCATTCAGGCTGCGCAA
CTGTTGGGAAGGGCGATCGGTGCGGGCCTCTTCGCTATTACGCCAGCTGGCGAAAG
GGGGATGTGCTGCAAGGCGATTAAGTTGGGTAACGCCAGGGTTTTCCCAGTCACGA
CGTTGTAAAACGACGGCCAGTGAGCGCGCGTAATACGACTCACTATAGGGCGAATT
GGGTACCGGGCCCCCCCTCGATCGAGGTCGACGGTATCGGGGGAGCTCGCAGGGTC
TCCATTTTGAAGCGGGAGGTTTGAACGCGCAGCCGCC
SEQ ID NO: 21 
GGTACCCAACTCCATGCTTAACAGTCCCCAGGTACAGCCCACCCTGCGTCGCAACCA
GGAACAGCTCTACAGCTTCCTGGAGCGCCACTCGCCCTACTTCCGCAGCCACAGTGC
GCAGATTAGGAGCGCCACTTCTTTTTGTCACTTGAAAAACATGTAAAAATAATGTAC
TAGGAGACACTTTCAATAAAGGCAAATGTTTTTATTTGTACACTCTCGGGTGATTATT
TACCCCCCACCCTTGCCGTCTGCGCCGTTTAAAAATCAAAGGGGTTCTGCCGCGCAT
CGCTATGCGCCACTGGCAGGGACACGTTGCGATACTGGTGTTTAGTGCTCCACTTAA
ACTCAGGCACAACCATCCGCGGCAGCTCGGTGAAGTTTTCACTCCACAGGCTGCGCA
CCATCACCAACGCGTTTAGCAGGTCGGGCGCCGATATCTTGAAGTCGCAGTTGGGGC
CTCCGCCCTGCGCGCGCGAGTTGCGATACACAGGGTTGCAGCACTGGAACACTATC
 
AGCGCCGGGTGGTGCACGCTGGCCAGCACGCTCTTGTCGGAGATCAGATCCGCGTC
CAGGTCCTCCGCGTTGCTCAGGGCGAACGGAGTCAACTTTGGTAGCTGCCTTCCCAA
AAAGGGTGCATGCCCAGGCTTTGAGTTGCACTCGCACCGTAGTGGCATCAGAAGGT
GACCGTGCCCGGTCTGGGCGTTAGGATACAGCGCCTGCATGAAAGCCTTGATCTGCT
TAAAAGCCACCTGAGCCTTTGCGCCTTCAGAGAAGAACATGCCGCAAGACTTGCCG
GAAAACTGATTGGCCGGACAGGCCGCGTCATGCACGCAGCACCTTGCGTCGGTGTT
GGAGATCTGCACCACATTTCGGCCCCACCGGTTCTTCACGATCTTGGCCTTGCTAGA
CTGCTCCTTCAGCGCGCGCTGCCCGTTTTCGCTCGTCACATCCATTTCAATCACGTGC
TCCTTATTTATCATAATGCTCCCGTGTAGACACTTAAGCTCGCCTTCGATCTCAGCGC
AGCGGTGCAGCCACAACGCGCAGCCCGTGGGCTCGTGGTGCTTGTAGGTTACCTCTG
CAAACGACTGCAGGTACGCCTGCAGGAATCGCCCCATCATCGTCACAAAGGTCTTGT
TGCTGGTGAAGGTCAGCTGCAACCCGCGGTGCTCCTCGTTTAGCCAGGTCTTGCATA
CGGCCGCCAGAGCTTCCACTTGGTCAGGCAGTAGCTTGAAGTTTGCCTTTAGATCGT
TATCCACGTGGTACTTGTCCATCAACGCGCGCGCAGCCTCCATGCCCTTCTCCCACG
CAGACACGATCGGCAGGCTCAGCGGGTTTATCACCGTGCTTTCACTTTCCGCTTCAC
TGGACTCTTCCTTTTCCTCTTGCGTCCGCATACCCCGCGCCACTGGGTCGTCTTCATT
CAGCCGCCGCACCGTGCGCTTACCTCCCTTGCCGTGCTTGATTAGCACCGGTGGGTT
GCTGAAACCCACCATTTGTAGCGCCACATCTTCTCTTTCTTCCTCGCTGTCCACGATC
ACCTCTGGGGATGGCGGGCGCTCGGGCTTGGGAGAGGGGCGCTTCTTTTTCTTTTTG
GACGCAATGGCCAAATCCGCCGTCGAGGTCGATGGCCGCGGGCTGGGTGTGCGCGG
CACCAGCGCATCTTGTGACGAGTCTTCTTCGTCCTCGGACTCGAGACGCCGCCTCAG
CCGCTTTTTTGGGGGCGCGCGGGGAGGCGGCGGCGACGGCGACGGGGACGACACGT
CCTCCATGGTTGGTGGACGTCGCGCCGCACCGCGTCCGCGCTCGGGGGTGGTTTCGC
GCTGCTCCTCTTCCCGACTGGCCATTTCCTTCTCCTATAGGCAGAAAAAGATCATGG
AGTCAGTCGAGAAGGAGGACAGCCTAACCGCCCCCTTTGAGTTCGCCACCACCGCC
TCCACCGATGCCGCCAACGCGCCTACCACCTTCCCCGTCGAGGCACCCCCGCTTGAG
GAGGAGGAAGTGATTATCGAGCAGGACCCAGGTTTTGTAAGCGAAGACGACGAGGA
TCGCTCAGTACCAACAGAGGATAAAAAGCAAGACCAGGACGACGCAGAGGCAAAC
GAGGAACAAGTCGGGCGGGGGGACCAAAGGCATGGCGACTACCTAGATGTGGGAG
ACGACGTGCTGTTGAAGCATCTGCAGCGCCAGTGCGCCATTATCTGCGACGCGTTGC
AAGAGCGCAGCGATGTGCCCCTCGCCATAGCGGATGTCAGCCTTGCCTACGAACGC
CACCTGTTCTCACCGCGCGTACCCCCCAAACGCCAAGAAAACGGCACATGCGAGCC
CAACCCGCGCCTCAACTTCTACCCCGTATTTGCCGTGCCAGAGGTGCTTGCCACCTA
TCACATCTTTTTCCAAAACTGCAAGATACCCCTATCCTGCCGTGCCAACCGCAGCCG
AGCGGACAAGCAGCTGGCCTTGCGGCAGGGCGCTGTCATACCTGATATCGCCTCGCT
CGACGAAGTGCCAAAAATCTTTGAGGGTCTTGGACGCGACGAGAAACGCGCGGCAA
ACGCTCTGCAACAAGAAAACAGCGAAAATGAAAGTCACTGTGGAGTGCTGGTGGAA
CTTGAGGGTGACAACGCGCGCCTAGCCGTGCTGAAACGCAGCATCGAGGTCACCCA
CTTTGCCTACCCGGCACTTAACCTACCCCCCAAGGTTATGAGCACAGTCATGAGCGA
GCTGATCGTGCGCCGTGCACGACCCCTGGAGAGGGATGCAAACTTGCAAGAACAAA
CCGAGGAGGGCCTACCCGCAGTTGGCGATGAGCAGCTGGCGCGCTGGCTTGAGACG
CGCGAGCCTGCCGACTTGGAGGAGCGACGCAAGCTAATGATGGCCGCAGTGCTTGT
TACCGTGGAGCTTGAGTGCATGCAGCGGTTCTTTGCTGACCCGGAGATGCAGCGCAA
GCTAGAGGAAACGTTGCACTACACCTTTCGCCAGGGCTACGTGCGCCAGGCCTGCA
AAATTTCCAACGTGGAGCTCTGCAACCTGGTCTCCTACCTTGGAATTTTGCACGAAA
ACCGCCTCGGGCAAAACGTGCTTCATTCCACGCTCAAGGGCGAGGCGCGCCGCGAC
 
TACGTCCGCGACTGCGTTTACTTATTTCTGTGCTACACCTGGCAAACGGCCATGGGC
GTGTGGCAGCAATGCCTGGAGGAGCGCAACCTAAAGGAGCTGCAGAAGCTGCTAAA
GCAAAACTTGAAGGACCTATGGACGGCCTTCAACGAGCGCTCCGTGGCCGCGCACC
TGGCGGACATTATCTTCCCCGAACGCCTGCTTAAAACCCTGCAACAGGGTCTGCCAG
ACTTCACCAGTCAAAGCATGTTGCAAAACTTTAGGAACTTTATCCTAGAGCGTTCAG
GAATTCTGCCCGCCACCTGCTGTGCGCTTCCTAGCGACTTTGTGCCCATTAAGTACC
GTGAATGCCCTCCGCCGCTTTGGGGTCACTGCTACCTTCTGCAGCTAGCCAACTACC
TTGCCTACCACTCCGACATCATGGAAGACGTGAGCGGTGACGGCCTACTGGAGTGTC
ACTGTCGCTGCAACCTATGCACCCCGCACCGCTCCCTGGTCTGCAATTCGCAACTGC
TTAGCGAAAGTCAAATTATCGGTACCTTTGAGCTGCAGGGTCCCTCGCCTGACGAAA
AGTCCGCGGCTCCGGGGTTGAAACTCACTCCGGGGCTGTGGACGTCGGCTTACCTTC
GCAAATTTGTACCTGAGGACTACCACGCCCACGAGATTAGGTTCTACGAAGACCAAT
CCCGCCCGCCAAATGCGGAGCTTACCGCCTGCGTCATTACCCAGGGCCACATCCTTG
GCCAATTGCAAGCCATCAACAAAGCCCGCCAAGAGTTTCTGCTACGAAAGGGACGG
GGGGTTTACCTGGACCCCCAGTCCGGCGAGGAGCTCAACCCAATCCCCCCGCCGCC
GCAGCCCTATCAGCAGCCGCGGGCCCTTGCTTCCCAGGATGGCACCCAAAAAGAAG
CTGCAGCTGCCGCCGCCGCCACCCACGGACGAGGAGGAATACTGGGACAGTCAGGC
AGAGGAGGTTTTGGACGAGGAGGAGGAGATGATGGAAGACTGGGACAGCCTAGAC
GAAGCTTCCGAGGCCGAAGAGGTGTCAGACGAAACACCGTCACCCTCGGTCGCATT
CCCCTCGCCGGCGCCCCAGAAATTGGCAACCGTTCCCAGCATCGCTACAACCTCCGC
TCCTCAGGCGCCGCCGGCACTGCCTGTTCGCCGACCCAACCGTAGATGGGACACCAC
TGGAACCAGGGCCGGTAAGTCTAAGCAGCCGCCGCCGTTAGCCCAAGAGCAACAAC
AGCGCCAAGGCTACCGCTCGTGGCGCGGGCACAAGAACGCCATAGTTGCTTGCTTG
CAAGACTGTGGGGGCAACATCTCCTTCGCCCGCCGCTTTCTTCTCTACCATCACGGC
GTGGCCTTCCCCCGTAACATCCTGCATTACTACCGTCATCTCTACAGCCCCTACTGCA
CCGGCGGCAGCGGCAGCGGCAGCAACAGCAGCGGTCACACAGAAGCAAAGGCGAC
CGGATAGCAAGACTCTGACAAAGCCCAAGAAATCCACAGCGGCGGCAGCAGCAGG
AGGAGGAGCGCTGCGTCTGGCGCCCAACGAACCCGTATCGACCCGCGAGCTTAGAA
ATAGGATTTTTCCCACTCTGTATGCTATATTTCAACAAAGCAGGGGCCAAGAACAAG
AGCTGAAAATAAAAAACAGGTCTCTGCGCTCCCTCACCCGCAGCTGCCTGTATCACA
AAAGCGAAGATCAGCTTCGGCGCACGCTGGAAGACGCGGAGGCTCTCTTCAGCAAA
TACTGCGCGCTGACTCTTAAGGACTAGTTTCGCGCCCTTTCTCAAATTTAAGCGCGA
AAACTACGTCATCTCCAGCGGCCACACCCGGCGCCAGCACCTGTCGTCAGCGCCATT
ATGAGCAAGGAAATTCCCACGCCCTACATGTGGAGTTACCAGCCACAAATGGGACT
TGCGGCTGGAGCTGCCCAAGACTACTCAACCCGAATAAACTACATGAGCGCGGGAC
CCCACATGATATCCCGGGTCAACGGAATCCGCGCCCACCGAAACCGAATTCTCCTCG
AACAGGCGGCTATTACCACCACACCTCGTAATAACCTTAATCCCCGTAGTTGGCCCG
CTGCCCTGGTGTACCAGGAAAGTCCCGCTCCCACCACTGTGGTACTTCCCAGAGACG
CCCAGGCCGAAGTTCAGATGACTAACTCAGGGGCGCAGCTTGCGGGCGGCTTTCGT
CACAGGGTGCGGTCGCCCGGGCGTTTTAGGGCGGAGTAACTTGCATGTATTGGGAAT
TGTAGTTTTTTTAAAATGGGAAGTGACGTATCGTGGGAAAACGGAAGTGAAGATTTG
AGGAAGTTGTGGGTTTTTTGGCTTTCGTTTCTGGGCGTAGGTTCGCGTGCGGTTTTCT
GGGTGTTTTTTGTGGACTTTAACCGTTACGTCATTTTTTAGTCCTATATATACTCGCTC
TGTACTTGGCCCTTTTTACACTGTGACTGATTGAGCTGGTGCCGTGTCGAGTGGTGTT
TTTTAATAGGTTTTTTTACTGGTAAGGCTGACTGTTATGGCTGCCGCTGTGGAAGCGC
TGTATGTTGTTCTGGAGCGGGAGGGTGCTATTTTGCCTAGGCAGGAGGGTTTTTCAG
 
GTGTTTATGTGTTTTTCTCTCCTATTAATTTTGTTATACCTCCTATGGGGGCTGTAATG
TTGTCTCTACGCCTGCGGGTATGTATTCCCCCGGGCTATTTCGGTCGCTTTTTAGCAC
TGACCGATGTTAACCAACCTGATGTGTTTACCGAGTCTTACATTATGACTCCGGACA
TGACCGAGGAACTGTCGGTGGTGCTTTTTAATCACGGTGACCAGTTTTTTTACGGTC
ACGCCGGCATGGCCGTAGTCCGTCTTATGCTTATAAGGGTTGTTTTTCCTGTTGTAAG
ACAGGCTTCTAATGTTTAAATGTTTTTTTTTTTGTTATTTTATTTTGTGTTTAATGCAG
GAACCCGCAGACATGTTTGAGAGAAAAATGGTGTCTTTTTCTGTGGTGGTTCCGGAA
CTTACCTGCCTTTATCTGCATGAGCATGACTACGATGTGCTTGCTTTTTTGCGCAGG
CTTTGCCTGATTTTTTGAGCAGCACCTTGCATTTTATATCGCCGCCCATGCAACAAGC
TTACATAGGGGCTACGCTGGTTAGCATAGCTCCGAGTATGCGTGTCATAATCAGTGT
GGGTTCTTTTGTCATGGTTCCTGGCGGGGAAGTGGCCGCGCTGGTCCGTGCAGACCT
GCACGATTATGTTCAGCTGGCCCTGCGAAGGGACCTACGGGATCGCGGTATTTTTGT
TAATGTTCCGCTTTTGAATCTTATACAGGTCTGTGAGGAACCTGAATTTTTGCAATCA
TGATTCGCTGCTTGAGGCTGAAGGTGGAGGGCGCTCTGGAGCAGATTTTTACAATGG
CCGGACTTAATATTCGGGATTTGCTTAGAGACATATTGATAAGGTGGCGAGATGAAA
ATTATTTGGGCATGGTTGAAGGTGCTGGAATGTTTATAGAGGAGATTCACCCTGAAG
GGTTTAGCCTTTACGTCCACTTGGACGTGAGGGCAGTTTGCCTTTTGGAAGCCATTGT
GCAACATCTTACAAATGCCATTATCTGTTCTTTGGCTGTAGAGTTTGACCACGCCACC
GGAGGGGAGCGCGTTCACTTAATAGATCTTCATTTTGAGGTTTTGGATAATCTTTTG
GAATAAAAAAAAAAAAACATGGTTCTTCCAGCTCTTCCCGCTCCTCCCGTGTGTGAC
TCGCAGAACGAATGTGTAGGTTGGCTGGGTGTGGCTTATTCTGCGGTGGTGGATGTT
ATCAGGGCAGCGGCGCATGAAGGAGTTTACATAGAACCCGAAGCCAGGGGGCGCCT
GGATGCTTTGAGAGAGTGGATATACTACAACTACTACACAGAGCGAGCTAAGCGAC
GAGACCGGAGACGCAGATCTGTTTGTCACGCCCGCACCTGGTTTTGCTTCAGGAAAT
ATGACTACGTCCGGCGTTCCATTTGGCATGACACTACGACCAACACGATCTCGGTTG
TCTCGGCGCACTCCGTACAGTAGGGATCGCCTACCTCCTTTTGAGACAGAGACCCGC
GCTACCATACTGGAGGATCATCCGCTGCTGCCCGAATGTAACACTTTGACAATGCAC
AACGTGAGTTACGTGCGAGGTCTTCCCTGCAGTGTGGGATTTACGCTGATTCAGGAA
TGGGTTGTTCCCTGGGATATGGTTCTGACGCGGGAGGAGCTTGTAATCCTGAGGAAG
TGTATGCACGTGTGCCTGTGTTGTGCCAACATTGATATCATGACGAGCATGATGATC
CATGGTTACGAGTCCTGGGCTCTCCACTGTCATTGTTCCAGTCCCGGTTCCCTGCAGT
GCATAGCCGGCGGGCAGGTTTTGGCCAGCTGGTTTAGGATGGTGGTGGATGGCGCC
ATGTTTAATCAGAGGTTTATATGGTACCGGGAGGTGGTGAATTACAACATGCCAAAA
GAGGTAATGTTTATGTCCAGCGTGTTTATGAGGGGTCGCCACTTAATCTACCTGCGC
TTGTGGTATGATGGCCACGTGGGTTCTGTGGTCCCCGCCATGAGCTTTGGATACAGC
GCCTTGCACTGTGGGATTTTGAACAATATTGTGGTGCTGTGCTGCAGTTACTGTGCTG
ATTTAAGTGAGATCAGGGTGCGCTGCTGTGCCCGGAGGACAAGGCGTCTCATGCTGC
GGGCGGTGCGAATCATCGCTGAGGAGACCACTGCCATGTTGTATTCCTGCAGGACG
GAGCGGCGGCGGCAGCAGTTTATTCGCGCGCTGCTGCAGCACCACCGCCCTATCCTG
ATGCACGATTATGACTCTACCCCCATGTAGGCGTGGACTTCCCCTTCGCCGCCCGTT
GAGCAACCGCAAGTTGGACAGCAGCCTGTGGCTCAGCAGCTGGACAGCGACATGAA
CTTAAGCGAGCTGCCCGGGGAGTTTATTAATATCACTGATGAGCGTTTGGCTCGACA
GGAAACCGTGTGGAATATAACACCTAAGAATATGTCTGTTACCCATGATATGATGCT
TTTTAAGGCCAGCCGGGGAGAAAGGACTGTGTACTCTGTGTGTTGGGAGGGAGGTG
GCAGGTTGAATACTAGGGTTCTGTGAGTTTGATTAAGGTACGGTGATCAATATAAGC
TATGTGGTGGTGGGGCTATACTACTGAATGAAAAATGACTTGAAATTTTCTGCAATT
 
GAAAAATAAACACGTTGAAACATAACATGCAACAGGTTCACGATTCTTTATTCCTGG
GCAATGTAGGAGAAGGTGTAAGAGTTGGTAGCAAAAGTTTCAGTGGTGTATTTTCCA
CTTTCCCAGGACCATGTAAAAGACATAGAGTAAGTGCTTACCTCGCTAGTTTCTGTG
GATTCACTAGAATCGATGTAGGATGTTGCCCCTCCTGACGCGGTAGGAGAAGGGGA
GGGTGCCCTGCATGTCTGCCGCTGCTCTTGCTCTTGCCGCTGCTGAGGAGGGGGGCG
CATCTGCCGCAGCACCGGATGCATCTGGGAAAAGCAAAAAAGGGGCTCGTCCCTGT
TTCCGGAGGAATTTGCAAGCGGGGTCTTGCATGACGGGGAGGCAAACCCCCGTTCG
CCGCAGTCCGGCCGGCCCGAGACTCGAACCGGGGGTCCTGCGACTCAACCCTTGGA
AAATAACCCTCCGGCTACAGGGAGCGAGCCACTTAATGCTTTCGCTTTCCAGCCTAA
CCGCTTACGCCGCGCGCGGCCAGTGGCCAAAAAAGCTAGCGCAGCAGCCGCCGCGC
CTGGAAGGAAGCCAAAAGGAGCGCTCCCCCGTTGTCTGACGTCGCACACCTGGGTT
CGACACGCGGGCGGTAACCGCATGGATCACGGCGGACGGCCGGATCCGGGGTTCGA
ACCCCGGTCGTCCGCCATGATACCCTTGCGAATTTATCCACCAGACCACGGAAGAGT
GCCCGCTTACAGGCTCTCCTTTTGCACGGTCTAGAGCGTCAACGACTGCGCACGCCT
CACCGGCCAGAGCGTCCCGACCATGGAGCACTTTTTGCCGCTGCGCAACATCTGGAA
CCGCGTCCGCGACTTTCCGCGCGCCTCCACCACCGCCGCCGGCATCACCTGGATGTC
CAGGTACATCTACGGATTACGTCGACGTTTAAACCATATGATCAGCTCACTCAAAGG
CGGTAATACGGTTATCCACAGAATCAGGGGATAACGCAGGAAAGAACATGTGAGCA
AAAGGCCAGCAAAAGGCCAGGAACCGTAAAAAGGCCGCGTTGCTGGCGTTTTTCCA
TAGGCTCCGCCCCCCTGACGAGCATCACAAAAATCGACGCTCAAGTCAGAGGTGGC
GAAACCCGACAGGACTATAAAGATACCAGGCGTTTCCCCCTGGAAGCTCCCTCGTG
CGCTCTCCTGTTCCGACCCTGCCGCTTACCGGATACCTGTCCGCCTTTCTCCCTTCGG
GAAGCGTGGCGCTTTCTCATAGCTCACGCTGTAGGTATCTCAGTTCGGTGTAGGTCG
TTCGCTCCAAGCTGGGCTGTGTGCACGAACCCCCCGTTCAGCCCGACCGCTGCGCCT
TATCCGGTAACTATCGTCTTGAGTCCAACCCGGTAAGACACGACTTATCGCCACTGG
CAGCAGCCACTGGTAACAGGATTAGCAGAGCGAGGTATGTAGGCGGTGCTACAGAG
TTCTTGAAGTGGTGGCCTAACTACGGCTACACTAGAAGAACAGTATTTGGTATCTGC
GCTCTGCTGAAGCCAGTTACCTTCGGAAAAAGAGTTGGTAGCTCTTGATCCGGCAAA
CAAACCACCGCTGGTAGCGGTGGTTTTTTTGTTTGCAAGCAGCAGATTACGCGCAGA
AAAAAAGGATCTCAAGAAGATCCTTTGATCTTTTCTACGGGGTCTGACGCTCAGTGG
AACGAAAACTCACGTTAAGGGATTTTGGTCATGAGATTATCAAAAAGGATCTTCACC
TAGATCCTTTTAAATTAAAAATGAAGTTTTAAATCAATCTAAAGTATATATGAGTAA
ACTTGGTCTGACAGTTACCAATGCTTAATCAGTGAGGCACCTATCTCAGCGATCTGT
CTATTTCGTTCATCCATAGTTGCCTGACTCCCCGTCGTGTAGATAACTACGATACGGG
AGGGCTTACCATCTGGCCCCAGTGCTGCAATGATACCGCGAGACCCACGCTCACCG
GCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTA
AGTAGTTCGCCAGTTAATAGTTTGCGCAACGTTGTTGCCATTGCTACAGGCATCGTG
GTGTCACGCTCGTCGTTTGGTATGGCTTCATTCAGCTCCGGTTCCCAACGATCAAGG
CGAGTTACATGATCCCCCATGTTGTGCAAAAAAGCGGTTAGCTCCTTCGGTCCTCCG
ATCGTTGTCAGAAGTAAGTTGGCCGCAGTGTTATCACTCATGGTTATGGCAGCACTG
CATAATTCTCTTACTGTCATGCCATCCGTAAGATGCTTTTCTGTGACTGGTGAGTACT
CAACCAAGTCATTCTGAGAATAGTGTATGCGGCGACCGAGTTGCTCTTGCCCGGCGT
CAATACGGGATAATACCGCGCCACATAGCAGAACTTTAAAAGTGCTCATCATTGGA
AAACGTTCTTCGGGGCGAAAACTCTCAAGGATCTTACCGCTGTTGAGATCCAGTTCG
ATGTAACCCACTCGTGCACCCAACTGATCTTCAGCATCTTTTACTTTCACCAGCGTTT
 
CTGGGTGAGCAAAAACAGGAAGGCAAAATGCCGCAAAAAAGGGAATAAGGGCGAC
ACGGAAATGTTGAATACTCATACTCTTCCTTTTTCAATATTATTGAAGCATTTATCAG
GGTTATTGTCTCATGAGCGGATACATATTTGAATGTATTTAGAAAAATAAACAAATA
GGGGTTCCGCGCACATTTCCCCGAAAAGTGCCACCTAAATTGTAAGCGTTAATATTT
TGTTAAAATTCGCGTTAAATTTTTGTTAAATCAGCTCATTTTTTAACCAATAGGCCGA
AATCGGCAAAATCCCTTATAAATCAAAAGAATAGACCGAGATAGGGTTGAGTGTTG
TTCCAGTTTGGAACAAGAGTCCACTATTAAAGAACGTGGACTCCAACGTCAAAGGG
CGAAAAACCGTCTATCAGGGCGATGGCCCACTACGTGAACCATCACCCTAATCAAG
TTTTTTGGGGTCGAGGTGCCGTAAAGCACTAAATCGGAACCCTAAAGGGAGCCCCC
GATTTAGAGCTTGACGGGGAAAGCCGGCGAACGTGGCGAGAAAGGAAGGGAAGAA
AGCGAAAGGAGCGGGCGCTAGGGCGCTGGCAAGTGTAGCGGTCACGCTGCGCGTAA
CCACCACACCCGCCGCGCTTAATGCGCCGCTACAGGGCGCGATGGATCC
SEQ ID NO: 22

CGTTACATAACTTACGGTAAATGGCCCGCCTGGCTGACCGCCCAACGACCCCCGCCC
ATTGACGTCAATAATGACGTATGTTCCCATAGTAACGCCAATAGGGACTTTCCATTG
ACGTCAATGGGTGGAGTATTTACGGTAAACTGCCCACTTGGCAGTACATCAAGTGTA
TCATATGCCAAGTACGCCCCCTATTGACGTCAATGACGGTAAATGGCCCGCCTGGCA
TTATGCCCAGTACATGACCTTATGGGACTTTCCTACTTGGCAGTACATCTACGTATTA
GTCATCGCTATTACCATGGTCGAGGTGAGCCCCACGTTCTGCTTCACTCTCCCCATCT
CCCCCCCCTCCCCACCCCCAATTTTGTATTTATTTATTTTTTAATTATTTTGTGCAGCG
ATGGGGGCGGGGGGGGGGGGGGGGCGCGCGCCAGGCGGGGCGGGGCGGGGCGAG
GGGCGGGGCGGGGCGAGGCGGAGAGGTGCGGCGGCAGCCAATCAGAGCGGCGCGC
TCCGAAAGTTTCCTTTTATGGCGAGGCGGCGGCGGCGGCGGCCCTATAAAAAGCGA
AGCGCGCGGCGGGCGGGAGTCGCTGCGACGCTGCCTTCGCCCCGTGCCCCGCTCCG
CCGCCGCCTCGCGCCGCCCGCCCCGGCTCTGACTGACCGCGTTACTCCCACAGGTGA
GCGGGCGGGACGGCCCTTCTCCTCCGGGCTGTAATTAGCTGAGCAAGAGGTAAGGG
TTTAAGGGATGGTTGGTTGGTGGGGTATTAATGTTTAATTACCTGGAGCACCTGCCT
GAAATCACTTTTTTTCAGGT

## Claims

1. A gene sequence construct for gene therapy of HIV infection, comprising one or more gene coding sequences of a single-chain antibody molecule (including a nanobody) without a constant region that has the ability to inhibit HIV infection and one or more gene coding sequences of a polypeptide (consisting of 2-50 amino acid residues) with the ability to inhibit HIV infection, to achieve the expression of a fusion protein molecule comprising the antibody molecule and the polypeptide against HIV infection encoded by a single gene, which has two or more than two target sites.

2. The gene sequence construct according to claim 1, wherein the single chain antibody molecule comprises a heavy chain variable region and/or a light chain variable region.

3. The gene sequence construct according to claim 2, wherein the light chain variable region comprises a light chain variable region of a kappa or lambda light chain.

4. The gene sequence construct according to any one of claims 1-3, comprising two or more than two gene coding sequences of the single-chain antibody molecules without the constant region that has the ability to inhibit HIV infection.

5. The gene sequence construct according to any one of claims 1-4, comprising three or more than three gene coding sequences of the single-chain antibody molecules without the constant region that has the ability to inhibit HIV infection.

6. The gene sequence construct according to any one of claims 1-5, comprising four or more than four gene coding sequences of the single-chain antibody molecules without the constant region that has the ability to inhibit HIV infection.

7. The gene sequence construct according to any one of claims 1-6, comprising two or more than two gene coding sequences of the polypeptide with the ability to inhibit HIV infection.

8. The gene sequence construct according to any one of claims 1-7, comprising three or more than three gene coding sequences of the polypeptide with the ability to inhibit HIV infection.

9. The gene sequence construct according to any one of claims 1-8, comprising four or more than four gene coding sequences of the polypeptide with the ability to inhibit HIV infection.

10. The gene sequence construct according to any one of claims 1-9, wherein the fusion protein molecule comprising the single-chain antibody molecule without the constant region and the polypeptide against HIV infection encoded by the single gene has three or more than three target sites.

11. The gene sequence construct according to any one of claims 1-10, wherein the fusion protein molecule comprising the single-chain antibody molecule without the constant region and the polypeptide against HIV infection encoded by the single gene has four or more than four target sites.

12. The gene sequence construct according to any one of claims 1-11, wherein the fusion protein molecule comprising the single-chain antibody molecule without the constant region and the polypeptide against HIV infection encoded by the single gene has five or more than five target sites.

13. The gene sequence construct according to any one of claims 1-12, wherein the fusion protein molecule comprising the single-chain antibody molecule without the constant region and the polypeptide against HIV infection encoded by the single gene has six or more than six target sites.

14. The gene sequence construct according to any one of claims 1-13, comprising two or more than two gene coding sequences of the single-chain antibody molecule without the constant region that has the ability to inhibit HIV infection and one or more gene coding sequences of the polypeptide with the ability to inhibit HIV infection.

15. The gene sequence construct according to claim 14, wherein the fusion protein molecule comprising the single-chain antibody molecule without the constant region and the polypeptide against HIV infection encoded by the single gene has three or more than three target sites.

16. The gene sequence construct according to any one of claims 1-15, wherein the gene coding sequence of the single-chain antibody molecule without the constant region that has the ability to inhibit HIV infection and the gene coding sequence of the polypeptide with the ability to inhibit HIV infection are directly or indirectly concatenated via a coding sequence of a linker polypeptide.

17. The gene sequence construct according to any one of claims 1-16, comprising two or more than two gene coding sequences of the single-chain antibody molecule without the constant region that has the ability to inhibit HIV infection, wherein the gene coding sequences of the antibody molecules are directly or indirectly concatenated via a coding sequence of a linker polypeptide.

18. The gene sequence construct according to any one of claims 1-17, comprising two or more than two gene coding sequences of the polypeptide with the ability to inhibit HIV infection, wherein the gene coding sequences of the polypeptides are directly or indirectly concatenated via a coding sequence of a linker polypeptide.

19. The gene sequence construct according to any one of claims 1-18, wherein the one or more gene coding sequences of the single-chain antibody molecule without the constant region that has the ability to inhibit HIV infection comprises a gene coding sequence of an anti-HIV-1-gp160 (or its cleavage products gp120 and gp41) antibody molecule.

20. The gene sequence construct according to any one of claims 1-19, wherein the one or more gene coding sequences of the single-chain antibody molecule without the constant region that has the ability to inhibit HIV infection comprises a gene coding sequence of an antibody molecule that binds to human CD4 receptor site.

21. The gene sequence construct according to any one of claims 1-20, wherein the one or more gene coding sequence of the polypeptide with the ability to inhibit HIV infection comprises a gene coding sequence of a polypeptide that inhibits the fusion of HIV and CD4+ T cell membranes.

22. The gene sequence construct according to any one of claims 1-21, comprising two or more than two gene coding sequences of the single-chain antibody molecules without the constant region that has the ability to inhibit HIV infection and one or more gene coding sequences of the polypeptide with the ability to inhibit HIV infection, wherein the two or more than two gene coding sequences of the single-chain antibody molecules without the constant region that has the ability to inhibit HIV infection comprise a gene coding sequence of an antibody molecule against HIV-1-gp160 (or its cleavage products gp120 and gp41) and a gene coding sequence of an antibody molecule that binds to human CD4 receptor site, and wherein the one or more gene coding sequences of the polypeptide with the ability to inhibit HIV infection comprise a gene coding sequence of a polypeptide that inhibits the fusion of HIV and CD4+ T cell membranes.

23. The gene sequence construct according to any one of claims 1-22, comprising (i) gene coding sequences of light chain complementarity determining regions (LCDR1, LCDR2 and LCDR3) and heavy chain complementarity determining regions (HCDR1, HCDR2 and HCDR3) of an anti-HIV-1-gp 160 (or its cleavage products gp120 and gp41) monoclonal antibody , (ii) gene coding sequences of light chain complementarity determining regions (LCDR1, LCDR2 and LCDR3) and heavy chain complementarity determining regions (HCDR1, HCDR2 and HCDR3) of a monoclonal antibody that binds to human CD4 receptor site, (iii) a gene coding sequence of a polypeptide that inhibits the fusion of HIV and CD4+ T cell membranes, wherein the coding sequences of the light chain and the heavy chain of the antibodies can be directly or indirectly concatenated via a coding sequence of a linker polypeptide in any order.

24. The gene sequence construct according to any one of claims 1-23, comprising (i) gene coding sequences of light chain variable region (VL) and heavy chain variable region (VH) of an anti-HIV-1-gp160 (or its cleavage products gp120 and gp41) monoclonal antibody , (ii) gene coding sequences of light chain variable region (VL) and heavy chain variable region (VH) of a monoclonal antibody that binds to human CD4 receptor site, (iii) a gene coding sequence of a polypeptide that inhibits the fusion of HIV and CD4+ T cell membranes, wherein the coding sequences of the light chain variable region (VL) and the heavy chain variable region (VH) of the antibodies can be directly or indirectly concatenated via a coding sequence of a linker polypeptide in any order.

25. The gene sequence construct according to any one of claims 1-24, further comprising a promoter located upstream of the gene coding sequence of the single-chain antibody molecule without the constant region that has the ability to inhibit HIV infection and the gene coding sequence of the polypeptide with the ability to inhibit HIV infection.

26. The gene sequence construct according to any one of claims 1-25, further comprising a secretion signal peptide coding sequence located upstream of the gene coding sequence of the single-chain antibody molecule without the constant region that has the ability to inhibit HIV infection and the gene coding sequence of the polypeptide with the ability to inhibit HIV infection.

27. The gene sequence construct according to any one of claims 1-26, comprising a first gene coding sequence of the single-chain antibody molecule without the constant region that has the ability to inhibit HIV infection, a second gene coding sequence of the single-chain antibody molecule without the constant region that has the ability to inhibit HIV infection, and a gene coding sequence of the polypeptide with the ability to inhibit HIV infection, and is selected from:
VL2-linker-VH2-linker-VL1-linker-VH1-linker; or
linker-VH2-linker-VL1-linker-VH1-linker-peptide inhibitor; or
other constructs that are constructed by arranging the combination of VL2 and VH2 or VL1 and VH1 in different orders in a construct;
wherein VL2 and VH2 are the variable region fragments of light chain and heavy chain of the first antibody molecule respectively; VL1 and VH1 are the variable region fragments of light chain and heavy chain of the second antibody molecule respectively; the linker is a linker polypeptide; the peptide inhibitor is a polypeptide that inhibits HIV infection (for example, a polypeptide that inhibits the fusion of HIV and CD4+ T cell membranes).

28. The gene sequence construct according to claim 27, which is VL2-linker-VH2-linker-VL1-linker-VH1, or a construct in which the combinations of VL2 and VH2 or VL1 and VH1 are arranged in different orders.

29. The gene sequence construct according to claim 27, which is VL2-linker-VH2-linker-VL1-linker-VH1-linker-peptide inhibitor, or a construct in which the combinations of VL2 and VH2 or VL1 and VH1 are arranged in different orders.

30. The gene sequence construct according to claims 27, 28 and 29, wherein the protein sequences of VL2 and VH2 include SEQ ID NO: 2, or a functional fragment thereof, or a homologous sequence that is at least 75%, 80%, 85%, 90%, 95%, 98%, or 99% identical thereto.

31. The gene sequence construct according to claims 27, 28 and 29, wherein the protein sequences of VL1 and VH1 include SEQ ID NO: 3, or a functional fragment thereof, or a homologous sequence that is at least 75%, 80%, 85%, 90%, 95%, 98%, or 99% identical thereto.

32. The gene sequence construct according to claims 27, 28 and 29, wherein the sequence of the linker polypeptide (linker) is selected from the following amino acid sequences: GGGGS, (GGGGS)₂, (GGGGS)₃, (GGGGS)₄, (GGGGS)₅, (GGGGS)₆ and (GGGGS)₇, or other optional linker polypeptide sequences.

33. The gene sequence construct according to claims 27 and 29, wherein the polypeptide that inhibits the fusion of HIV and CD4+ T cell membranes can be selected from the group consisting of membrane fusion inhibitory polypeptides P52, C34, T20, etc.

34. The gene sequence construct according to claim 33, wherein the sequence of the membrane fusion inhibitory polypeptide P52 includes SEQ ID NO: 5 or a homologous sequence that is at least 75%, 80%, 85%, 90%, 95%, 98%, or 99% identical thereto; the polypeptide sequence of C34 includes SEQ ID NO: 6 or a homologous sequence that is at least 75%, 80%, 85%, 90%, 95%, 98%, or 99% identical thereto; the polypeptide sequence of T20 includes SEQ ID NO: 7 or a homologous sequence that is at least 75%, 80%, 85%, 90%, 95%, 98%, or 99% identical thereto.

35. A viral vector genome comprising the construct of any one of the preceding claims.

36. A viral vector system comprising the genome according to claim 35.

37. The viral vector system according to claim 36, which is a lentiviral vector system or an adeno-associated virus vector system.

38. The lentiviral vector system according to claim 37, comprising the genome according to claim 35 and other nucleotide sequences encoding and expressing packaging components required for the production of lentivirus, which are introduced into production cells to produce a lentiviral particle containing the genome according to claim 35.

39. The adeno-associated virus vector system according to claim 37, comprising the genome according to claim 35 and other nucleotide sequences encoding and expressing packaging components required for the production of adeno-associated virus, which are introduced into production cells to produce an adeno-associated virus particle containing the genome according to claim 35.

40. A viral particle comprising the genome of the construct of any one of claims 1-34.

41. A pharmaceutical composition, comprising the viral particle according to claim 40, and a pharmaceutically acceptable carrier or diluent, or cells transduced by the lentiviral particle according to claim 38 in vitro, including but not limited to transduced muscle cells, liver cells, or CD4+ T cells.

42. The virus particle according to claims 38-40 or the pharmaceutical composition according to claim 41, for use in injection into the body to express an antibody molecule protein with two or more than two target sites, which can effectively and broadly block the infection of HIV against human CD4+ T cells by binding to multiple binding sites involved in different steps of HIV infection against human CD4+ T cells, and can be used for gene therapy of HIV infection, thereby achieving long-term treatment for a HIV-infected individual.

43. A method of inhibiting HIV infection, comprising administering to cells the viral particle or the pharmaceutical composition according to claims 38-41.

44. The method according to claim 43, wherein the cells comprise muscle cells, liver cells, or CD4+ T cells.

45. The method according to claim 43, wherein the method comprises transducing the cells of claim 44 in vitro or in vivo with the viral particle or the pharmaceutical composition according to claims 38-41.

46. A method of treating HIV infection in a subject in need thereof, wherein the method comprises administering to the subject a therapeutically effective dose of the viral particle or the pharmaceutical composition of claims 38-41.

47. The method according to claim 46, wherein the subject includes an early-stage HIV infector, an HIV-infected individual who is already received cocktail drug therapy, or an HIV-infected individual who is resistant to cocktail drug therapy.

48. The method according to claim 42, wherein the viral particle or the pharmaceutical composition according to claims 38-41 is injected intramuscularly.

49. The method according to claim 42, wherein the viral particle or the pharmaceutical composition according to claims 38-41 or CD4+ T cells transduced thereby is injected intravenously.

50. The virus particle and the pharmaceutical composition injected into the body according to the method of claims 48 and 49, which can express anti-HIV protein molecules with multiple targets sites and secrete them into blood, which can act on multiple nodes of HIV infection, and can effectively block HIV infection path and effectively avoid the loss of the ability to inhibit HIV infection due to escape mutations of HIV, thereby achieving a long-term (for example, therapeutically effective lasts for one or several years) or even permanent therapeutic effect on HIV infection with a single injection.
